(19) Europäisches Patentamt
European Patent Office
Office européen des brevets

(11) Publication number: **0 505 151 A2**

(12) **EUROPEAN PATENT APPLICATION**

(21) Application number: **92302298.2**

(22) Date of filing: **18.03.92**

(51) Int. Cl.$^5$: **C12N 15/57,** C12N 15/31, C12N 15/62, C12N 9/52, C07K 3/18, C12Q 1/37, B01J 20/32

(30) Priority: **19.03.91 US 672704**

(43) Date of publication of application: **23.09.92 Bulletin 92/39**

(84) Designated Contracting States: **AT BE CH DE DK ES FR GB GR IT LI NL PT SE**

(71) Applicant: **ELI LILLY AND COMPANY**
**Lilly Corporate Center**
**Indianapolis Indiana 46285 (US)**

(72) Inventor: **Blaszczak, Larry Chris**
**5825 North Broadway**
**Indianapolis, Indiana 46220 (US)**
Inventor: **Skatrud, Paul Luther**
**1045 Fiesta Drive**
**Greenwood, Indiana 46143 (US)**
Inventor: **Smith, Michele Ceceil**
**4515 North Lakeridge Drive**
**Indianapolis, Indiana 46234 (US)**
Inventor: **Wu, Chyun-Yeh Earnest**
**828 Gettysburg Court**
**Indianapolis, Indiana 46217 (US)**

(74) Representative: **Hudson, Christopher Mark et al**
**Erl Wood Manor**
**Windlesham Surrey GU20 6PH (GB)**

(54) **Preparation of the penicilline binding protein 2A from staphylococcus aureus 27R, purification and use in a resistant-assay.**

(57) The invention provides a gene encoding a novel PBP2A penicillin binding protein isolated from the methicillin resistant *S. aureus* strain 27R. The invention further provides soluble forms of PBP2A proteins lacking the transmembrane association region yet retain their active site configuration of the PBP2A protein. The invention also provides a method of assaying for agents useful against methicillin resistant staphylococcus strains by creating a kinetically inert complex between a support-mobilized transition metal ion and a modified soluble form of the PBP2A protein incorporating a chelating agent. The invention further provides a cell free method of assaying for agents which bind the PBP2A-27R protein.

EP 0 505 151 A2

## BACKGROUND OF THE INVENTION

The β-lactam antibiotics are capable of killing sensitive bacterial cells. Although the mechanism of action of these antibiotics is not completely understood, it is generally believed that they interfere with the formation of the bacterial cell wall. The proper formation of the bacterial cell wall is necessary for normal growth and development of most bacteria. One component of the bacterial cell wall is peptidoglycan. This molecule is a polymer of heterogeneous composition which when cross-linked provides a latticework structure covering the cell surface which in turn provides rigid mechanical properties to the cell. In gram-positive bacteria, the cell wall is 50-100 molecules thick. However, in gram-negative bacteria, the cell wall is only 1 to 2 molecules thick.

The peptidoglycan layer is composed of long glycan chains cross-linked by short peptide chains. The glycan chains are composed of two alternating sugar molecules, N-acetylglucosamine and N-acetylmuramic acid. In Staphylococcus aureus, short (4 amino acids) peptides are attached to the N-acetylmuramic acid residues. These short peptides are in turn linked via a pentaglycine peptide which serves to complete the linkage between the long peptidoglycan chains. This final crosslinking reaction is catalyzed by a membrane-bound transpeptidase protein. It is this last step which is inhibited by the β-lactam antibiotics. The antibiotic mimics the enzyme's natural substrate and upon binding to the enzyme inactivates this key enzyme, probably by acylation of the enzyme.

While the foregoing provides a brief outline of the interaction of β-lactams with a transpeptidase it is essential to understand that there are several types of transpeptidases involved in bacterial cell wall biosynthesis. When radioactive pencillin is used as a inhibitor, these transpeptidases can be identified and studied. Hence they are termed "penicillin binding proteins" (PBP's).

Recently, a major concern has arisen over the growing number of reports of bacteria which are resistant to β-lactam antibiotics. It is known that such β-lactam-resistant cells produce PBPs different from those found in cells sensitive to β-lactam antibiotics. These modified PBPs have a very low affinity for β-lactam antibiotics. The affinity of these modified PBPs for penicillin (and other β-lactam antibiotics) is so low that to refer to them as "penicillin binding proteins" may seem to be a misnomer. However, they are clearly related to true PBPs and, given that they are commonly referred to in the art as penicillin binding proteins, that nomenclature will be perpetuated hereinafter. While there are other mechanisms to confer β-lactam resistance (Tomasz, A., et al (1989) Antimicrob. Agents and Chemotherapy 33(11) 1869-1874), it is widely believed that one key to development of antibiotics against these β-lactam resistant microorganisms lies in finding agents which will bind to these modified PBPs.

Modified PBPs have been isolated from methicillin resistant strains of Staphylococcus and their DNA coding sequences have been cloned and sequenced. Compounds having high affinity for these modified PBPs would have substantial therapeutic value as antibiotics against methicillin resistant strains of staphylococcus. To provide as broad a range of antibiotic activity as possible, it is necessary to analyze a potential drug's affinity for as many PBPs produced by MRS as possible. The instant invention provides one such PBP, the PBP2A of S. aureus strain 27R, useful in the rational design of therapeutic compounds effective against MRS infections.

## SUMMARY OF THE INVENTION

The instant invention provides the DNA sequence and amino acid sequence of one such modified penicillin binding protein, the PBP2A penicillin binding protein isolated from Staphylococcus aureus strain 27R hereinafter referred to as PBP2A-27R. The instant invention also provides the DNA sequence of a PBP2A-27R derivative so as to encode a PBP2A-27R protein which possesses a chelating peptide in-frame to the reading frame of PBP2A-27R thereby expressing a PBP2A-27R$^s$ protein containing a chelating peptide amino acid sequence hereinafter referred to as PBP2A-27R-CP. This PBP2A-27R-CP molecule may then be purified on an immobilized metal ion column in substantial accordance with the teaching of Smith et al., United States Patent No. 4,569,794 the entire teaching of which is hereby incorporated by reference. The instant invention also provides a PBP2A-27R-CP molecule and the DNA sequence encoding this molecule wherein a proline residue has been placed between the chelating peptide and the PBP2A-27R$^s$ amino acid sequences to provide a convenient and specific dipeptidase digestion-termination site facilitating removal of the chelating peptide from PBP2A-27R$^s$ subsequent to purification by CP-IMAC. The invention further provides a method for purifying PBPs by operably linking such PBPs to chelating peptides, such as by incorporating a DNA sequence encoding such chelating peptides at either the 5′ or 3′ end of the DNA coding sequence encoding the PBP2A-27R protein. The instant invention also provides a soluble form of the PBP2A-27R protein (denoted PBP2A-27R$^s$) derived from a full PBP2A-27R protein, said PBP2A-27R$^s$ protein being devoid of its natural transmembrane region. The invention further provides a method for using the PBP2A-27R or PBP2A-27R$^s$ or PBP2A-27R$^s$-CP proteins in a screening procedure for the detection of compounds having therapeutic value in the treatment of methicillin resistant

staphylococci. The soluble forms of the PBP2A-27R protein facilitate crystallization. Crystalline forms of soluble PBP2A-27R may be studied by x-ray crystallography assisting in the rational design of antibiotic compounds useful against MRS infections.

## BRIEF DESCRIPTION OF THE FIGURES

The restriction site and function maps presented in the accompanying drawings are approximate representations of the recombinant DNA vectors discussed herein. The restriction site information is not exhaustive; therefore there may be more restriction sites of a given type on the vector than are illustrated in the drawings.

Figure 1 -- A restriction site and function map of plasmid pUC19.
Figure 2 -- A restriction site and function map of plasmid pEWSA8.
Figure 3 -- A restriction site and function map of plasmid pEWSA37.
Figure 4 -- A restriction site and function map of plasmid M13mp18 RF.
Figure 5 -- A restriction site and function map of plasmid M13mp19 RF.
Figure 6 -- A restriction site and function map of plasmid pEWM13-1.
Figure 7 -- A restriction site and function map of plasmid pEWM13-7.
Figure 8 -- A restriction site and function map of plasmid pEWM13-9.
Figure 9 -- A restriction site and function map of plasmid pEWM13-12.
Figure 10 -- A restriction site and function map of plasmid pEWM13-21.
Figure 11 -- A restriction site and function map of plasmid pEWM13-22.
Figure 12 -- A restriction site and function map of plasmid pEWM13-23.
Figure 13 -- A restriction site and function map of plasmid pEWSA24.
Figure 14 -- A restriction site and function map of plasmid pEWSA25.
Figure 15 -- A restriction site and function map of plasmid pEWSA26.
Figure 16 -- A restriction site and function map of plasmid pEWSA27.
Figure 17 -- A restriction site and function map of plasmid pEWSA28.
Figure 18 -- A restriction site and function map of plasmid pEWSA29.
Figure 19 -- A restriction site and function map of plasmid pOW241.
Figure 20 -- A restriction site and function map of plasmid pEWSA30.
Figure 21 -- A restriction site and function map of plasmid pEWSA31.
Figure 22 -- A restriction site and function map of plasmid pEWSA32.
Figure 23 -- The DNA sequence of the mecA-27R gene of Staphylococcus aureus strain 27R which encodes the PBP2A penicillin binding protein denoted PBP2A-27R.
Figure 24 -- The amino acid sequence of the PBP2A-27R protein.
Figure 25 -- The DNA sequence and corresponding amino acid sequence of the mecA-27R gene of Staphylococcus aureus strain 27R which encodes the PBP2A penicillin binding protein denoted PBP2A-27R.
Figure 26 -- Autoradiograms of SDS-PAGE gels illustrating the expression of the PBP2A-27R protein from cells transformed with the pEWSA8 and pEWSA37 plasmids with varying concentrations of sodium clavulanate.
Figure 27 -- A restriction site and function map of plasmid pEWM39.
Figure 28 -- A restriction site and function map of plasmid pEWSA40.
Figure 29 -- A restriction site and function map of plasmid pEWSA41.
Figure 30 -- DNA sequences of the known forms of PBP2A proteins of MRS strains. The sequence labelled "Mecaepi" is the mecA gene isolated from S. epidermidis strain WT55. The sequence labelled "Mecaaur" is the mecA gene isolated from S. aureus strain BB270. The sequence labelled "Matso" is the mecA gene isolated from S. aureus strain TK784. The sequence labelled "Ewpbp" is the mecA-27R gene isolated from S. aureus strain 27R.

## DEFINITIONS

Ala -- the amino acid alanine

Analog -- a compound which is structurally similar to another. When used in reference to polypeptides, it refers to primary, secondary, or tertiary structure.

Arg -- the amino acid arginine.

Asn -- the amino acid asparagine.

Asp -- the amino acid aspartic acid.

Base pair (bp) -- refers to DNA or RNA. The abbreviations A,C,G, and T correspond to the 5′-monophosphate forms of the nucleotides (deoxy)adenine, (deoxy)cytidine, (deoxy)guanine, and (deoxy)thymidine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with U or C with G.

CP-IMAC -- an abbreviation for CP-Immobilized Metal Ion Affinity Chromotography.

CP-Immobilized Metal Ion Affinity Chromotography-- a method of linking a fusion protein containing a chelating peptide or a free chelating peptide to a solid support or another fusion protein with a chelating peptide where this solid support or alternative CP-protein possesses a coordinately complexed metal ion as described in United States Patent 4,569,794, the entire teaching of which is hereby incorporated by reference.

Chelating Peptide -- An amino acid sequence capable of complexing with a multivalent metal ion. Chelating peptides are used in the practice of CP-Immobilized Metal Ion Affinity Chromotography purification and immobilization

CP-protein -- a fusion protein comprising an amino acid sequence of a chelating peptide operably linked to a protein molecule.

Cys -- the amino acid cysteine or one-half of a cystine residue covalently linked via a disulfide bridge to another one-half cystine residue.

DNA -- Deoxyribonucleic acid.

EDTA -- an abbreviation for ethylenediamine tetraacetic acid.

Functional analog -- refers to a molecule having similar functional properties but a modified structure relative to the naturally occurring form of that molecule or compound. A functional analog includes fragments of or additions to the parent molecule which have similar functional properties and reactivities as the parent molecule.

Functional Equivalent(s) -- Two molecules which have the same function but different structures, When used in reference to DNA and RNA compounds, it encompasses those multiple DNA sequences which encode a single protein by virtue of the inherent degeneracy of the genetic code. It will be readily appreciated by those of ordinary skill in the art that a given DNA sequence is only one of a large but definite body of DNA sequences which will encode a given protein. The natural degeneracy of the genetic code is well documented. Stryer, L. Biochemistry, 2d ed. (1981) W. H. Freeman and Company, San Francisco, pp. 619-640. Therefore, a number of DNA and RNA sequences may be created, either by synthetic methodology or site-specific mutagenesis of an existing sequence, the transcription and/or translation of which will produce a particular protein.

Fusion Protein -- refers to a two-domain polypeptide molecule of which the first domain comprises the amino acid sequence of a protein molecule operably linked to a second domain comprising an amino acid sequence heterogeneous to the aforesaid protein molecule.

| | |
|---|---|
| Gln -- | the amino acid glutamine. |
| Glu -- | the amino acid glutamic acid. |
| Gly -- | the amino acid glycine. |
| His -- | the amino acid histidine. |
| | IMAC -- an abbreviation for immobilized metal ion affinity chromotography. |
| Ile -- | the amino acid isoleucine. |
| Leu -- | the amino acid leucine |
| Lys -- | the amino acid lysine. |
| Met -- | the amino acid methionine or its deformylated analog. |
| mRNA -- | messenger RNA. |
| MRS -- | an abbreviation for methicillin resistant Staphylococccus. |
| MRSA -- | an abbreviation for methicillin resistant Staphylococccus aureus. |
| PBP -- | an abbreviation for penicillin binding protein |
| PBP2A-AUR -- | A PBP2a protein isolated from S. aureus strain BB270 comprising the amino acid sequence: |

1 49
MKKIKIVPLI LIVVVVGFGI YFYASKDKEI NNTIDAIEDK NFKQVYKDSS

50 99
YISKSDNGEV EMTERPIKIY NSLGVKDINI QDRKIKKVSK NKKRVDAQYK

100 149
IKTNYGNIDR NVQFNFVKED GMWKLDWDHS VIIPGMQKDQ SIHIENLKSE

150 199
RGKILDRNNV ELANTGTHMR LGIVPKNVSK KDYKAIAKEL SISEDYINNK

```
200                                                249
WIKIGYKMIP SFHFKTVKKM DEYLSDFAKK FHLTTNETES RNYPLEKATS

250                                                299
HLLGYVGPIN SEELKQKEYK GYKDDAVIGK KGLEKLYDKK LQHEDGYRVT

300                                                349
IVDDNSNTIA HTLIEKKKKD GKDIQLTIDA KVQKSIYNNM KNDYGSGTAI

350                                                399
HPQTGELLAL VSTPSYDVYP FMYGMSNEEY NKLTEDKKEP LLNKFQITTS

400                                                449
PGSTQKILTA MIGLNNKTLD DKTSYKIDGK GWQKDKSWGG YNVTRYEVVN

450                                                499
GNIDLKQAIE SSDNIFFARV ALELGSKKFE KGMKKLGVGE DIPSDYPFYN

500                                                549
AQISNKNLDN EILLADSGYG QGEILINPVQ ILSIYSALEN NGNINAPHLL

550                                                599
KDTKNKVWKK NIISKENINL LNDGMQQVVN KTHKEDIYRS YANLIGKSGT

600                                                649
AELKMKQGES GRQIGWFISY DKDNPNMMMA INVKDVQDKG MASYNAKISG

650                668
KVYDELYENG NKKYDIDE
```

PBP2A-aur[s] -- a soluble (i.e., not membrane bound) form of PBP2A-aur.

PBP2A-epi -- A PBP2A protein isolated from *S. epidermidis* strain WT55 comprising the amino acid (single letter designations) sequence:

```
1                                                49
MKKIKIVPLI LIVVVVGFGI YFYASKDKEI NNTIDAIEDK NFKQVYKDSS

50                                               99
YISKSDNGEV EMTERPIKIY NSLGVKDINI QDRKIKKVSK NKKRVDAQYK

100                                             149
IKTNYGNIDR NVQFNFVKED GMWKLDWDHS VIIPGMQKDQ SIHIENLKSE

150                                             199
RGKILDRNNV ELANTGTAYE IGIVPKNVSK KDYKAIAKEL SISEDYIKQQ

200                                             249
MDQNWVQDDT FVPLKTVKKM DEYLSDFAKK FHLTTNETES RNYPLGKATS

250                                             299
HLLGYVGPIN SEELKQKEYK GYKDDAVIGK KGLEKLYDKK LQHEDGYRVT

300                                             349
IVDDNSNTIA HTLIEKKKKD GKDIQLTIDA KVQKSIYNNM KNDYGSGTAI

350                                             399
HPQTGELLAL VSTPSYDVYP FMYGMSNEEY NKLTEDKKEP LLNKFQITTS
```

```
400                                                    449
PGSTQKILTA MIGLNNKTLD DKTSYKIDGK GWQKDKSWGG YNVTRYEVVN

450                                                    499
GNIDLKQAIE SSDNIFFARV ALELGSKKFE KGMKKLGVGE DIPSDYPFYN

500                                                    549
AQISNKNLDN EILLADSGYG QGEILINPVQ ILSIYSALEN NGNINAPHLL

550                                                    599
KDTKNKVWKK NIISKENINL LTDGMQQVVN KTHKEDIYRS YANLIGKSGT

600                                                    649
AELKMKQGET GRQIGWFISY DKDNPNMMMA INVKDVQDKG MASYNAKISG

650                 668
KVYDELYENG NKKYDIDE
```

PBP2A-epi$^s$ -- a soluble (i.e., not membrane-bound) form of PBP2A-epi.

PBP2A-mat -- A PBP2A protein isolated from *S. aureus* strain TK784 comprising the amino acid (single letter designations) sequence:

```
1                                                      49
MKKIKIVPLI LIVVVVGFGI YFYASKDKEI NNTIDAIEDK NFKQVYKDSS

50                                                     99
YISKSDNGEV EMTERPIKIY NSLGVKDINI QDRKIKKVSK NKKRVDAQYK
```

```
100                                              149
IKTNYGNIDR NVQFNFVKED GMWKLDWDHS VIIPGMQKDQ SIHIENLKSE

150                                              199
RGKILDRNNV ELANTGTHMR LGIVPKNVSK KDYKAIAKEL SISEDYINNK

200                                              249
WIKIGYKMIP SFHFKTVKKM DEYLSDFAKK FHLTTNETES RNYPLGKATS

250                                              299
HLLGYVGPIN SEELKQKEYK GYKDDAVIGK KGLEKLYDKK LQHEDGYRVT

300                                              349
IVRVDDNSNT IAHTLIEKKK KDGKDIQLTI DAKVQKSIYN NMKNDYGSGT

350                                              399
AIHPQTGELL ALVSTPSYDV YPFMYGMSNE EYNKLTEDKK EPLLNKFQIT

400                                              449
TSPGSTQKIL TAMIGLNNKT LDDKTSYKID GKGWQKDKSW GGYNVTRYEV

450                                              499
VNGNIDLKQA IESSDNIFFA RVALELGSKK FEKGMKKLGV GEDIPSDYPF

500                                              549
YNAQISNKNL DNEILLADSG YGQGEILINP VQILSIYSAL ENNGNINAPH

550                                              599
LLKDTKNKVW KKNIISKENI NLLNDGMQQV VNKTHKEDIY RSYANLIGKS

600                                              649
GTAELKMKQG ETGRQIGWFI SYDKDNPNMM MAINVKDVQD KGMASYNAKI

650                  670
SGKVYDELYE NGNKKYDIDE
```

PBP2A-mat$^s$ -- a soluble (i.e., not membrane-bound) form of PBP2A-mat.

PBP2A-27R -- the modified penicillin binding protein isolated from Staphylococcus aureus strain 27R, the primary structure of which comprises the amino acid sequence:

| | | |
|---|---|---|
| 1 | MetLysLysIleLysIleValProLeuIleLeuIleValValValVal | 16 |
| 17 | GlyPheGlyIleTyrPheTyrAlaSerLysAspLysGluIleAsnAsn | 32 |
| 33 | ThrIleAspAlaIleGluAspLysAsnPheLysGlnValTyrLysAsp | 48 |
| 49 | SerSerTyrIleSerLysSerAspAsnGlyGluValGluMetThrGlu | 64 |
| 65 | ArgProIleLysIleTyrAsnSerLeuGlyValLysAspIleAsnIle | 80 |
| 81 | GlnAspArgLysIleLysLysValSerLysAsnLysLysArgValAsp | 96 |
| 97 | AlaGlnTyrLysIleLysThrAsnTyrGlyAsnIleAspArgAsnVal | 112 |
| 113 | GlnPheAsnPheValLysGluAspGlyMetTrpLysLeuAspTrpAsp | 128 |
| 129 | HisSerValIleIleProGlyMetGlnLysAspGlnSerIleHisIle | 144 |
| 145 | GluAsnLeuLysSerGluArgGlyLysIleLeuAspArgAsnAsnVal | 160 |
| 161 | GluLeuAlaAsnThrGlyThrAlaTyrGluIleGlyIleValProLys | 176 |
| 177 | AsnValSerLysLysAspTyrLysAlaIleAlaLysGluLeuSerIle | 192 |
| 193 | SerGluAspTyrIleLysGlnGlnMetAspGlnAsnTrpValGlnAsp | 208 |
| 209 | AspThrPheValProLeuLysThrValLysLysMetAspGluTyrLeu | 224 |
| 225 | SerAspPheAlaLysLysPheHisLeuThrThrAsnGluThrGluSer | 240 |
| 241 | ArgAsnTyrProLeuGluLysAlaThrSerHisLeuLeuGlyTyrVal | 256 |
| 257 | GlyProIleAsnSerGluGluLeuLysGlnLysGluTyrLysGlyTyr | 272 |
| 273 | LysAspAspAlaValIleGlyLysLysGlyLeuGluLysLeuTyrAsp | 288 |
| 289 | LysLysLeuGlnHisGluAspGlyTyrArgValThrIleValAspAsp | 304 |

305 AsnSerAsnThrIleAlaHisThrLeuIleGluLysLysLysLysAsp 320
321 GlyLysAspIleGlnLeuThrIleAspAlaLysValGlnLysSerIle 336
337 TyrAsnAsnMetLysAsnAspTyrGlySerGlyThrAlaIleHisPro 352
353 GlnThrGlyGluLeuLeuAlaLeuValSerThrProSerTyrAspVal 368
369 TyrProPheMetTyrGlyMetSerAsnGluGluTyrAsnLysLeuThr 384
385 GluAspLysLysGluProLeuLeuAsnLysPheGlnIleThrThrSer 400
401 ProGlySerThrGlnLysIleLeuThrAlaMetIleGlyLeuAsnAsn 416
417 LysThrLeuAspAspLysThrSerTyrLysIleAspGlyLysGlyTrp 432
433 GlnLysAspLysSerTrpGlyGlyTyrAsnValThrArgTyrGluVal 448
449 ValAsnGlyAsnIleAspLeuLysGlnAlaIleGluSerSerAspAsn 464
465 IlePhePheAlaArgValAlaLeuGluLeuGlySerLysLysPheGlu 480
481 LysGlyMetLysLysLeuGlyValGlyGluAspIleProSerAspTyr 496
497 ProPheTyrAsnAlaGlnIleSerAsnLysAsnLeuAspAsnGluIle 512
513 LeuLeuAlaAspSerGlyTyrGlyGlnGlyGluIleLeuIleAsnPro 528
529 ValGlnIleLeuSerIleTyrSerAlaLeuGluAsnAsnGlyAsnIle 544
545 AsnAlaProHisLeuLeuLysAspThrLysAsnLysValTrpLysLys 560
561 AsnIleIleSerLysGluAsnIleAsnLeuLeuThrAspGlyMetGln 576
577 GlnValValAsnLysThrHisLysGluAspIleTyrArgSerTyrAla 592
593 AsnLeuIleGlyLysSerGlyThrAlaGluLeuLysMetLysGlnGly 608
609 GluThrGlyArgGlnIleGlyTrpPheIleSerTyrAspLysAspAsn 624
625 ProAsnMetMetMetAlaIleAsnValLysAspValGlnAspLysGly 640
641 MetAlaSerTyrAsnAlaLysIleSerGlyLysValTyrAspGluLeu 656
657 TyrGluAsnGlyAsnLysLysTyrAspIleAspGluEnd 668,

or a functional derivative thereof.

PBP2A-27R$^s$ -- a soluble, i.e. non-membrane associated, derivative of the PBP2A-27R protein which retains the penicillin binding activity of the PBP2A-27R molecule.

PBP2A-27R-CP -- a PBP2A-27R molecule or a functional derivative thereof which is covalently linked to a chelating peptide.

PBP2A-CP -- a PBP2A molecule (either soluble or membrane bound form), or functional derivative thereof, covalently linked to a chelating peptide

PCR -- an abbreviation for polymerase chain reaction.

Phe -- the amino acid phenylalanine.

Plasmid -- an extrachromosomal self-replicating genetic element.

PMSF -- an abbreviation for phenylmethylsulfonyl fluoride.

Pro -- the amino acid proline.

Promoter-Operator Region -- a DNA sequence which facilitates and regulates the initiation of transcription of a given gene.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- any recombinant DNA cloning vector in which a promoter has been incorporated to drive expression of a foreign gene.

| | |
|---|---|
| Replicon -- | A DNA sequence that controls and allows for autonomous replication of a plasmid or other vector. |
| RNA -- | ribonucleic acid. |
| RP-HPLC -- | an abbreviation for reversed-phase high performance liquid chromatography. |
| Ser -- | the amino acid serine. |
| Thr -- | the amino acid threonine. |
| Transcription -- | the process whereby information contained in a nucleotide sequence of DNA is transferred to a complementary RNA sequence. |
| Translation -- | the process whereby the genetic information of messenger RNA is used to specify and direct the synthesis of a polypeptide chain. |
| Tris -- | an abbreviation for tris(hydroxymethyl)aminomethane. |
| Trp -- | the amino acid tryptophan. |
| Tyr -- | the amino acid tyrosine. |
| Val -- | the amino acid valine. |
| Vector -- | a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors. |

## DETAILED DESCRIPTION OF THE INVENTION

The instant invention provides a compound comprising a penicillin binding protein isolated from the methicillin resistant Staphyloccous strain 27R, said compound comprising the amino acid sequence of the formula 1:

| | | |
|---|---|---|
| 1 | MetLysLysIleLysIleValProLeuIleLeuIleValValValVal | 16 |
| 17 | GlyPheGlyIleTyrPheTyrAlaSerLysAspLysGluIleAsnAsn | 32 |
| 33 | ThrIleAspAlaIleGluAspLysAsnPheLysGlnValTyrLysAsp | 48 |
| 49 | SerSerTyrIleSerLysSerAspAsnGlyGluValGluMetThrGlu | 64 |
| 65 | ArgProIleLysIleTyrAsnSerLeuGlyValLysAspIleAsnIle | 80 |
| 81 | GlnAspArgLysIleLysLysValSerLysAsnLysLysArgValAsp | 96 |
| 97 | AlaGlnTyrLysIleLysThrAsnTyrGlyAsnIleAspArgAsnVal | 112 |
| 113 | GlnPheAsnPheValLysGluAspGlyMetTrpLysLeuAspTrpAsp | 128 |
| 129 | HisSerValIleIleProGlyMetGlnLysAspGlnSerIleHisIle | 144 |
| 145 | GluAsnLeuLysSerGluArgGlyLysIleLeuAspArgAsnAsnVal | 160 |
| 161 | GluLeuAlaAsnThrGlyThrAlaTyrGluIleGlyIleValProLys | 176 |
| 177 | AsnValSerLysLysAspTyrLysAlaIleAlaLysGluLeuSerIle | 192 |
| 193 | SerGluAspTyrIleLysGlnGlnMetAspGlnAsnTrpValGlnAsp | 208 |
| 209 | AspThrPheValProLeuLysThrValLysLysMetAspGluTyrLeu | 224 |
| 225 | SerAspPheAlaLysLysPheHisLeuThrThrAsnGluThrGluSer | 240 |
| 241 | ArgAsnTyrProLeuGluLysAlaThrSerHisLeuLeuGlyTyrVal | 256 |
| 257 | GlyProIleAsnSerGluGluLeuLysGlnLysGluTyrLysGlyTyr | 272 |
| 273 | LysAspAspAlaValIleGlyLysLysGlyLeuGluLysLeuTyrAsp | 288 |
| 289 | LysLysLeuGlnHisGluAspGlyTyrArgValThrIleValAspAsp | 304 |
| 305 | AsnSerAsnThrIleAlaHisThrLeuIleGluLysLysLysLysAsp | 320 |
| 321 | GlyLysAspIleGlnLeuThrIleAspAlaLysValGlnLysSerIle | 336 |
| 337 | TyrAsnAsnMetLysAsnAspTyrGlySerGlyThrAlaIleHisPro | 352 |

```
353 GlnThrGlyGluLeuLeuAlaLeuValSerThrProSerTyrAspVal 368
369 TyrProPheMetTyrGlyMetSerAsnGluGluTyrAsnLysLeuThr 384
385 GluAspLysLysGluProLeuLeuAsnLysPheGlnIleThrThrSer 400
401 ProGlySerThrGlnLysIleLeuThrAlaMetIleGlyLeuAsnAsn 416
417 LysThrLeuAspAspLysThrSerTyrLysIleAspGlyLysGlyTrp 432
433 GlnLysAspLysSerTrpGlyGlyTyrAsnValThrArgTyrGluVal 448
449 ValAsnGlyAsnIleAspLeuLysGlnAlaIleGluSerSerAspAsn 464
465 IlePhePheAlaArgValAlaLeuGluLeuGlySerLysLysPheGlu 480
481 LysGlyMetLysLysLeuGlyValGlyGluAspIleProSerAspTyr 496
497 ProPheTyrAsnAlaGlnIleSerAsnLysAsnLeuAspAsnGluIle 512
513 LeuLeuAlaAspSerGlyTyrGlyGlnGlyGluIleLeuIleAsnPro 528
529 ValGlnIleLeuSerIleTyrSerAlaLeuGluAsnAsnGlyAsnIle 544
545 AsnAlaProHisLeuLeuLysAspThrLysAsnLysValTrpLysLys 560
561 AsnIleIleSerLysGluAsnIleAsnLeuLeuThrAspGlyMetGln 576
577 GlnValValAsnLysThrHisLysGluAspIleTyrArgSerTyrAla 592
593 AsnLeuIleGlyLysSerGlyThrAlaGluLeuLysMetLysGlnGly 608
609 GluThrGlyArgGlnIleGlyTrpPheIleSerTyrAspLysAspAsn 624
625 ProAsnMetMetMetAlaIleAsnValLysAspValGlnAspLysGly 640
641 MetAlaSerTyrAsnAlaLysIleSerGlyLysValTyrAspGluLeu 656
657 TyrGluAsnGlyAsnLysLysTyrAspIleAspGluEnd 668,
```

and functional derivatives thereof.

The invention further provides DNA compounds the in vivo or in vitro transcription and translation of which results in the production of compounds of the formula 1, said DNA compound comprising the deoxyribonucleotide compound of the formula 2.

```
1  ATGAAAAAGATAAAAATTGTTCCACTTATTTTAATAGTTGTAGTTGTCGGGTTTGGTATA 60
61 TATTTTTATGCTTCAAAAGATAAAGAAATTAATAATACTATTGATGCAATTGAAGATAAA 120
```

121 AATTTCAAACAAGTTTATAAAGATAGCAGTTATATTTCTAAAAGCGATAATGGTGAAGTA 180

181 GAAATGACTGAACGTCCGATAAAAATATATAATAGTTTAGGCGTTAAAGATATAAACATT 240

241 CAGGATCGTAAAATAAAAAAAGTATCTAAAAATAAAAAACGAGTAGATGCTCAATATAAA 300

301 ATTAAAACAAACTACGGTAACATTGATCGCAACGTTCAATTTAATTTTGTTAAAGAAGAT 360

361 GGTATGTGGAAGTTAGATTGGGATCATAGCGTCATTATTCCAGGAATGCAGAAAGACCAA 420

421 AGCATACATATTGAAAATTTAAAATCAGAACGTGGTAAAATTTTAGACCGAAACAATGTG 480

481 GAATTGGCCAATACAGGAACAGCATATGAGATAGGCATCGTTCCAAAGAATGTATCTAAA 540

541 AAAGATTATAAAGCAATCGCTAAAGAACTAAGTATTTCTGAAGACTATATCAAACAACAA 600

601 ATGGATCAAAATTGGGTACAAGATGATACCTTCGTTCCACTTAAAACCGTTAAAAAAATG 660

661 GATGAATATTTAAGTGATTTCGCAAAAAAATTTCATCTTACAACTAATGAAACAGAAAGT 720

721 CGTAACTATCCTCTAGAAAAAGCGACTTCACATCTATTAGGTTATGTTGGTCCCATTAAC 780

781 TCTGAAGAATTAAAACAAAAAGAATATAAAGGCTATAAAGATGATGCAGTTATTGGTAAA 840

841 AAGGGACTCGAAAAACTTTACGATAAAAAGCTCCAACATGAAGATGGCTATCGTGTCACA 900

901 ATCGTTGACGATAATAGCAATACAATCGCACATACATTAATAGAGAAAAAGAAAAAAGAT 960

961 GGCAAAGATATTCAACTAACTATTGATGCTAAAGTTCAAAAGAGTATTTATAACAACATG 1020

1021 AAAAATGATTATGGCTCAGGTACTGCTATCCACCCTCAAACAGGTGAaTTATTAGCACTT 1080

```
1081  GTAAGCACACCTTCATATGACGTCTATCCATTTATGTATGGCATGAGTAACGAAGAATAT 1140

1141  AATAAATTAACCGAAGATAAAAAGAACCTCTGCTCAACAAGTTCCAGATTACAACTTCA 1200

1201  CCAGGTTCAACTCAAAAAATATTAACAGCAATGATTGGGTTAAATAACAAAACATTAGAC 1260

1261  GATAAAACAAGTTATAAAATCGATGGTAAAGGTTGGCAAAAAGATAAATCTTGGGGTGGT 1320

1321  TACAACGTTACAAGATATGAAGTGGTAAATGGTAATATCGACTTAAAACAAGCAATAGAA 1380

1381  TCATCAGATAACATTTTCTTTGCTAGAGTAGCACTCGAATTAGGCAGTAAGAAATTTGAA 1440

1441  AAAGGCATGAAAAAACTAGGTGTTGGTGAAGATATACCAAGTGATTATCCATTTTATAAT 1500

1501  GCTCAAATTTCAAACAAAAATTTAGATAATGAAATATTATTAGCTGATTCAGGTTACGGA 1560

1561  CAAGGTGAAATACTGATTAACCCAGTACAGATCCTTTCAATCTATAGCGCATTAGAAAAT 1620

1621  AATGGCAATATTAACGCACCTCACTTATTAAAAGACACGAAAAACAAAGTTTGGAAGAAA 1680

1681  AATATTATTTCCAAAGAAAATATCAATCTATTAACTGATGGTATGCAACAAGTCGTAAAT 1740

1741  AAAACACATAAAGAAGATATTTATAGATCTTATGCAAACTTAATTGGCAAATCCGGTACT 1800

1801  GCAGAACTCAAAATGAAACAAGGAGAAACTGGCAGACAAATTGGGTGGTTTATATCATAT 1860

1861  GATAAAGATAATCCAAACATGATGATGGCTATTAATGTTAAAGATGTACAAGATAAAGGA 1920

1921  ATGGCTAGCTACAATGCCAAAATCTCAGGTAAAGTGTATGATGAGCTATATGAGAACGGT 1980

1981  AATAAAAAATACGATATAGATGAATAA  2007
```

and functional equivalents thereof.

It will be readily appreciated by those of ordinary skill in the art that the DNA compound of formula 2 is only one of a large but definite body of DNA sequences which will encode the PBP2A-27R protein of formula 1. The natural degeneracy of the genetic code is well documented. stryer, L. Biochemistry, 2d ed. (1981) W.H. Freeman and Company, San Francisco, pp. 619-635. Therefore, a number of DNA and corresponding RNA sequences may be created, either by synthetic methodology or site-specific mutagenesis of the DNA compound of formula 1, the in vitro or in vivo transcription and/or translation of which will produce compounds of formula 1.

A sample of the methicillin resistant Staphylococcus aureus strain 27R was obtained from Dr. Richard Novick (NYC Public Health Department). (Staphylococcus aureus strain 27R is available from Chyun-Yeh Wu, Eli Lilly & Co., Indianapolis, IN). The cells were grown in TY broth and used as a source of S. aureus DNA containing the mecA-27R gene which encodes the penicillin binding protein PBP2A-27R that is responsible for methicillin resistance in this bacterium. The cells were isolated by centrifugation, lysed and the DNA isolated by phenol:chloroform extraction and centrifugation.

This DNA was used to construct a DNA library in the λphage EMBL3. The DNA was digested with the restriction endonuclease SauIIIA. Samples of the SauIIIA partially digested DNA from example 1-C were ligated to EMBL3 phage arms (Stratagene, 11099 North Torrey Pines Road, La Jolla, CA 92037) in substantial accordance with the conditions prescribed by the manufacturer. The ligated DNA was then packaged (Gigapack II Gold Packaging Extract, Stratagene, 11099 North Torrey Pines Road, La Jolla, CA 92037) into lambda phage heads. The packaging extracts were used to infect Escherichia coli strain CJ236 provided by Stratagene. The infected E. coli cells were plated in soft agar overlays on agar plates as described (stratagene). Plaques which developed were screened for the presence of the mecA-27R gene.

The plaques were screened for the mecA-27R gene by hybridization in substantial accordance with the procedure described for plaque hybridization in Maniatis et al. ("Molecular Cloning, A Laboratory Manual, 1982, Cold Spring Harbor Laboratories). The hybridization probe used to screen the library was generated by the polymerase chain reaction (PCR). PCR oligonucleotide primers were synthesized on an automated DNA synthesizer such as an Applied Biosystems Model 380A or 380B (Applied Biosystems, Foster City, CA) as suggested by the manufacturer. The sequence of the primers, illustrated below, was based on the DNA sequence of a mecA gene published by Song, et al. (1987, FEBS Letters, Vol. 221(1) pages 167-171).

5' - GTTGTAGTTGTCGGGTTTGG - 3'

5' - GAGGGTGGATAGCAGTACCTGAGCC - 3'

These oligonucleotide primers, when used in a PCR reaction under the conditions specified by the manufacturer of the DNA Thermal Cycler (Perkin Elmer Cetus), amplified the 5′ end (~1,000 bases) of the mecA-27R gene from Staphyloccus aureus strain 27R and other MRS strains.

Plaques which exhibited a positive hybridization response with the probe described above were purified. DNA was extracted from the phage responsible for formation of these plaques of interest (phage isolate #1A). The purified DNA was digested with a selection of restriction enzymes. The mecA-27R gene was found to lie completely within an approximately 4.0 kb HindIII restriction fragment. This HindIII restriction fragment was the source of the mecA-27R gene for further studies.

The approximately 4.0 kb HindIII restriction fragment was cleaved by the restriction enzyme XbaI and the resultant fragments were cloned into M13mp18 and M13mp19 for DNA sequencing. The entire DNA sequence of the mecA-27R gene from Staphylococcccus aureus strain 27R was determined by the sanger dideoxy-mediated chain-termination method (Sanger et al., 1977, Proceedings of the National Academy of Science USA, Vol. 74, page 5463) using the TaqTrack Sequencing System (Promega Corporation, 2800 Woods Hollow Road, Madison, WI 53711-5399). The DNA sequence is presented in Figure 23. The amino acid sequence deduced from that DNA sequence is presented in Figure 24.

DNA isolated from phage #1A was digested to completion with the restriction endonuclease HindIII, electrophoresed, and transferred to a DEAE cellulose membrane. The DNA was removed from the membrane in high salt buffer and precipitated with ethanol. The DNA was pelleted and dried. This DNA contained the desired 4.0 kb HindIII restriction fragment containing the mecA-27R gene from Staphylococcus aureus strain 27R.

The DNA fragment was ligated into the HindIII digested pUC19 plasmid by techniques well known in the art. The ligated DNA constituted the desired plasmids pEWSA8 and pEWSA37 along with other ligation products. The only difference in these plasmids is the orientation of the HindIII restriction fragment containing the mecA-27R gene. Restriction/function maps of plasmids pEWSA8 and pEWSA37 are provided in Figures 2 and 3 respectively. This ligation reaction was used to transform competent Escherichia coli K12 DH5α (MAX Efficiency) (purchased from Bethesda Research Laboratories, Gaithersburg, MD). Post-transformation, the cells were distributed to agar plates containing ampicillin (100 μg/ml) and the plates were incubated overnight at 37°C.

Escherichia coli transformants containing a plasmid with an insert were identified by inactivation of the β-galactosidase gene present on pUC19. Those transformants harboring an insert in the HindIII site of pUC19 formed colorless colonies while those uninterrupted formed blue colonies. Miniprep plasmid DNA prepared from colorless colonies was screened by restriction endonuclease digestion in substantial accordance with the procedures described by Maniatis et al.(1982, "Molecular Cloning, A Laboratory Manual, Cold Spring Harbor

Laboratory). One isolate was recovered which contained plasmid pEWSA8 and another contained plasmid pEWSA37. The cells containing these plasmids are designated DH5α/pEWSA8 and DH5α/pEWSA37 respectively. E. Coli K12 DH5α/pEWSA37 has been deposited with the Northern Regional Research Laboratories (Peoria, Illinois) National Culture Collection and is available under the accession number B-18753.

Escherichia coli transformants obtained with plasmids pEWSA37 or pEWSA8 express detectable levels of the active mecA-27R gene product (PBP2A-27R). In these transformants, mecA-27R gene expression is driven by the native Staphylococcus aureus promoter. Matsuhashi,M., et al. (1986) J.Bacteriol 167:975-980. Cell membrane preparations from E. coli, cells transformed with pEWSA37 and pEWSA8 were analyzed for the presence of the mecA-27R gene product (PBP2A-27R) in the following manner.

The cells were broken by passing three times through a French press at 16,000 lb/in$^2$ (SLM Aminco, Urbana, Illinois). After centrifugation to remove unbroken bacterial cells, supernatants were loaded onto a discontinuous sucrose gradient. After centrifugation (18 hours at 135,000 x g), the fraction containing the inner cell membrane and associated proteins was collected.

The cell membrane preparation was analyzed for PBP's by labeling with $^{125}$I-Penicillin V (IPV) in substantial accordance with the procedure described by Preston et al., (Antimicrobial Agents and Chemotherapy, 1990, pages 718-721). A 4 μl aliquot of membrane preparation was incubated with 2 μl of sodium clavulanate (concentration of sodium clavulanate ranged from 1 to 243 μg/ml) and 6 μl of $^{125}$IPV (96 μg/ml) for 15 minutes at 35°C. The reaction was stopped by the addition of 12 μl of blue mix (120 mg of unlabeled PenV per ml-20% sarcosyl-polyacrylamide gel electrophoresis sample buffer, 1:2:12). Proteins were separated by sodium dodecyl sulfate-polyacrylamide gel electrophoresis(SDS-PAGE), using 8% acrylamide-0.21% N,N′-methylene-bisacrylamide. Dried gels were exposed to Kodak X-OMAT film without fluorographic enhancement at 24°C for 8 to 24 hours. Escherichia coli membrane preparations which did not contain plasmid pEWSA37 or plasmid pEWSA8 did not exhibit a radiolabeled band at a position consistent with PBP2A-27R. In contrast, transformed cells carrying pEWSA37 or pEWSA8 did exhibit a distinct radioactive band at a position consistent with PBP2A from S. aureus 27R. The intensity of that band increased with increasing levels of sodium clavulanate used in the reaction. Therefore E. coli cells were recovered which produced the Staphylococcus aureus PBP2A-27R protein. Reproductions of the autoradiograms of these gels appear in Figure 26.

The invention further provides polypeptide compounds which are derivatives of the polypeptide compound of formula 1 which lack the naturally occurring membrane association region of the molecule, said polypeptide compounds comprising compounds of the formula 3:

Met-X-(Pro)$_n$-TyrAlaSerLysAspLysGluIleAsnAsn
ThrIleAspAlaIleGluAspLysAsnPheLysGlnValTyrLysAsp
SerSerTyrIleSerLysSerAspAsnGlyGluValGluMetThrGlu
ArgProIleLysIleTyrAsnSerLeuGlyValLysAspIleAsnIle
GlnAspArgLysIleLysLysValSerLysAsnLysLysArgValAsp
AlaGlnTyrLysIleLysThrAsnTyrGlyAsnIleAspArgAsnVal
GlnPheAsnPheValLysGluAspGlyMetTrpLysLeuAspTrpAsp
HisSerValIleIleProGlyMetGlnLysAspGlnSerIleHisIle
GluAsnLeuLysSerGluArgGlyLysIleLeuAspArgAsnAsnVal
GluLeuAlaAsnThrGlyThrAlaTyrGluIleGlyIleValProLys
AsnValSerLysLysAspTyrLysAlaIleAlaLysGluLeuSerIle
SerGluAspTyrIleLysGlnGlnMetAspGlnAsnTrpValGlnAsp
AspThrPheValProLeuLysThrValLysLysMetAspGluTyrLeu
SerAspPheAlaLysLysPheHisLeuThrThrAsnGluThrGluSer
ArgAsnTyrProLeuGluLysAlaThrSerHisLeuLeuGlyTyrVal

GlyProIleAsnSerGluGluLeuLysGlnLysGluTyrLysGlyTyr
LysAspAspAlaValIleGlyLysLysGlyLeuGluLysLeuTyrAsp
LysLysLeuGlnHisGluAspGlyTyrArgValThrIleValAspAsp
AsnSerAsnThrIleAlaHisThrLeuIleGluLysLysLysLysAsp
GlyLysAspIleGlnLeuThrIleAspAlaLysValGlnLysSerIle
TyrAsnAsnMetLysAsnAspTyrGlySerGlyThrAlaIleHisPro
GlnThrGlyGluLeuLeuAlaLeuValSerThrProSerTyrAspVal
TyrProPheMetTyrGlyMetSerAsnGluGluTyrAsnLysLeuThr
GluAspLysLysGluProLeuLeuAsnLysPheGlnIleThrThrSer
ProGlySerThrGlnLysIleLeuThrAlaMetIleGlyLeuAsnAsn
LysThrLeuAspAspLysThrSerTyrLysIleAspGlyLysGlyTrp
GlnLysAspLysSerTrpGlyGlyTyrAsnValThrArgTyrGluVal
ValAsnGlyAsnIleAspLeuLysGlnAlaIleGluSerSerAspAsn
IlePhePheAlaArgValAlaLeuGluLeuGlySerLysLysPheGlu
LysGlyMetLysLysLeuGlyValGlyGluAspIleProSerAspTyr
ProPheTyrAsnAlaGlnIleSerAsnLysAsnLeuAspAsnGluIle
LeuLeuAlaAspSerGlyTyrGlyGlnGlyGluIleLeuIleAsnPro
ValGlnIleLeuSerIleTyrSerAlaLeuGluAsnAsnGlyAsnIle
AsnAlaProHisLeuLeuLysAspThrLysAsnLysValTrpLysLys
AsnIleIleSerLysGluAsnIleAsnLeuLeuThrAspGlyMetGln
GlnValValAsnLysThrHisLysGluAspIleTyrArgSerTyrAla
AsnLeuIleGlyLysSerGlyThrAlaGluLeuLysMetLysGlnGly
GluThrGlyArgGlnIleGlyTrpPheIleSerTyrAspLysAspAsn
ProAsnMetMetMetAlaIleAsnValLysAspValGlnAspLysGly
MetAlaSerTyrAsnAlaLysIleSerGlyLysValTyrAspGluLeu
TyrGluAsnGlyAsnLysLysTyrAspIleAspGluEnd

wherein n= 0 or 1, X = Val or a Gly-CP where CP is a chelating peptide of the formula:

$$(His)_x-(A)_y-(His)_z$$

where A is an amino acid

$$x = 1\text{-}10$$
$$y = 0\text{-}4$$
$$z = 1\text{-}10$$

and monomers, dimers and trimers thereof wherein each monomer unit is the same or different, and functional derivatives thereof.

The one constant in the clinical experience with staphylococcal infections has been the ability of this organism to hurdle virtually every antibiotic obstacle placed in its path. Prior to the advent of penicillin, the prognosis of patients with Staph. infections was poor. Prior to the 1940's, Staphylococci were almost universally susceptible to penicillin. Such susceptibility, however, was to be short lived. Thornsberry, C. (1988) J. of Antimicrobial Chemotherapy 21: Suppl. C, p. 9-16. Reports of penicillin resistant strains of staphylococci increased through the 1940's. These early strains of penicillin resistant staphylococci were shown to produce an exoenzyme (β-lactamase) capable in inactivating β-lactam antibiotics. The advent of methicillin and its derivatives in the 1960's provided effective weapons against penicillin resistant strains of staphlococci. However, this advance was again thwarted by reports of staphylococci resistant to these antibiotics. These staphylococci present significant concerns to the clinician who finds his antibiotic arsenal antiquated more rapidly than new antibiotic compounds

are discovered

The rational design of antibiotic compounds useful against staphylococcal infections is dependent upon the ability to characterize the binding domains of penicillin binding proteins produced by methicillin resistant strains of staphylococci. The characteristics of such potential antibiotic binding sites is determined by x-ray crystallography studies. A major obstacle to performing x-ray crystallography studies on the penicillin binding proteins, particularly the PBP2As has been the characteristic membrane-bound nature of these proteins. It is therefore, desirable to produce these penicillin binding proteins in large quantities in soluble form from which they can be used in x-ray crystallography studies. The instant invention provides such soluble penicillin-active forms of the PBP2A-27R protein and other PBP2A proteins.

As previously stated, the formation of the peptidoglycan matrix is created by transglycosylation and peptide cross-linking. Penicillin inhibits the peptide cross-linking step. Penicillin inactivates the enzymes involved in peptide cross-linking by creating acylated addition products which are sufficiently stable to be isolated by SDS gel electrophoresis. These enzymes, i.e. penicillin binding proteins, show widely varying affinity to penicillin and other β-lactam antibiotics. Ghuysen, J.M. (1990) Biotechnology and Applied Biochemistry 12: 468-472. Gene cloning and sequencing, structural studies, and homology searches indicate that the serine β-lactamases, the low molecular weight penicillin binding proteins, and the high molecular weight PBP's (particularly their penicillin binding domains) form a superfamily of serine peptidases. They all comprise a "penicillin-interactive domain" which possesses a similar overall three dimensional structure. Ghuysen, J.M., et al., 1988, Biochemical Journal 250: 313-324. This "penicillin interactive domain" consists of two regions: one is of the "all α" type structure, and the other has a central core of a 5-stranded β-sheet protected by α-helices on both faces. Id. The active site serine occurs approximately 60 residues downstream of the amino terminus of the domain. This serine belongs to a conserved tetrad Ser-Xaa-Xaa-Lys and is located at the amino terminus of one α-helix of the all α region. Id. A conserved triad His-Thr-Gly, Lys-Thr-Gly or Lys-ser-Gly occurs 60 residues upstream of the carboxy terminus of the domain. This triad is on the innermost strand of the β-sheet which forms one side of the active site and, presumably provides hydrogen bond interactions with substrates and/or inhibitors. Id.

Conservation of this "penicillin-interactive domain" in PBP2A analogs will maintain the penicillin binding nature of the protein. However, the flanking amino acid sequences necessary to preserve the penicillin binding nature of the intact PBP structural protein were heretofore unknown. The unpredictable nature of protein chemistry fails to provide guidance as to which modifictions in these flanking regions will result in the formation of an inactive or active penicillin binding protein. Mere characterization of the active site residues does not possess one of sufficient knowledge to know which of the flanking sequences is necessary to preserve the overall three dimensional structure of the penicillin binding protein. The instant invention provides a soluble form of the mecA-27R product which preserves this penicillin interactive domain, yet retains the penicillin binding protein functionality of the complete PBP2A-27R molecule.

Deletions in the DNA sequence encoding the PBP2A-27R molecule up to approximately base pair 734 will maintain the production of a functional derivative of a PBP2A molecule. A functional derivative of a PBP2A molecule may be defined as a PBP2A molecule which has the same or similar affinity for penicillin or β-lactam antibiotics as the naturally occurring form of the PBP2A molecule and retains the enzymatic function of the natural PBP2A molecule yet possesses a different primary amino acid sequence. It will be readily apparent to those of ordinary skill in the art that the design of antibiotic compunds useful against MRS strains which produce these modified PBP2A molecules, that maintenance of full enzymatic activity of the PBP2A molecule is not necessary. One need only maintain the proper three dimensional conformation of the active site of the PBP2A molecule in order to characterize which of the particular test compounds under study will bind to these PBP2A proteins and inactivate them. In the preferred practice of the invention, the first 734 bp of the gene are removed taking advantage of the naturally occurring XbaI site. In the most preferred practice of the invention, approximately the first 66 bp are removed to maintain a functional derivative of the PBP2A molecule. Therefore, sequential deletions in the coding sequence up to approximately base pair 734 of the naturally occurring DNA sequence of the mecA-27R molecule will result in the production of a functional derivative of the PBP2A molecule. Similarly, the high degree homology among known PBP2A molecules indicates that similar deletions in the coding sequences of these alternate PBP2As will also result in soluble functional derivatives of these PBP2As.

The instant invention provides soluble forms of the PBP2A proteins appropriate for x-ray crystallography studies. Information gained from these studies will provide insight into the structure of the penicillin binding proteins derived from methicillin resistant staphylococci which have a reduced affinity for the binding of penicillin. This information, combined with the fact that the transmembrane regions of known PBP2A's are identical, will assist in providing an overall structural picture of the modified penicillin binding domains of PBP2A molecules derived from MRS strains. Such a model will provide information useful in the rational design of antibiotic compounds useful against a broad spectrum of methicillin resistant staphylococci.

A deletion from the 5′ end of the mecA-27R coding region to the NcoI restriction endonuclease cleavage

site, inserted by in vitro mutagenesis, produces a DNA sequence encoding a soluble form of the PBP2A-27R protein (PBP2A-27R[s]). Comparison of the DNA sequence encoding the PBP2A-27R protein with other published mecA sequences as shown in Figure 30 demonstrates that the DNA sequence encoding the transmembrane region (amino acids 1-22) of PBP2A-27R is identical with all known forms of mecA. Therefore, it is apparent that removal of the DNA sequence (and amino acids concomitantly in the resultant protein) in a similar manner from the genes encoding these alternate PBP2A proteins will also result in cytosolic or soluble forms of these proteins. The soluble form of these proteins may also be used in x-ray crystallography studies to characterize the antibiotic binding sites of these other PBP2As.

The mecA-27R gene product, PBP2A-27R, is a penicillin binding protein which is membrane associated. Analysis of the amino acid sequence deduced from the DNA sequence suggested that a transmembrane region was located at the amino terminus of the protein, approximately the first 22 amino acids. Therefore, in vitro site-specific mutagenesis was employed to modify the mecA-27R gene and thus remove the putative transmembrane region from PBP2A-27R. In addition to removal of the transmembrane region, a useful NcoI restriction site was incorporated at the initiation codon to allow expression in Escherichia coli based expression vectors. The new PBP2A-27R protein encoded by this modified mecA-27R gene is not associated with the cell membrane when expressed in Escherichia coli and thus is readily available for structural studies.

The mecA-27R gene cloned from Staphylococcus aureus was subcloned into M13 vectors to obtain DNA sequence and to perform in-vitro mutagenesis experiments. The in-vitro, mutagenesis experiments produced modified forms of mecA-27R which prodced proteins that lacked the transmembrane region of the protein and incorporated chelating peptides and a signal sequence at the N-termini of the new PBP2A-27R proteins.

The principles involved in vitro site specific mutagenesis are described in Kunkel, T.A. (1985) PNAS, USA 82:488-492 and Smith, M. (1985) Ann.Rev.Genet. 19:423-463. The basic protocol for this procedure is described in Current Protocols in Molecular Biology, Ausubel, F.M., et al editors, Chapter 8, Wiley Interscience, 1989, and supplements. M13mp18 RF DNA and M13mp19 RF DNA (commercially available from New England Biolabs) provided the basic vehicle for these mutagenesis experiments. Numerous other vectors and protocols may be substituted for those described herein such as those described in Current Protocols in Molecular Biology above. Restriction sites and function maps of plasmids M13mp18 RF and M13mp19 RF are shown in figures 4 and 5 respectively of the accompanying drawings. An overall schematic of the mutagenesis experiments is provided in Figure 31. These two vectors were independently digested with the HindIII and XbaI restriction endonucleases and the vector DNA isolated.

The approximately 4.0 kb HindIII-HindIII restriction fragment of pEWSA37 containing the mecA-27R gene was isolated by the following procedure. After digestion with HindIII, the resultant fragments were reported by the electrophoresis and visualized by staining with ethidium bromide. After the fragment was located, a small slit was made in the gel in front of the ~4.0 kb HindIII, and a piece of Schleicher and Schuell (Keene, NH 03431) DEAE membrane cut to the appropriate size was placed in the slit. Upon further electrophoresis, the DNA noncovalently bound to the DEAE membrane. After the fragment was bound to the DEAE membrane, the membrane was removed and rinsed with low salt buffer. The DNA was removed from the membrane by elution in a high salt buffer. The volume of the high salt-DNA solution was adjusted so that the NaCl concentration was 0.25 M, and then three volumes of cold, absolute ethanol were added to the solution. The resulting solution was mixed and placed at -70°C for 30 minutes. The solution was then centrifuged, the supernatant discarded and the pellet composed of DNA air dried. The DNA pellet was resuspended in water and ethanol precipitated once more to remove residual salt. The air dried DNA pellet was resuspended in 20 μl of water and constituted the ~4.0 kb HindIII fragment of pEWSA37. The 4.0 kb HindII-HindIII restriction fragment was further digested with the XbaI restriction endonuclease. The resulting ~1.82 kb fragment constituting approximately 738 bp of the 5′ region of the gene and an additional upstream sequence and the approximately 2.1 kb fragment constituting approximately 1269 bp of the 3′ end of the gene and additional 3′ sequence were seperated by electrophoresis on a 0.8% agarose gel and isolated as described above.

The HindIII/XbaI digested M13mp18 DNA prepared above and the 1.82 kb HindIII/XbaI restriction fragment containing the 5′ end of the mecA-27R gene were mixed and ligated in the presence of T4 DNA ligase. Competent Escherichia coli K12 JM109 ("Epicurean Coli™", commercially available from Stratagene, 3770 Tansy Street, San Diego, CA 92121) were transformed with the ligation mixture constituting plasmid DNA pEWM13-1. For more detailed descriptions and explanations of M13 procedures, see M13 Cloning/Dideoxy Sequencing Instruction Manual., Bethesda Research Laboratories [BRL], Life Technologies, Inc., Gaithersburg, MD 20877 the entire teaching of which is hereby incorporated by reference. Transformants containing an insertion of the desired approximately 1.82 kb HindIII/XbaI fragment carrying the 5′ end of the mecA-27R gene were identified by insertional inactivation of β-galactosidase activity (colorless plaque phenotype) and restriction enzyme analysis of replicative form (RF) DNA. For screening purposes, clear plaques were plugged from the plate overlay with a Pasteur pipette into 3 ml per plaque of early logarithmic growth phase E. coli K12 JM109 cells. Cultures were incubated from 6 to 18 hours at 37°C with aeration. Following this incubation, 1.5 ml of each culture was pelleted in separate 1.5 ml Eppendorf tubes. The supernatants, which contained phage particles, were decanted into fresh tubes and stored at 4°C to serve as a source of phage inoculum.

Replicative form DNA was prepared from the cell pellets in substantial accordance with the teaching of the alkaline plasmid preparation procedure of Birnboim and Doly, 1979, Nuc. Acids Res. 7(6):1513-1523, with the following exceptions. The procedure was scaled up such that 1.5 volumes of Solutions I, II, and III are used, and the cleared lysate was extracted once with an equal volume of $CHCl_3$. The DNA was then precipitated by the addition of 0.4 volumes of isopropanol and incubated at room temperature for 20 minutes. The DNA was collected by centrifugation and then precipitated with ethanol out of 0.3 M NaOAc. Restriction analysis of the DNA thus isolated was used to confirm the presence of the approximately 1.82 kb HindIII/XbaI restriction fragment. By this method, E. coli K12 JM109/pEWM13-1 cells were identified; these cells were then used as a source of phage and plasmid pEWM13-1 for site-specific mutagenesis and DNA sequencing. Figure 6 presents a restriction/function map of phage pEWM13-1.

To obtain the opposite orientation, the HindIII/XbaI fragment containing the 5′ end of mecA-27R was cloned into M13mp19. This plasmid was useful in DNA sequencing experiments. The HindIII/XbaI digested M13mp19 DNA prepared above and the 1.82 kb HindIII/XbaI restriction fragment containing the 5′ end of the mecA-27R gene were mixed and ligated in the presence of T4 DNA ligase. Transformation of E. coli with this ligation mixture and screening of plaques was performed as described above. E. coli JM109/pEWM13-7 cells containing the desired plasmid were identified as described above. These cells were the source of single stranded DNA for sequencing reactions. A restriction and function map of plasmid pEWM13-7 is provided in Figure 7.

The 3′ end of mecA-27R was also cloned into both M13 vectors for DNA sequencing. The HindIII/XbaI digested M13mp18 DNA and the 2.1 kb HindIII/XbaI restriction fragment containing the 3' end of the mecA-27R gene were mixed and ligated in the presence of T4 DNA ligase. Transformation of E. coli with this ligation mixture and screening of plaques was performed to identify E. coli JM109/pEWM13-9 cells. These cells were used as the source of single stranded DNA for sequencing reactions. A restriction/function map of plasmid pEWM13-9 is provided in Figure 8. To obtain the opposite orientation of the 3' end of mecA-27R the HindIII/XbaI fragment containing the desired region of the gene was cloned into M13mp19. The HindIII/XbaI digested M13mp19 DNA and the 2.1 kb HindIII/XbaI restriction fragment containing the 3' end of the mecA-27R gene were mixed and ligated in the presence of T4 DNA ligase. Transformation of E. coli with this ligation mixture and screening of plaques was performed to identify E. coli JM109/pEWM13-12. These cells were used as the source of single stranded DNA for sequencing reactions. A restriction/function map of plasmid pEWM13-12 is provided in Figure 9.

Single stranded DNA, for use in in-vitro mutagenesis, representing the 5′ end of mecA-27R was produced as follows. A 10 mL culture of early logarithmic growth phase Escherichia coli K12 CJ236 (Bio-Rad Laboratories, 1414 Harbor Way South, Richmond, CA 94804) was inoculated with ~200 µl of phage stock (pEWM13-1) and incubated ~18 hours at 37°C with aeration. The pellet contained the single-stranded phage pEWM13-1. The solution was extracted first with $CHCl_3$ and then with TE-saturated phenol to liberate single-stranded DNA. The solution was then extracted twice with a mixture of TE-saturated phenol:$CHCl_3$ (1:1, v/v), and twice with $CHCl_3$ alone. The single-stranded DNA was then precipitated out of 0.3 M NaOAc, collected by centrifugation, and the resulting pellet resuspended in 0.1X TE buffer. This solution contained the single-stranded pEWM13-1 DNA.

Synthetic oligonucleotides used in the in-vitro mutagenesis were synthesized on an automated DNA synthesizer in substantial accordance with instructions provided by the manufacturer. The mutagenesis primers were designated as follows:

(1) 2ALOOP-1, a single-stranded DNA fragment 48 nucleotides in length that is identical to 21 bases immediately 5′ of the translation initiation codon of mecA-27R, incorporates an NcoI restriction site at the initiation codon, and is identical with 21 bases beginning with the codon for amino acid 23 of PBP2A-27R with the DNA sequence:

NcoI

5'-GTCGGGTTTGGTATATATTTTCCATGGTGTATGCTTCAAAAGATAAAG - 3'

In-vitro mutagenesis with the above oligonucleotide (2ALOOP-1) was performed in substantial accordance with the procedure described in the MUTAGENE™ *IN VITRO* MUTAGENESIS KIT instruction manual (Catalog number 170-3571, Bio-Rad Laboratories, 1414 Harbour Way South, Richmond, CA 94804) the entire teaching of which is hereby incorporated by reference. Plaques containing phage DNA with the desired mutations were identified by restriction analysis of RF DNA isolated with a Qiagen Plasmid Midi Kit in substantial accordance

with the manufacturer's recommendations (Qiagen Inc., 9259 Eton Avenue, Chatsworth, CA 91311). Isolates were obtained which contained RF DNA that represented the desired mutations. These cultures were used as a source of RF DNA containing an approximately 0.74 kb NcoI/XbaI restriction fragment possessing a modified form of the 5′ end of the mecA-27R gene.

Restriction fragments containing the modified form of the 5′ end of the mecA-27R gene were isolated as follows. Escherichia coli culture JM109/pEWM13-21 was inoculated into 200 ml of TY broth and incubated overnight with aeration. The cells were harvested from each overnight culture by centrifugation (8,000 rpm, 4°C, GSA rotor, 10 minutes, Beckman Sorvall centrifuge). The supernatant was discarded and the replicative form of pEWM13-21 was isolated from the pelleted cells with a Qiagen Plasmid Midi Kit in substantial accordance with the manufacturer's recommendations (Qiagen Inc., 9259 Eton Avenue, Chatsworth, CA 91311). The HindIII/XbaI restriction fragment comprising the 5′ end of the mecA-27R gene from this plasmid was isolated. The resultant purified DNA restriction fragment is illustrated below. The fragment was resuspended in approximately 20 μl of sterile water and comprised approximately 2 μg of the desired restriction fragment.

In order to reconstruct the mecA-27R gene containing the desired modifications, it was necessary to produce intermediate plasmid pEWSA24. This was accomplished as follows. The plasmid pUC19 (Bethesda Research Laboratories, Gaithersburg, MD 20877, Figure 1) was digested with the HindIII and XbaI restriction enzymes. The desired HindIII/XbaI-digested pUC19 vector was mixed with the ~1.8 kb HindIII/XbaI restriction fragment of plasmid pEWM13-1 and ligated. The ligated DNA constituted the desired plasmid pEWSA24 and other ligation products. Transformation competent Escherichia coli K12 DH5α (MAX Efficiency) were transformed with the ligation reaction mixture constituting plasmid pEWSA24. Post-transformation, the cells were distributed to agar plates containing ampicillin (100 μg/ml), 40 μg/ml 5-bromo-4-chloro-3-indolyl-β-D-galactoside (X-gal) and 0.1 M isopropylthio-β-galactoside (IPTG), and the plates were incubated overnight at 37°C. Escherichia coli transformants containing a plasmid with an insert were identified by inactivation of the β-galactosidase gene present on pUC19. Those transformants harboring an insert ligated into the HindIII and XbaI sites of pUC19 formed colorless colonies while those uninterrupted formed blue colonies. Miniprep plasmid DNA prepared from colorless colonies was screened by restriction endonuclease digestion in substantial accordance with the procedures described by Maniatis et al. (1982, "Molecular Cloning, A Laboratory Manual, (1982) Cold spring Harbor Laboratory). One isolate was recovered which contained plasmid pEWSA24. The isolate containing this plasmid was designated DH5α/pEWSA24.

Having cloned the mutated form of the 5′ end of mecA-27R into pUC19 it was necessary to attach the wild-type 3′ end to these mutant 5′ ends. Plasmid pEWSA37 provided a source for the wild type 3′ end of the mecA-27R gene. Plasmid DNA purified from Escherichia coli DH5α/pEWSA37 was digested to completion with the restriction endonuclease XbaI. The DNA fragments formed in the restriction endonuclease digestion were separated by electrophoresis on a 0.8% agarose gel until the desired approximately 2.1 kb XbaI fragment was clearly separated from the other digestion products.

The mutant 5′ ends of the mecA-27R gene were joined with the wild-type 3′ end in the following procedures. Plasmid DNA was isolated from Escherichia coli strain DH5α/pEWSA24 using a Qiagen Plasmid Midi Kit. The pEWSA24 DNA (Bethesda Research Laboratories, Gaithersburg, MD 20877) was digested with the restriction enzyme XbaI. The reaction was terminated by extraction with phenol:chloroform and the DNA, recovered in the aqueous phase, was concentrated by ethanol precipitation. The resulting pellet was resuspended in 20μl water and constituted ~2 μg of the desired XbaI-digested pEWSA24 vector. Vector DNA obtained in this manner was stored at 20°C.

The ~2.1 kb XbaI restriction fragment isolated from pEWSA37 was mixed with the XbaI-digested pEWSA24 plasmid vector DNA and ligated in the presence of T4 DNA ligase. The ligated DNA constituted the desired plasmid pEWSA27 and other ligation products. Transformation competent Escherichia coli, K12 DH5α (MAX Efficiency, Bethesda Research Laboratories, Gaithersburg, MD 20877) were transformed with the ligation reaction mixture constituting plasmid pEWSA24. Post-transformation, the cells were distributed to agar plates containing ampicillin (100 μg/ml). The plates were incubated overnight at 37°C. Escherichia coli transformants containing a plasmid with an insert in the XbaI site of pEWSA24 were identified by restriction endonuclease digestion of miniprep plasmid DNA prepared using a Qiagen Plasmid Midi Kit. Isolates recovered which contained plasmids with an insert in the XbaI site were then screened for orientation of the XbaI insert. The restriction endonucleases SphI and BamHI were particularly useful for determination of the orientation of the inserted DNA. Plasmids containing the insert in the desired orientation would produce an approximately 0.52 kb restriction fragment plus other fragments when simultaneously cut with both of these enzymes. If the insert was in the wrong orientation, the 0.52 kb restriction fragment was present and an additional 1.58 kb SphI restriction fragment appeared. An isolate containing the desired plasmid was found and designated DH5α-/pEWSA27. Escherichia coli, cultures containing mutant form of the mecA-27R gene (i.e. DH5α/pEWSA27) were grown overnight in 200 ml of TY broth. The cells were harvested and the plasmid DNA was extracted using

a Qiagen Plasmid Midi Kit. Plasmid DNA isolated in this manner was resuspended in 200 μl sterile water and constituted plasmid pEWSA27.

In order to express the modified mecA-27R gene efficiently in Escherichia coli, the gene was moved into an E. coli expression vector. The plasmid isolated above was digested with NcoI and BamHI restriction enzymes. The reactions were loaded onto a 0.8% agarose gel and electrophoresed until the desired NcoI/BamHI fragment (approximately 2.1 kb) was clearly separated from the other digestion products. This fragment was isolated as above. The desired NcoI/BamHI restriction fragment carrying the mutant form of the mecA-27R gene from plasmid pEWSA27 (Fragment A, lacks the transmembrane region and contains an NcoI site).

Plasmid pOW241 is an expression vector useful for production of foreign proteins in Escherichia coli. Expression of foreign proteins is dependent upon induction of the thermal-inducible lambda pL promoter. This plasmid also contains a unique NcoI site at the translation initiation site and a BamHI site downstream from that position which is useful for the 3′ end of restriction fragments containing genes of interest. In pOW241 the pcbC gene occupies the region between the NcoI site and the BamHI site. The pcbC gene must be removed to make pOW241 useful for the expression of the various forms of the mecA-27R gene. A restriction site and function map of plasmid pOW241 is provided in Figure 19. Plasmid pOW241 is a derivative of plasmid pCZ336 (Samson et al., 1987, Bio/Technology, Vol. 5, No. 11, pages 1207-1214).

Plasmid pOW241 was digested with the NcoI and BamHI restriction endonucleases and the DNA fragments separated by agarose gel electrophoresis until the desired approximately 6.1 kb fragment was clearly separated from other digestion products. The approximately 6.1 kb fragment was isolated and stored at -20°C.

The ~2.8 kb NcoI/BamHI restriction fragment of plasmid pEWSA27 was mixed with the NcoI/BamHI-digested pOW241 plasmid vector DNA and ligated. The ligated DNA constituted the desired plasmid pEWSA30 and other ligation products. Transformation competent Escherichia coli K12 DH5α (MAX Efficiency) were transformed with the ligation reaction mixture constituting plasmid pEWSA30 as described above. Post-transformation, the cells were distributed to agar plates containing L-agar and tetracycline (100 μg/ml), and the plates were incubated overnight at 30°C. Escherichia coli transformants containing a plasmid with an insert were identified by restriction endonuclease digestion of miniprep plasmid DNA prepared using a Qiagen Plasmid Midi Kit. Isolates recovered which contained an approximately 2.8 kb NcoI-BamHI restriction fragment were further analyzed by restriction analysis to verify that the proper fragment had been inserted. An isolate found possessing the proper plasmid was designated DH5α/pEWSA30. A restriction site/function map of pEWSA30 is presented in Figure 20.

Activity of the modified PBP2A-27R protein expressed with plasmid pEWSA30 was analyzed as follows. An Escherichia coli K12 DH5α/pEWSA30 transformant was grown at 30°C overnight in 500 ml of L broth (containing 10 μg/ml of tetracycline) in a gyrotory incubator (250 rpm). The cells were diluted 100-fold by adding 10 ml of the overnight culture to 990 ml of fresh L broth containing 10 μg/ml tetracycline in a 2.8 L flask and incubated for one hour at 30°C under the same growth conditions. The temperature was then raised to 42°C to induce protein expression and incubation continued. The cI857 temperature-sensitive repressor of the lambda pL promoter, positioned to drive expression of the modified mecA-27R coding sequence on plasmid pEWSA30, is inactivated at 42°C. Therefore, at 42°C expression of the modified form of mecA-27R occurs in the host cells. After induction, the cells were harvested by centrifugation and used as a preferred source of E. coli produced PBP2A-27R activity.

Cells harvested from the cultures were assayed for PBP2A-27R activity. In this case it was not necessary to prepare membranes of the host cells, however it is necessary to lyse the E. coli cells. The transmembrane portion of the PBP2A-27R protein which causes the native protein to be associated with the membrane, has been removed in the mutant forms of the mecA-27R gene. As a result the PBP2A-27R protein is expressed in a cytosolic form (PBP2A-$27R^s$).

Plasmids pEWSA25, pEWSA26, and Escherichia coli strains DH5α/pEWSA25 and DH5α/pEWSA26 were constructed according to the method described immediately above for plasmid pEWSA24 and E. coli strain DH5α/pEWSA24. Plasmid pEWSA25 was constructed using the HindIII-XbaI fragment of pEWM13-22 containing the 5′ end of the mecA-27R gene possessing the sequence encoding the Met-Gly-His-Trp-His-His-His chelating peptide. Plasmid pEWSA26 was constructed using the HindIII-XbaI fragment of pEWM13-23 containing the 5′ end of the mecA-27R gene possessing the sequence encoding the Met-Gly-His-Trp-His-His-His-Pro chelating peptide. Restriction/function maps for plasmids pEWSA24, pEWSA25, and pEWSA26 are provided in Figures 13, 14, and 15 respectively.

The invention further provides derivatives of the compound of the formula 3 and derivative compounds thereof which lack the naturally occurring membrane association region and which are operably linked to a chelating peptide or other organic chelating moiety capable of forming a coordinate covalent complex with a transition metal ion.

The soluble form of PBP2A-27R proteins may also be modified by the incorporation of a DNA sequence

encoding a chelating peptide the resultant protein being denoted by the addition of the "CP" suffix (e.g., PBP2A-CP, PBP2A-27R-CP). DNA sequence encoding the chelating peptide may be placed at either the 5′ or 3′ end of the sequence encoding the penicillin binding protein. The decision of whether to incorporate the chelating peptide encoding sequence at the 5′ or 3′ end of the PBP encoding sequence will in large part be dependent upon the particular molecule employed and whether the chelating peptide is to be used merely as a purification moiety or as an immobilization feature as described below. A variety of chelating peptides may be employed which will form a complex with an immobilized transition metal ion. Such chelating peptides are represented by the formula:

$$(His)_x\text{-}(A)_y\text{-}(His)_z$$

where A is an amino acid

$$x = 1\text{-}10$$
$$y = 0\text{-}4$$
$$z = 1\text{-}10$$

and monomers, dimers and trimers thereof wherein

each monomer unit is the same or different. In the preferred practice of the invention, the chelating peptide may be from 2 to 10 amino acids in length containing from at least one, and preferably 2 or more, histidine residues. In the preferred practice of the invention A is selected from the group comprising Trp, Gly, or Tyr. Those chelating peptides most preferred in the practice of the instant invention are shown in Table I.

Table I. Chelating Peptides

| letter designation | amino acid sequence |
|---|---|
| (a) | His-Trp-His-Met-Tyr |
| (b) | His-His-His-Met-Tyr |
| (c) | His-His-His-His-Tyr |
| (d) | His-Trp-His-Trp-His |
| (e) | His-Trp-His-His-His |
| (f) | His-His-His-His-Tyr-Met-His-His-His-His-Tyr |
| (g) | His-His-His-His-His |
| (h) | His-Trp-His-His-His-Pro |

It is permissible to have an N-terminal methionine residue, characteristic of recombinant proteins, and still retain the functional aspects of the chelating peptide sequences shown above. Furthermore, it is permissible to have from 1 to approximately 6 amino acids between the N-terminus of the CP-protein and the chelating peptide and retain the metal binding properties of the chelating peptide. It is also permissible to have from 1 to approximately 6 amino acids following the chelating peptide when the chelating peptide is located at the C-terminus of the protein. It is also permissible to incorporate the chelating peptide into the primary structure of the protein so that it is surface exposed and is capable of complexing with a metal ion.

A variety of metal ions may be incorporated into the practice of the instant invention. Octahedral complexes with filled ($d^6$) or half-filled ($d^3$) levels such as Cr(III), V(II), Mn(IV) and the low spin forms of Co(III), Fe(II), Ru(II), Os(II), Rh(III), Ir(III), Pd(IV) and Pt(IV) tend to be extremely inert and useful in the practice of the instant invention. Hanzik, Robert P. in Inorganic Aspects of Biological and Organic Chemistry, Academic Press, New York, 1976, P. 109. See also Cotton, F. A. and Wilkinson, G. supra. One of skill in the art would appreciate the parameters involved in incorporating these metal ions into the practice of the instant invention. In the preferred practice of the invention the metal ion is selected from the group comprising Co, Cr, and Ru. In the most preferred practice of the invention the metal ion is Co. In the most preferred practice of the invention it is desirable to proceed from Co(II), Cr(II), or Ru(III) to Co(III), Cr(III), or Ru(II) respectively to form the inert complex. The

metal ions Zn, Cu, or Ni are useful for purification purposes but do not appear to afford complexes as inert as those provided with the preferred metal ions, such as cobaltic ion.

Certain metal ions are preferred when using any particular chelating agent. In general, for the purposes of this invention, hard metal ions should be complexed with harder ligands and soft metal ions should be complexed with soft ligands. For example, when the chelating agent employed is iminodiacetic acid (IDA) preferred metal ions include Cr(III), V(II), Mn(IV), Co(III), Fe(II), Ru(II) and Pt(IV). When the chelating agent employed is nitrilotriacetic acid (NTA) preferred metal ions include Cr(III), V(II), Mn(IV), Co(III), Fe(II), Ru(II) and Pt(IV). When the chelating agent employed is terpyridine, bipyridine,or polypyrazolyl borate ligands, preferred metal ions include Co(III), Fe(II), Ru(II), Os(II), Rh(III), Ir(III), Pd(IV) and Pt(IV). When the chelating agent employed is triethylenetetraamine, biethylenetetraamine, or 1,4,7-triazacyclonane, preferred metal ions include Co(III), Cr(III), Fe(II), Ru(II), Os(II), Rh(III), Ir(III), Pd(IV) and Pt(IV).

The instant invention also provides PBP2A-CPs of the form wherein a proline residue has been incorporated between an N-terminal chelating peptide and the structural protein. The incorporation of a proline residue and even number of amino acids downstream of the end terminus allows for easy removal of the chelating peptide by the action of diaminopeptidase. Removal of the amino terminal chelating peptide results in the soluble form of the penicillin binding protein.

As exemplified herein, a chelating peptide sequence was incorporated into the amino terminal end of the PBP2A-27R$^s$ protein. This protein incorporating a chelating peptide into its primary structure was denoted PBP2A-27R-CP to reflect the presence of the chelating peptide. A PBP2A protein which has been modified to incorporate a chelating peptide is generally referred to herein as a PBP2A-CP protein. The use of chelating peptides as purification moieties is described in Smith, M., et al., U.S. Patent No. 4,569,794, the entire teaching of which is hereby incorporated by reference. Purification of the penicillin binding proteins may be achieved by the CP-IMAC principles disclosed in this reference.

Production of the PBP2A-27R$^s$-CP proteins was achieved in substantial accordance with the site-specific mutagenesis procedure described above for the production of the solubilized version of the PBP2A-27R protein except that the following oligonucleotides were used in place of the 2ALOOP-1 oligonucleotide:

(2) 2ALOOP-2, a single-stranded DNA fragment 63 nucleotides in length that is identical to 21 bases immediately 5′ of the translation initiation codon of mecA-27R, incorporates an NcoI restriction site at the initiation codon, encodes a metal chelating peptide, and is identical to 19 bases of mecA-27R beginning with the codon which specifies amino acid 23 of PBP2A-27R with the DNA sequence:

```
                             NcoI
5' -
GTCGGGTTTGGTATATATTTTCCATGGGTCATTGGCACCATCAC
      TATGCTTCAAAAGATAAAG - 3'
```

(3) 2ALOOP-3, a single-stranded DNA fragment 66 nucleotides in length that is identical to 21 bases immediately 5′ of the translation initiation codon of mecA-27R, incorporates an NcoI restriction site at the initiation codon, encodes a metal chelating peptide which ends with a codon for proline to facilitate dipeptidase removal of the metal chelating peptide, and is identical with 19 bases of mecA-27R beginning with the codon which specifies amino acid 23 of PBP2A-27R with the DNA sequence:

```
                             NcoI
5' -
GTCGGGTTTGGTATATATTTTCCATGGGTCATTGGCACCATTGGCCT
      TATGCTTCAAAAGATAAAG - 3'
```

Plasmids pEWSA28 and pEWSA29 and Escherichia coli strains DH5α/pEWSA28 and DH5α/pEWSA29 were constructed according to the method described immediately above for plasmid pEWSA27 and E. coli strain DH5α/pEWSA27 with the following exceptions. Plasmid pEWSA28 was constructed using the HindIII-XbaI fragment of pEWM13-25 containing the 5′ end of the mecA-27R gene possessing the sequence encoding the Met-Gly-His-TrpHis-His-His chelating peptide. Plasmid pEWSA29 was constructed using the HindIII-XbaI fragment of pEWM13-26 containing the 5′ end of the mecA-27R gene possessing the sequence encoding the Met-Gly-His-Trp-His-His-His-Pro chelating peptide. Restriction site and function maps for plasmids pEWSA28, and

pEWSA29 are provided in Figures 17 and 18 respectively. Plasmid pEWSA28, formed with 2ALOOP-2, lacks the transmembrane region, contains an NcoI site, and a chelating peptide. Plasmid pEWSA29, formed with 2ALOOP-3, lacks the transmembrane region, contains an NcoI site, and a proline chelating peptide.

Plasmids pEWSA31 pEWSA32 and Escherichia coli strains DH5α/pEWSA31 and DH5α/pEWSA32 were constructed according to the method described immediately above for plasmid pEWSA30 and E. coli, strain DH5α/pEWSA30 using the NcoI/BamHI restriction fragments from pEWSA28 and pEWSA29 respectively. Restriction/function maps for plasmids pEWSA31 and pEWSA32 are provided in Figures 21 and 22 respectively.

Furthermore, in a manner analogous to that described above for expression of pEWSA30 in E. coli, cultures containing plasmids pEWSA31 (K12 DH5α/pEWSA31) and pEWSA32 (K12 DH5α/pEWSA32) were grown and induced to express their forms of the mecA-27R gene.

It will be readily appreciated by those skilled in the art that PBP2A-CPs may be produced by recombinant DNA technology or well known chemical procedures, such as solution or solid-phase peptide synthesis, or semi-synthesis in solution beginning with protein fragments coupled through conventional solution methods. In the preferred practice of the invention, PBP2A-CPs are produced through the use of recombinant DNA technology by the addition of an in-frame DNA sequence encoding a chelating peptide into the coding sequence of the protein of interest. Incorporation of the chelating peptide coding sequence may be achieved by conventional genetic cloning techniques or by the use of the polymerase chain reaction (PCR) to create and amplify the synthetic gene encoding the PBP2A-CP. Synthetic PCR primers may be created which contain the coding sequence of the chelating peptide thereby directly incorporating the chelating peptide coding sequence into the DNA produced via PCR. The expression products of such coding sequences are "PBP2A-CPs" which possess a chelating peptide.

The placement of the chelating peptide in the ultimate PBP2A-CP is largely dictated by the properties of the protein. The chelating peptide coding sequence may be incorporated into the protein coding sequence at or near either the 5′ or 3′ ends or the protein coding sequence such that the chelating peptide will occur at or near either the amino or carboxyl terminus of the protein. Furthermore, the chelating peptide may be incorporated into the primary structure of a protein so that it is surface exposed and is capable of complexing with a metal ion. In all circumstances, it is preferred that the chelating peptide should be incorporated into the protein so as to minimize any potential effect on the biological properties of the protein. In some instances, such as where steric hinderance may interfere with the binding of the chelating peptide to the metal ion thereby preventing immobilization, it may be desirable to provide some "spacer" between the chelating peptide and the protein sought to be immobilized. Such "spacers" would include peptides of from 1 to approximately 30 amino acids. Chelating peptides may also be linked to proteins through organic linkers of varying length to provide the desired spacing effect. The inclusion of a polypeptide spacer is readily achieved when the CP-protein is produced via recombinant DNA technology by insertion a DNA sequence encoding the desired polypeptide spacer (preserving the proper reading frame of the chelating peptide and protein molecules) between the DNA sequences encoding the chelating peptide and the protein sought to be immobilized.

It is useful in some applications to engineer a linkage between the chelating peptide and the protein to provide a specific proteolytic cleavage point between the chelating peptide and the protein. Proteolysis may be achieved by enzymatic (e.g., trypsin, chymotrypsin) or chemical (e.g., cyanogen bromide) means. Furthermore, the incorporation of a proline residue at an even numbered position from the N-terminus of the CP-protein facilitates removal of the chelating peptide through the action of DAP-I (diaminopeptidase), where the chelating peptide sequence does not contain a proline residue.

However, the penicillin binding protein possessing a chelating peptide may be immobilized on a column possessing an immobilized transition metal ion in a labile oxidation state. Subsequent oxidation of the bound transition metal ion by various means enables one to create a kinetically inert coordinate covalent complex between the penicillin binding protein possessing the chelating peptide and the column. This immobilized form of the PBP2A-CP provides an ideal substrate to assay for compounds which bind to penicillin binding proteins produced by methicillin resistant strains of staphylococci.

The invention further provides a method of purifying and assaying for agents which possess the ability to bind penicillin binding proteins, said method comprising the steps of:

1. producing a penicillin binding protein covalently bonded to a chelating agent capable of forming a coordinate covalent complex with a transition metal ion,

2. introducing said penicillin binding protein covalently bonded to a chelating agent to a solid support, said solid support possessing an immobilized transition metal ion, said transition metal ion being in a kinetically labile oxidation state, so as to form a coordinate covalent complex between the chelating agent and said immobilized transition metal ion,

3. changing the oxidation state of said transition metal ion to a kinetically inert oxidation state so as to form a kinetically inert coordinate covalent complex between the chelating agent and the immobilized transition

metal ion.

4. introducing antibiotic agents to said kinetically inert immobilized PBP-CP complex,

5. assaying for binding of said antibiotic agents to the penicillin binding protein portion of said kinetically inert coordinate covalent complex.

The process of the instant invention provides the means to develop an assay system to study the interaction of an immobilized PBP2A-CP with various potential ligands.

As exemplified herein, the procedure of such a PBP2A binding assay proceeds as follows. The protein with which one wishes to study the interaction of secondary molecules is produced in PBP2A-CP form as outlined above. The PBP2A-27R$^s$-CP protein was produced by the addition of a DNA sequence encoding the Met-Gly-His-Trp-His-His-His chelating peptide at the 5′ end of the DNA sequence encoding the PBP2A-27R$^s$ protein by techniques well known in the art. This gene encoding the PBP2A-27R$^s$-CP protein was then incorporated into the pEWSA31 expression plasmid and transformed into competent E. coli K12 DH5α cells by techniques well known in the art. Expression of the PBP2A-27R-CP was confirmed by PBP binding assays.

A CP-IMAC column is prepared in substantial accordance with the teaching of Example 4.U. herein and according to the teaching of Smith, et al., United States Patent No. 4,569,794 herein incorporated by reference. Care must be taken when using many transition metal ions to avoid the presence or introduction of redox agents to the column matrix. The immobilized metal ion must be in the labile oxidation state. As exemplified herein, an iminodiacetic acid (IDA) molecule possessing a bound Co(II) ion is covalently attached to a hydrophilic resin, such as fast flow agarose or TSK-PW gel. The immobilized Co(II) ion interacts with the chelating peptide portion of the PBP2A-CP protein linking the PBP2A-CP protein to the column. This provides a facile method for purification of the PBP2A-CP protein. The PBP2A-CP protein is purified from the crude mixture by virtue of the formation of the [CP-protein/Co(II)-Resin] complex, i.e., the "unlocked" form. Non-specifically bound molecules are then liberated by the introduction of a weak competitor such as imidazole.

When one has achieved the linkage of the PBP2A-CP to the immobilized metal ion while in the labile oxidation state, three alternatives are available:

(1) release the PBP2A-CP protein from the bound metal ion by lowering the pH or by the addition of competitors such as imidazole (i.e., conventional CP-IMAC purification), or

(2) cleave the immobilized PBP2A-CP by chemical or enzymatic means at an appropriate point between the structural protein and chelating peptide sequences allowing isolation of substantially pure protein while leaving the chelating peptide bound to the matrix, or

(3) "lock" the PBP2A-CP to the column matrix by changing the oxidation state of the metal ion.

Alternative (1) above results in the recovery of purified PBP2A-CP protein. PBP2A-27R-CP protein purified in this manner may be used in structural studies of the protein and in assays to detect inhibitors of PBP2A-27R which may prove useful as therapeutic agents. However, by changing the oxidation state of the bound metal ion, as referred to in alternative (3) above, it is possible to change the kinetic parameters of the [metal ion-PBP2A-CP] complex so as to achieve a kinetically inert linkage of the chelating peptide or PBP2A-CP to the support matrix. This PBP2A-CP protein, immobilized through the kinetically inert metal ion linkage, may then be used to study interactions between the protein, ligands, potential ligands, and compounds which may interfere with the binding of ligands to the PBP2A-CP protein.

A kinetically inert complex is formed by changing the oxidation state of the bound metal ion causing the metal ion to change from a labile to a kinetically inert oxidation state. In the case of PBP2A-27R$^s$-CP, the protein is "locked" to the matrix by oxidation of the bound cobalt ion from the +2 to +3 oxidation state by introducing to the column oxygen, an oxygen containing gas, or other oxidant capable of oxidizing the Co(II) to Co(III) but will not oxidize the PBP2A-27R$^s$-CP molecule to an inactive state.

Producing the necessary change in the oxidation state of the metal ion may be achieved by a variety of redox reagents. For example oxidizing agents such as oxygen, hydrogen peroxide, and peracids may be used in the practice of the invention. Examples of reducing agents include thiols, potassium ferrocyanide, potassium thiocyanate, sulfites, and sodium dithionite. These will be prepared in aqueous solutions of appropriate concentrations. It will be recognized by those skilled in the art that not all redox reagents will be appropriate to change the oxidiation state of these metal ions under all conditions so as to form kinetically inert complexes with the chelating agents provided. One factor in selecting the appropriate redox reagent is to take into consideration the electrochemical potential of the labile complex and use a reagent such that the overall free energy of the reaction is spontaneous. It will also be recognized by those skilled in the art that not all redox reagents would be appropriate depending on the nature of the molecules to be crosslinked. The susceptibility of proteins or reporter molecules to inactivation by certain redox reagents must be considered as well.

In the preferred practice of the invention as exemplified herein, formation of the kinetically inert complex is accomplished by the following batch process. The contents of the column containing the immobilized IDA-Co(II)-PBP2A-27R-CP complex was transferred to a test tube. The space above the settled gel was replaced

with the oxygen or an oxygen containing gas and then capped. The tube was continuously inverted for 24 hours to mix the oxygen containing gas with the bead containing the IDA-Co(II)-PBP2A-27R-CP complex to oxidize the Co(II) to Co(III). The PBP2A-27R-CP thereby assumes the "locked" conformation. Equal aliquots of the beads possessing the IDA-Co(III)-PBP2A-27R-CP complex may then be transferred to a reaction vessel for example an Eppendorf tube, or the well of a microtiter plate.

The "locked" form of PBP2A-CP provides an ideal ligand for assay of those molecules which bind to PBP2A. A sample of the labelled antibiotic test compound is introduced to the reaction vessel containing the immobilized PBP2A-CP. Unbound test compound is then washed free. The presence of the labelled compound indicates the formation of the [PBP2A-test compound] complex. The presence of the formation of the PBP2A-27R-CP-/ligand complex may be determined in a variety of manners. In the preferred practice of the invention as exemplified herein, the binding of test compound is determined by the comparative level of binding of $^{125}$I-penV in the presence of the test compound versus the absence of the test commpound. The binding may also be measured by scoring the level of radioactivity through the use of radioactive labelled ligands in the assay. The PBP2A ligand may be labelled in other manners such as by fluorescent dyes or enzymatic conjugation. All direct modifications of the PBP2A ligands must be achieved in such a manner so as not to inhibit the formation of the PBP2A-27R-CP/ligand complex. In the alternative, enzymatic, fluorescently, or radioactively labelled antibodies against the PBP2A ligand may be introduced to the tube to determine the presence of the PBP2A-27R-CP/ligand complex.

The assay system developed according to the practice of the instant invention, such as the PBP2A-CP assay described above, are readily adaptable to automated procedures such as where the reaction vessel is the well of a multiwell Pandex® assay plate or a microtiter plate.

The instant invention further provides derivatives of the compounds of formula 4:

$$SP - CP - (Pro)_n - PBP2A^s.$$

wherein SP is a signal peptide, CP is a chelating peptide of the formula:

$$(His)_x\text{-}(A)_y\text{-}(His)_z$$

where A is an amino acid

$$x = 1\text{-}10$$
$$y = 0\text{-}4$$
$$z = 1\text{-}10$$

and monomers, dimers and trimers thereof wherein each monomer unit is the same or different, n = 0 or 1, and PBP2A$^s$ is a soluble version of the PBP2A molecule.

It is well known in the art that signal peptides facilitate the extracellular discharge of secretory proteins in both procaryotic and eucaryotic environments. It has been shown that the addition of a heterologous signal peptide to a normally cytosolic protein will result in the extracellular transport of the normally cytosolic protein in E. coli. MacIntyre, et al.,(1987) J.Biol.Chem. 262: 8416-8422. The recombinant production of such fusion proteins is accomplished by the addition of a DNA sequence encoding a signal peptide appropriate to the host organism inserted 5′ to, and in reading frame with, the protein coding sequence.

Although the principles of signal peptides are similar in both procaryotic and eucaryotic organisms, significant differences exist. The procaryotic system is much less understood than the eucaryotic system, however certain generalizations may be made. The presence of basic and/or charged amino acid residues near the amino terminus of the structural protein inhibits secretion. Yamane, K., et al. (1988) J.Biol.Chem. 263:19690-19696, Summers, R.G., et al (1989) J.Biol.Chem. 264:20082-20088. In other respects the signal peptides of procaryotes are similar to eucaryotes. Both procaryotic and eucaryotic signal peptides possess an overall three domain structure and with no precise sequence conservation necessary to preserve function. von Heijne, G. (1990) 1. Membrane Biol. 115: 195-201, the entire teaching of which is hereby incorporated by reference. Synthetic "idealized" signal peptides have been shown to function in both procaryotic and eucaryotic environments. , Id.

In the preferred practice of the invention the signal peptide used is a signal peptide native to a secretory protein of the host cell line. For example, when expressing proteins in E. coli, it is preferable to use an E. coli signal peptide. The amino acid sequences of a variety of E. coli secretory proteins are well known in the art. Examples of these signal peptides include the signal peptides of the ompA gene, the ampC gene, and the β-lactamase gene. In the most preferred practice of the invention, the signal peptide of the ampC gene is incorporated into the 5′ end of the DNA sequence encoding a soluble form of the PBP2A-27R protein. In order to insure the efficient cleavage of the signal peptide from the fusion protein construct, it is desirable to maintain the nature of the amino acid sequence at the interface between the signal peptide and the coding sequence of the mature art protein. The signal peptidase cleavage appears to be governed primarily by the secondary and tertiary structure of the amino acid sequence of the interface. Conservation of charge and hydrophobicity and the elimination of charged residues immediately downstream of the signal peptide cleavage point appear

to be critical to efficient translocation in procaryotes. However, it is not critical that any one particular amino acid sequence be maintained.

It may desirable in some applications to modify the coding sequence of the signal peptide relative to the structural protein so as to incorporate a convenient protease sensitive cleavage site between the signal peptide and the structural protein. This facilitates the controlled excision of the signal peptide from the fusion protein construct. For example, incorporation of a Lys or Arg residue into the carboxyl terminal residue of the signal peptide facilitates the removal of the signal sequence by the action of trypsin. Similar incorporations of Phe, Trp or Tyr residues facilitates the removal of the signal peptide by the action of chymotrypsin. Removal of the signal peptide may also be achieved by a chemical means such as cyanogen bromide cleavage through the incorporation of a methionine residue into the carboxyl terminal residue of the signal peptide. In the preferred practice of the invention as exemplified herein, plasmids encoding secretory forms of the PBP2A-27R^s variants are formed by the insertion of a DNA sequence encoding a signal peptide at the 5′ end of the PBP2a-27R^s coding sequence. The following dsDNA sequence:

```
5'   CATGTTCAAAACGACGCTCTGCGCCTTATTAATTACCGCCTCTTGCTCCACATTTGCTGC 3'
       ||||||||||||||||||||||||||||||||||||||||||||||||||||||||||||
3'       AAGTTTTGCTGCGAGACGCGGAATAATTAATGGCGGAGAACGAGGTGTAAACGACGGTAC 5'
```

encodes the amino acid sequence:

MetPheLysThrThrLeuCysAlaLeuLeuIleThrAlaSerCysSerThrPhe
AlaAlaMetVal -- PBP2A)

corresponding to the ampC signal peptide. When inserted into plasmids pEWSA30, pEWSA31 and pEWSA32 will result in PBP2A-27R^s proteins (and PBP2A-27R^s derivatives with chelating peptides) with the ampC signal peptide at the amino terminus of the resultant protein.

For example, the plasmid pEWSA30 is digested with the NcoI restriction endonuclease taking advantage of the unique NcoI site at the start codon of the PBP2A-27R^s coding sequence. The linearized vector DNA is isolated by agarose gel electrophoresis and dephosphorylated with calf intestinal phosphatase. The above ssDNA sequences are synthesized on an Applied Biosystems Model 380A or 380B DNA synthesizer (Applied Biosystems, Foster City, CA). The single-stranded molecules are purified by polyacrylamide gel electrophoresis and isolated from the gel. The ends are phosphorylated using T4-polynucleotide kinase. The ssDNA molecules are annealed. The phosphorylated dsDNA encoding the signal peptide is mixed with the dephosphorylated vector DNA in the presence of T4 DNA ligase. The resultant mixture contains plasmid pEWSA39 containing the proper orientation of the synthetic dsDNA insert and other ligation products. The mixture was used to transform competent E. coli K12 DH5α cells. The cells were grown and colonies picked and resuspended in 2x TY broth.

Identification of those strains containing pEWSA39 are determined by visualization of the restriction products by miniprep agarose gel electrophoresis. The DNA is digested with the NcoI and XbaI restriction endonucleases. Cells carrying the pEWSA39 vector should produce an ~675 bp fragment. Cells carrying the dsDNA fragment encoding the ampC signal peptide in reverse orientation will lack the band corresponding to the ~675 bp fragment and will produce a band corresponding to ~740 bp fragment.

An Escherichia coli culture containing the mutant form of the mecA-27R gene (*i.e* DH5α/pEWSA39) was grown overnight in 200 ml of TY broth. The cells were harvested and plasmid DNA was extracted using Qiagen Plasmid Midi kit. Plasmid DNA isolated in this manner was resuspended in 200 microliters of sterile water and constituted plasmid pEWSA39. Plasmid pEWSA39, formed with the double stranded insert as described above, lacks the transmembrane region, contains an NcoI site, and the ampC signal peptide coding sequence.

Plasmids pEWSA40 and pEWSA41 and E. coli strains DH5α/pEWSA40 and pEWSA41 were constructed according to the method described immediately above for plasmid pEWSA39, and E. coli strain DH5α-/pEWSA39 except that plasmids pEWSA31 and pEWSA32 were used in place of plasmid pEWSA30 to construct pEWSA40 and pEWSA41, respectively. A restriction site and function maps of plasmids pEWSA40 and pEWSA41 are provided in figures 29 and 30, respectively. Plasmid pEWSA40 contains the Nco1 site, a chelating peptide, and a signal peptide of ampC. Plasmid pEWSA41 comprises the Nco1 site, the ampC signal peptide, the chelating peptide and a proline residue between the chelating peptide and structural protein.

In a manner analogous to that described above for expression of pEWSA39 and E. coli, cultures containing plasmids pEWSA40 (K12 DH5α/pEWSA40) and pEWSA41 (K12 DH5α/pEWSA41) were grown and induced to express their forms of the mecA gene.

## EXAMPLES

The following Examples are merely illustrative of the practice of the invention and should not be considered to limit the scope of the invention in any way.

### Example 1

### Source of the *Staphylococcus aureus mecA*-27R gene

#### Culture of *Staphylococcus aureus* strain 27R

A sample of the methicillin resistant Staphylococcus aureus strain 27R was obtained from Dr. Richard Novick (New York City Public Health Department). Two hundred ml of TY broth (8 g tryptone, 5 g NaCl, and 5 g yeast extract per liter) was inoculated with S. aureus strain 27R and incubated at 37°C in a gyrotory shaker (250 rpm with a two inch throw) overnight (15-18 hours). The cells from the resulting culture were used as a source of S. aureus DNA containing the mecA-27R gene which encodes the penicillin binding protein PBP2A-27R that is responsible for methicillin resistance in this bacterium.

#### B. Isolation of DNA from *Staphylococcus aureus* stain 27R

The culture prepared in example 1-A was centrifuged at 6000 rpm for 10 minutes at 4°C to pellet the cells (Beckman Sorvall centrifuge, SS34 rotor). The resulting supernatant was discarded. The cell pellet was resuspended in 5 ml TS buffer (TS buffer = 0.05 M Tris pH 8.0 and 25% w/v sucrose). This cell suspension was dispensed into a sterile 50 ml polyethylene centrifuge tube.

One ml of a solution composed of 10 mg lysozyme/ml, 10 mg Lysostaphin/ml, and 0.05 M Tris-pH 8.0, was added to the tube containing the cell suspension. The tube was incubated for 30 minutes at 37°C. Two ml of a solution containing 0.25 M EDTA-pH 8.0, was added to the tube. The contents of the tube was mixed thouroghly and then incubated at 37°C for an additional 5 minutes. Eight ml of a lysis solution (2% SDS, 0.05 M EDTA, 20 μg Proteinase K/ml, and 20 μg/ml RNase-pH 8.0) were then added to the mixture. The tube, containing this mixture, was incubated at 37°C for 30 min. At this point the cells had lysed and released their DNA into the medium (total volume = 16 ml).

An equal volume of phenol, saturated with TE buffer, was added to the tube containing the lysed cell mixture. This tube was then centrifuged to enhance phase separation. Cell debris collected at the bottom of the tube while protein precipitated out at the interface of the phenol and aqueous phases. The upper aqueous phase, containing DNA, was carefully removed by aspiration, avoiding the proteinacious interface, and then was extracted once more with phenol in the same manner. The resulting aqueous phase was extracted twice with an equal volume of chloroform.

The upper aqueous phase, which contained the extracted nucleic acids, was saved and dispensed in 4 ml aliquots in 15 ml polyethylene centrifuge tubes. One tenth volume 3.0 M sodium acetate and two volumes ice cold absolute ethanol were added to precipitate the DNA. This mixture was incubated at -70°C for approximately 30 minutes. DNA was harvested by centrifugation in a Beckman Sorvall centrifuge (HB-4 rotor, 10,000 rpm, -15°C, 25 minutes). The resulting DNA pellet was air-dried and resuspended in TE buffer at a concentration of approximately 1 μg DNA/μl.

#### C. Preparation of *Staphylococcus aureus* 27R genomic DNA restriction fragments for construction of a DNA library

The restriction endonuclease SauIIIA forms restriction fragments of DNA which have ends compatible for ligation with those ends created by BamHI. A broad size range of DNA restriction fragments were prepared by partial digestion of samples of DNA prepared in example 1-B. Restriction digests consisted of 5 μl DNA (~5 μg), 2 μl 10x restriction enzyme buffer (Boehringer Mannheim, Indianapolis, IN), 11 μl water, and 2 μl of the restriction enzyme SauIIIA (10 units) [Boehringer Mannheim, Indianapolis, IN] and were incubated at 37°C for 30 seconds, 1 minute, or 2 minutes in separate mixtures. Reactions were terminated by extraction with phenol and chloroform as described in example 1-B. The DNA was then ethanol precipitated (see example 1-B) and resuspended in water. DNA fragments generated in this manner represent a set of fragments with randomly generated ends (defined by the SauIIIA restriction endonuclease cleavage site) covering the entire genome, thus any particular gene should be represented intact in a subset of the fragments.

D. Construction of a *Staphylococcus aureus* 27R genomic library

A genomic library was constructed in the lambda phage EMBL3. Samples of the SauIIIA partially digested DNA from example 1-C were ligated to EMBL3 phage arms (Stratagene, 11099 North Torrey Pines Road, La Jolla, CA 92037) in substantial accordance with the conditions prescribed by the manufacturer. The ligated DNA was then packaged (Gigapack II Gold Packaging Extract, Stratagene, 11099 North Torrey Pines Road, La Jolla, CA 92037) into lambda phage heads. The packaging extracts were used to infect Echerichia coli, strain P2392 provided by Stratagene. The infected E. coli cells were plated in soft agar overlays on agar plates as described (stratagene). The inoculated plates were incubated at 37°C overnight (12-16 hrs.) and plaques which had formed were screened for the presence of the mecA-27R gene.

E. Screening of the *Staphylococcus aureus* 27R genomic library for the *mec*A-27R gene by plaque hybridization

The plaques generated in example 1-D were screened for the mecA-27R gene by hybridization in substantial accordance with the procedure described for plaque hybridization in Maniatis et al. ("Molecular Cloning, A Laboratory Manual, 1982, Cold Spring Harbor Laboratories). The hybridization probe used to screen the library was generated by the polymerase chain reaction (PCR). PCR oligonucleotide primers were synthesized on an automated DNA synthesizer as suggested by the manufacturer. The sequence of the primers, illustrated below, was based on the DNA sequence of the mecA-27R gene published by Song et al. (1987, FEBS Letters, Vol. 221(1) pages 167-171).

5' - GTTGTAGTTGTCGGGTTTGG - 3'

5' - GAGGGTGGATAGCAGTACCTGAGCC - 3'

These oligonucleotide primers, when used in a PCR reaction under the conditions specified by the manufacturer of the DNA Thermal Cycler (Perkin Elmer Cetus), amplified the 5′ end (~1,000 bases) of the mecA-27R gene from Staphyloccus aureus strain 27R and other MRS strains. In order to make a hybridization probe of high specific activity, (α-$^{32}$P)ATP (New England Nuclear, Boston MA) was added to the PCR reaction and the amount of unlabeled dATP was reduced to about one tenth the normal concentration (PCR conditions included the following parameters of a thermocycle file: melting temp.= 94°C for 30 sec., annealing temp. = 55°C for 30 sec., extension = 72°C for 1 min., and 30 cycles) . Unincorporated (α-$^{32}$P)ATP was removed by spin column chromatography in substantial accordance with the manufacturer's recommendations (Quick Spin™ Columns, G25 Sephadex, Boehringer Mannheim Biochemicals, Indianapolis, IN). Radiolabeled oligonucleotides prepared in this manner were denatured by boiling for five minutes and used directly as hybridization probes.

Plaques which exhibited a positive hybridization response with the probe described above were purified. DNA was extracted from the phage responsible for formation of these plaques of interest. The purified DNA was digested with a selection of restriction enzymes. The restriction fragments generated were separated on a 0.8% agarose gel and analyzed for the presence of the mecA-27R gene by the procedure of Southern (1975, Journal of Molecular Biology, Vol. 98, pages 503-517). The mecA-27R gene was found to lie completely within an approximately 4.0 kb HindIII restriction fragment (illustrated below) found in phage isolate #1A .

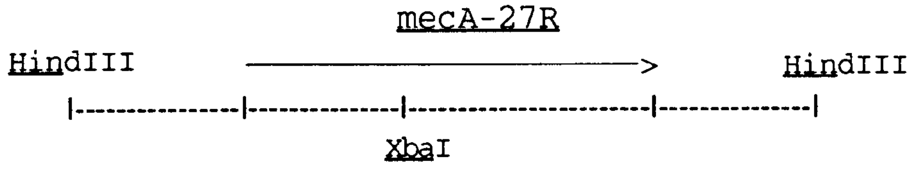


This HindIII restriction fragment was the source of the mecA-27R gene for further studies. The approximately 4.0 kb HindIII restriction fragment was cleaved by the restriction enzyme XbaI and the resultant fragments were cloned into M13mp18 and M13mp19 (Figures 4 and 5) for DNA sequencing. This process resulted in the formation of plasmids pEWM13-1, pEWM13-7, pEWM13-9 and pEWM13-12 (illustrated in Figures 6,7,8, and 9 respectively). The entire DNA sequence of the mecA-27R gene from Staphylococcus aureus 27R was determined by the Sanger dideoxy-mediated chain-termination method (Sanger et al., 1977, Proceedings of the National Academy of Science USA, Vol. 74, page 5463) using the TaqTrack Sequencing System (Promega Corporation, 2800 Woods Hollow Road, Madison, WI 53711-5399). The DNA Sequence is presented in Figure

23. The amino acid sequence deduced from that DNA sequence is presented in Figure 24.

Example 2

Construction of plasmids pEWSA8 and pEWSA37 and *Escherichia coli* strains K12 DH5α/pEWSA8 and DH5α/pEWSA37

A. Isolation of the 4,0 kb *Hind*III restriction fragment containing the *mec*A-27R gene from *Staphyloccus aureus* 27R

DNA purified from phage isolate #1A was digested to completion with the restriction endonuclease HindIII as follows. Ten µl (~10 µg) of phage DNA prepared in example 1-E were mixed with 33 µl sterile water, 5 µl 10X HindIII restriction enzyme buffer, and 2 µl of the restriction enzyme HindIII (-24 units). The restriction enzyme reaction was incubated for approximately 3 hours at 37°C. The DNA fragments formed in the restriction endonuclease digestion were separated by electrophoresis on a 0.8% agarose gel until the desired 4.0 kb HindIII fragment was clearly separated from the other digestion products. The electrophoresed DNA was visualized by staining with ethidium bromide (0.5 µg/ml) and exposing the stained gel to long-wave UV light. After the fragment was located, a small slit was made in the gel in front of the ~4.0 kb HindIII fragment, and a piece of Schleicher and Schuell (Keene, NH 03431) DEAE membrane cut to the appropriate size was placed in the slit. Upon further electrophoresis, the DNA non-covalently bound to the DEAE membrane. After the fragment was bound to the DEAE membrane, the membrane was removed and rinsed with low salt buffer (100 mM KCl; 0.1 mM EDTA; and 20 mM Tris-HCl, pH 8.0). Next, the membrane was placed in a small tube and immersed in high salt buffer (1 M NaCl; 0.1 mM EDTA; and 20 mM Tris-HCl, pH 8.0) and then incubated at 65°C for 30 minutes to remove the DNA from the DEAE paper. After the 65°C incubation, the incubation buffer, which contained the DNA, was collected, and the membrane was rinsed with high salt buffer. The rinse buffer was pooled with the incubation buffer.

The volume of the high salt-DNA solution was adjusted so that the NaCl concentration was 0.25 M, and then three volumes of cold, absolute ethanol were added to the solution. The resulting solution was mixed and placed at -70°C for 30 minutes. The solution was then centrifuged at 10,000 rpm in an HB-4 rotor for 25 minutes at -15°C in a Beckman sorvall centrifuge. The supernatant was discarded and the pellet composed of DNA was air dried. The DNA pellet was resuspended in water and ethanol precipitated once more to remove residual salt as described in example 1-B. This time the air dried DNA pellet was resuspended in 50 µl of water and constituted the desired 4.0 kb HindIII restriction fragment containing the mecA-27R gene from Staphylococcus aureus strain 27R .

B. Preparation of *Hind*III-digested vector pUC19

Approximately 2.5 µg of plasmid pUC19 (available from Bethesda Research Laboratories, Gaithersburg, MD 20877) [restriction/function map provided in Figure 1] were digested with HindIII as described in example 2-A (total reaction volume was reduced to 20 µl and the amount of enzyme used was reduced to 2 µl). The restriction enzyme digestion was incubated at 37°C for approximately 2 hours. To terminate the activity of the restriction enzyme, the reaction was extracted with an equal volume of phenol:chloroform and the DNA in the aqueous phase was transferred to a fresh tube and purified by ethanol precipitation. The resultant DNA pellet was resuspended in 20 µl of water and constituted ~2 µg of the desired HindIII-digested pUC19 plasmid vector. Vector DNA obtained in this manner was stored at -20°C.

C. Final construction of plasmids pEWSA8 and pEWSA37 and *Esherichia coli* strains K12 DH5α/pEWSA8 and DH5α/pEWSA37.

Two µl of the ~4.0 kb HindIII restriction fragment prepared in example 2-A were mixed with 1 µl of HindIII-digested pUC19 plasmid vector DNA (prepared in example 2-B). This mixture was ligated in a 20 µl reaction containing the specified DNA fragments (3 µl), 2 µl of 10X ligase buffer (0.5 M Tris-HCl, pH 7.5, and 100 mM $MgCl_2$), 2 µl of 5 mM ATP, 1 µl of 6 µg/ml BSA, 12 µl of glass-distilled water, and 1 µl (1 Weiss unit) of T4 DNA ligase (New England Biolabs). The reaction was incubated approximately 18 hours at 15°C. The ligated DNA constituted the desired plasmids pEWSA8 and pEWSA37 along with other ligation products.

Transformation competent Escherichia coli K12 DH5α (MAX Efficiency) were purchased from Bethesda Research Laboratories (Gaithersburg, MD) and transformed with the ligation reaction mixture constituting pEWSA8 and pEWSA37 in substantial accordance with the manufacturer's directions except that the DNA was

in a volume of 20 μl. Post-transformation, the cells were distributed to agar plates containing ampicillin (100 μg/ml), 40 μg/ml 5-bromo-4-chloro-3-indolyl-β-D-galactoside (X-gal) and 0.1 M isopropylthio-β-galactoside (IPTG), and the plates were incubated overnight at 37°C.

Escherichia coli transformants containing a plasmid with an insert were identified by inactivation of the β-galactosidase gene present on pUC19. Those transformants harboring an insert in the HindIII site of pUC19 formed colorless colonies while those uninterrupted formed blue colonies under these cultural conditions. Miniprep plasmid DNA prepared from colorless colonies was screened by restriction endonuclease digestion in substantial accordance with the procedures described by Maniatis et al.(1982, "Molecular Cloning, A Laboratory Manual, Cold Spring Harbor Laboratory). One isolate was recovered which contained plasmid pEWSA8 and another contained plasmid pEWSA37. The cells containing these plasmids are designated DH5α/pEWSA8 and DH5α/pEWSA37 respectively. The only difference in these plasmids is the orientation of the HindIII restriction fragment containing the mecA-27R gene. Restriction/function maps of plasmids pEWSA8 and pEWSA37 are provided in Figures 2 and 3 respectively. DH5α/pEWSA37 has been deposited with the Northern Regional Research Laboratories National Culture Collection and is available under the accession number B-18753.

## Example 3

### A. Expression of the *mec*A-27R gene from *Staphylococcus aureus* on plasmids pEWSA37 and pEWSA8 in *Escherichia coli*

Escherichia coli transformants obtained with plasmids pEWSA37 and pEWSA8 express detectable levels of the active mecA-27R gene product. In these transformants, mecA-27R gene expression is driven by the native Staphylococcus aureus promoter. A sample of these cultures were grown at 37°C overnight in 200 ml of TY broth (containing 100 μg/ml of ampicillin) in a gyrotory incubator (250 rpm). Cells were harvested from these overnight cultures by centrifugation in a Beckman Sorvall centrifuge (GSA rotor, 10,000 rpm, 4°C, 10 minutes). After removal of the supernatant, cell membrane preparations from these cells were analyzed for the presence of the mecA-27R gene product, PBP2A-27R.

### B. Preparation of *Escherichia coli* strain DH5α/pEWSA37 and DH5a/pEWSA8 cell membranes.

Escherichia coli cells harboring plasmid pEWSA37 or plasmid pEWSA8 harvested in Example 3-A were washed by resuspension in 10 mM sodium phosphate buffer at pH 7.0. Cells were harvested by centrifugation. One more wash was performed, upon completion, the pelleted cells were resuspended in fresh sodium phosphate buffer containing 50 μg/ml of DNaseI per ml, 1 mM phenylmethylsulfonyl flouride, and 0.14 mM 2-mercaptoethanol. The cells were broken by passing three times through a French press at 16,000 lb/in$^2$ (SLM Aminco, Urbana, Illinois). After centrifugation to remove unbroken bacterial cells, supernatants were loaded onto a discontinuous sucrose gradient composed of 9 ml of 58% sucrose overlaid with 9 ml of 52% sucrose in 10 mM sodium phosphate buffer, pH 7.0, and centrifuged for 18 hours at 135,000 x g. The 52% sucrose fraction containing the inner membrane was collected and centrifuged at 135,000 x g for 1 hour. Membrane preparations from each culture were suspended in 50 mM sodium phosphate buffer, pH 7.0, at 12 mg of protein per ml, and were stored at -70°C.

### C. Assay of *Escherichia coli* membrane proteins for the presence the *mec*A-27R gene product, PBP2A-27R.

The cell membrane preparations made in Example 3B were analyzed for PBP's by labeling with $^{125}$I-Penicillin V (IPV) in substantial accordance with the procedure described by Preston et al., (Antimicrobial Agents and Chemotherapy, 1990, pages 718-721). A 4 μl aliquot of membrane preparation was incubated with 2 μl of sodium clavulanate (concentration of sodium clavulanate ranged from 1 to 243 μg/ml) and 6 μl of $^{125}$IPV (96 μg/ml) for 15 minutes at 35°C. Sodium clavulanate was included to inhibit the activity of β-lactamases produced by the host cells. The reaction was stopped by the addition of 12 μl of blue mix (120 mg of cold IPV per ml-20% sarcosyl-polyacrylamide gel electrophoresis sample buffer, 1:2:12). Proteins were separated by sodium dodecyl sulfate-polyacrylamide gel electrophoresis, using 8% acrylamide-0.21% N,N'-methylenebisacrylamide. Dried gels were exposed to Kodak X-OMAT film without fluorographic enhancement at 24°C for 8 to 24 hours. Escherichia coli membrane preparations which did not contain plasmid pEWSA37 or pEWSA8 did not exhibit a radiolabeled band at a position consistent with PBP2A-27R even with the highest level of sodium clavulanate. In contrast, transformed cells carrying pEWSA37 or pEWSA8 did exhibit a distinct radioactive band at a position consistent with purified PBP2A-27R. The intensity of that band increased with increasing levels of sodium clavulanate used in the reaction. Therefore E. coli, cells were recovered which produced the

Staphylococcus aureus PBP2A-27R protein.

Example 4

Expression of altered forms of the *mec*A-27R gene in *Escherichia coli*

The mecA-27R gene product, PBP2A-27R, is a penicillin binding protein which is membrane associated. Analysis of the amino acid sequence deduced from the DNA sequence suggested that a transmembrane region was located at the amino terminus of the protein, approximately the first 22 amino acids. Therefore, in-vitro site-specific mutagenesis was employed to modify the mecA-27R gene and thus remove the putative transmembrane region from the resultant PBP2A-27R protein. In addition to removal of the transmembrane region, a useful restriction site was incorporated at the initiation codon to allow expression in Escherichia coli based vectors. The new PBP2A-27R protein encoded by this modified mecA-27R gene should not be associated with the cell membrane when expressed in Escherichia coli and thus be readily available for structural studies and use in assays directed towards discovery new compounds having therapeutic efficacy against MRS. In addition, other mutants were formed which included a metal binding region to help purify the protein after expression in E. coli.

A. Preparation of *Hind*III/*Xba*I-digested vector M13mp18 RF DNA

Approximately 3.0 μg of M13mp18 RF DNA (available from New England Biolabs [NEB], see Figure 4) were digested in 100 μl of HindIII buffer with 1 μl (~12 units) of restriction enzyme HindIII for 90 minutes at 37°C. The reaction was terminated by extraction with phenol:chloroform and the DNA, recovered in the aqueous phase was concentrated by ethanol precipitation. The DNA pellet was resuspended in 20 μl of sterile water. Another restriction enzyme digestion was performed on the concentrated DNA. This time XbaI buffer and the restriction enzyme XbaI (1 μl, ~12 units) were used in place of HindIII. After 90 minutes digestion at 37°C the enzyme was inactivated by extraction with phenol:chloroform and concentrated by ethanol precipitation. The pellet was resuspended in 20μl of water and constituted ~2 μg of the desired HindIII/XbaI-digested M13mp18 vector. Vector DNA obtained in this manner was stored at -20°C.

B. Preparation of *Hind*III/*Xba*-digested vector M13mp19 RF DNA

Approximately 3.0 μg of M13mp19 RF DNA (available from New England Biolabs [NEB], see Figure 5) were digested in 100 μl of HindIII buffer with 1 μl (~12 units) of restriction enzyme HindIII for 90 minutes at 37°C. The reaction was terminated by extraction with phenol:chloroform and the DNA, recovered in the aqueous phase was concentrated by ethanol precipitation. The DNA pellet was resuspended in 20 μl of sterile water. Another restriction enzyme digestion was performed on the concentrated DNA. This time XbaI buffer and the restriction enzyme XbaI (1 μl, 12 units) were used in place of HindIII. After 90 minutes digestion at 37°C the enzyme was inactivated by extraction with phenol:chloroform and concentrated by ethanol precipitation. The pellet was resuspended in 20μl of water and constituted ~2 μg of the desired HindIII/XbaI-digested M13mp19 vector. Vector DNA obtained in this manner was stored at -20°C.

C. Preparation of *Hind*III/*Xba*I restriction fragments containing the 5′ and 3′ end of the *mec*A-*27R* gene.

The approximately 4.0 kb HindIII restriction fragment containing the mecA-27R gene was obtained as described in example 2-A. This restriction fragment was further digested in 100 μl of XbaI buffer containing 1 μl of the restriction enzyme XbaI at 37°C for 90 minutes. The reaction was terminated by extraction with phenol:chloroform and the DNA, recovered in the aqueous phase was concentrated by ethanol precipitation. The DNA pellet was resuspended in 20 μl of sterile water and constituted the desired restriction fragments: one approximately 1.82 kb in length which covered approximately 738 bp of the 5′ end of the mecA-27R gene plus additional upstream sequence and the other fragment was approximately 2.1 kb in length and included approximately 1269 bp of the 3′ end of the mecA-27R gene plus DNA sequence downstream of mecA-27R.

The 1.82 and 2.1 kb HindIII/XbaI restriction fragments described above were separated on a 0.8% agarose gel. These DNA fragments were then removed from the agarose gel as described in Example 2-A. The final pellet of DNA, composed of the respective restriction fragments, was resuspended in 20 μl of sterile water and constituted approximately 2 μg of the 1.82 kb HindIII/XbaI restriction fragment and approximately 2 μg of the 2.1 kb HindIII/XbaI restriction fragment.

D. Final construction of plasmid pEWM13-1 and *E.coli* K12 JM109/pEWM13-1

Approximately 0.2 μg of HindIII/XbaI digested M13mp18 DNA prepared in Example 4-A and approximately 0.4 μg of the 1.82 kb HindIII/XbaI restriction fragment containing the 5′ end of the mecA-27R gene prepared in Example 4-C were mixed and ligated in the presence of T4 DNA ligase.

Competent Escherichia coli K12 JM109 ("Epicurean Coli™") were purchased from Stratagene (3770 Tansy Street, San Diego, CA 92121) and transformed with the ligation mixture constituting plasmid DNA pEWM13-1 in substantial accordance with the manufacturer's directions, except that the DNA was in a volume of 20 μl and no dilution into medium or expression time was necessary. Post-transformation, the cells were distributed in ~1, 10, 20, 40, and 50 μl samples to 13 X 100 mm sterile glass tubes containing 0.25 ml/tube E. coli 1M109 in logarithmic growth phase. To these tubes were added 3 ml of top agar (L-broth with 0.8% agar kept molten at 45°C). The cell-top agar mixture was then poured onto L-agar plates containing 40 μg/ml 5-bromo-4-chloro-3-indolyl-β-D-galactoside (X-gal) and 0.1 M isopropylthio-β-galactoside (IPTG), and the plates were incubated at 37°C overnight. (For more detailed descriptions and explanations of M13 procedures, see M13 Cloning/Dideoxy Sequencing Instruction Manual, Bethesda Research Laboratories [BRL], Life Technologies, Inc., Gaithersburg, MD 20877.) Transformants containing an insertion of the desired approximately 1.82 kb HindIII/XbaI fragment carrying the 5′ end of the mecA-27R gene were identified by insertional inactivation of β-galactosidase activity (colorless plaque phenotype) and restriction enzyme analysis of replicative form (RF) DNA. For screening purposes, clear plaques were plugged from the plate overlay with a Pasteur pipette into 3 ml per plaque of early logarithmic growth phase E. coli K12 JM109 cells. Cultures were incubated from 6 to 18 hours at 37°C with aeration.

Following this incubation, 1.5 ml of each culture was pelleted in separate 1.5 ml Eppendorf tubes. The supernatants, which contained phage particles, were decanted into fresh tubes and stored at 4°C to serve as a source of phage inoculum. Replicative form DNA was prepared from the cell pellets in substantial accordance with the teaching of the alkaline plasmid preparation procedure of Birnboim and Doly, 1979, Nuc. Acids Res. 7(6):1513-1523, with the following exceptions. The procedure was scaled up such that 1.5 volumes of Solutions I, II, and III are used, and the cleared lysate was extracted once with an equal volume of $CHCl_3$. The DNA was then precipitated by the addition of 0.4 volumes of isopropanol and incubated at room temperature for 20 minutes. The DNA was collected by centrifugation and then precipitated with ethanol out of 0.3 M NaOAc. Restriction analysis of the DNA thus isolated was used to confirm the presence of the approximately 1.82 kb HindIII/XbaI restriction fragment. By this method, E. coli K12 JM109/pEWM13-1 cells were identified; these cells were then used as a source of phage and plasmid pEWM13-1 for site-specific mutagenesis and DNA sequencing. Figure 6 presents a restriction/function map of plasmid pEWM13-1.

E. Final construction of plasmid pEWM13-7 and *E. coli* K12 JM109/pEWM13-7

The 5′ end of mecA-27R was also ligated into M13mp19 in order to obtain the opposite orientation of this fragment. Approximately 0.2 μg of HindIII/XbaI digested M13mp19 DNA prepared in Example 4-B and approximately 0.4 μg of the 1.82 kb HindIII/XbaI restriction fragment containing the 5′ end of the mecA-27R gene prepared in Example 4-C were mixed and ligated in the presence of T4 DNA ligase.

Transformation of E. coli with this ligation mixture and screening of plaques was performed as described in Example 4-D. E. coli JM109/pEWM13-7 cells were identified as described in Example 4-D. These cells were the source of single stranded DNA for sequencing reactions (isolation procedure for single stranded phage DNA is described in Example 4-H). A restriction/function map of plasmid pEWM13-7 is provided in Figure 7.

F. Final construction of plasmid pEWM13-9 and *E. coli* K12 JM109/pEWM13-9

Approximately 0.2 μg of HindIII/XbaI digested M13mp18 DNA prepared in Example 4-A and approximately 0.4 μg of the 2.1 kb HindIII/XbaI restriction fragment containing the 3′ end of the mecA-27R gene prepared in Example 4-C were mixed and ligated in the presence of T4 DNA ligase.

Transformation of E. coli with this ligation mixture and screening of plaques was performed as described in Example 4-D. E. coli JM109/pEWM13-9 cells were identified as described in Example 4-D. These cells were used as the source of single stranded DNA for sequencing reactions (isolation procedure for single stranded phage DNA is described in Example 4-H). A restriction/function map of plasmid pEWM13-9 is provided in Figure 8.

G. Final construction of plasmid pEWM13-12 and *E. coli* K12 JM109/pEWM13-12.

Approximately 0.2 μg of HindIII/XbaI digested M13mp19 DNA prepared in Example 4-B and approximately 0.4 μg of the 2.1 kb HindIII/XbaI restriction fragment containing the 3′ end of the mecA-27R gene prepared in Example 4-C were mixed and ligated in the presence of T4 DNA ligase.

Transformation of E. coli with this ligation mixture and screening of plaques was performed as described in Example 4-D. E. coli JM109/pEWM13-12 cells were identified as described in Example 4-D. These cells were used as the source of single stranded DNA for sequencing reactions (isolation procedure single stranded phage DNA is described in Example 4-H). A restriction/function map of plasmid pEWm13-12 is provided in Figure 9.

H. Preparation of single-stranded phage pEWM13-1 DNA for site-specific mutagenesis to construct phage pEWM13-21.

A 10 mL culture of early logarithmic growth phase Escherichia coli K12 CJ236 (Bio-Rad Laboratories, 1414 Harbor Way South, Richmond, CA 94804) was inoculated with ~200 μl of phage stock pEWM13-1 (prepared in Example 4-D) and incubated ~18 hours at 37°C with aeration. The culture was transferred to a sterile centrifuge tube and centrifuged to pellet the cells (Beckman Sorvall centrifuge, SS34 rotor, 6,000 rpm, 4°C, 10 minutes), the resulting supernatant was transferred to a new tube and recentrifuged (same conditions). The supernatant was again decanted to a fresh tube. One mL of a solution of 25% polyethylene glycol 6000 in 3 M NaCl was added to the supernatant, which was then incubated for 15 minutes at room temperature. The resulting mixture was centrifuged for 30 minutes at 10,000 rpm in a SS34 rotor at 4°C (Beckman Sorvall centrifuge). The pellet obtained by this centrifugation contained the single-stranded phage pEWM13-1 and was dissolved in 400 μl of TE buffer. The solution was extracted first with $CHCl_3$ and then with TE-saturated phenol to liberate single-stranded DNA. The phenol was allowed to stay in contact with the aqueous phase for 15 minutes. The solution was then extracted twice with a mixture of TE-saturated phenol:$CHCl_3$ (1:1, v/v), and twice with $CHCl_3$ alone. The single-stranded DNA was then precipitated out of 0.3 M NaOAc, collected by centrifugation, and the resulting pellet resuspended in 100 μl of 0.1X TE buffer. This solution constituted ~5 μg of single-stranded phage pEWM13-1 DNA.

I. Site specific mutagenesis of phage pEWM13-1 to create phage pEWM13-22, pEWM13-23 and pEWM24.

The single-stranded DNA fragments used in the mutagenesis were synthesized on an automated DNA synthesizer. The mutagenesis primers were designated as follows: (1) 2ALOOP-1, a single-stranded DNA fragment 48 nucleotides in length that is identical to 21 bases immediately 5′ of the translation initiation codon of mecA-27R, incorporates an NcoI restriction site at the initiation codon, and is identical with 21 bases beginning with the codon which specifies amino acid 23 of PBP2A-27R with the DNA sequence:

NcoI

5' - GTCGGGTTTGGTATATATTTTCCATGGTGTATGCTTCAAAAGATAAAG - 3'

(2) 2ALOOP-2, a single-stranded DNA fragment 63 nucleotides in length that is identical to 21 bases immediately 5′ of the translation initiation codon of mecA-27R , incorporates an NcoI restriction site at the initiation codon, encodes a metal chelating peptide, and is identical to 19 bases of mecA-27R beginning with the codon which specifies amino acid 23 of PBP2A-27R with the DNA sequence:

NcoI

5' - GTCGGGTTTGGTATATATTTTCCATGGGTCATTGGCACCATCAC
TATGCTTCAAAAGATAAAG - 3'

(3) 2ALOOP-3, a single-stranded DNA fragment 66 nucleotides in length that is identical to 21 bases immediately 5′ of the translation initiation codon of mecA-27R, incorporates an NcoI restriction site at the initiation codon, encodes a metal chelating peptide which ends with a codon for proline to facilitate dipeptidase removal of the metal chelating peptide, and is identical with 19 bases of mecA-27R beginning with the codon which specifies amino acid 23 of PBP2A-27R with the DNA sequence:

NcoI

5' - GTCGGGTTTGGTATATATTTTCCATGGGTCATTGGCACCATTGGCCT TATGCTTCAAAAGATAAAG - 3'

In-vitro mutagenesis with each of the above oligonuceotides (2ALOOP-1, 2ALOOP-2 and 2ALOOP-3) was performed in substantial accordance with the procedure described in the MUTAGENE™ *IN VITRO* MUTAGENESIS KIT instruction manual (Catalog number 170-3571, Bio-Rad Laboratories, 1414 Harbour Way South, Richmond, CA 94804). The template used was single stranded phage DNA from pEWM13-1. Plaques containing phage DNA with the desired mutations were identified by restriction analysis of RF DNA isolated with a Qiagen Plasmid Midi Kit in substantial accordance with the manufacturer's recommendations (Qiagen Inc., 9259 Eton Avenue, Chatsworth, CA 91311). Isolates were obtained which contained RF DNA that represented each of the desired mutations. These cultures were designated JM109/pEWM13-21 (contains the mutation induced by 2ALOOP-1), JM109/pEWM13-22 (contains the mutation induced by 2ALOOP-2), and JM-109/pEWM13-23 (contains the mutation induced by 2ALOOP-3). Restriction site/function maps of each of these plasmids are presented in Figures 10, 11 and 12, respectively. Each of these cultures was used as a source of RF DNA containing an approximately 0.8 kb NcoI/XbaI restriction fragment possessing a modified form of the 5′ end of the mecA-27R gene.

J. Isolation of the *Hind*III/*Xba*I restriction fragments containing mutagenized forms of the 5′ end of the *mec*A-27R gene.

Escherichia coli cultures JM109/pEWM13-21, JM109/pEWM13-22 and JM109/pEWM13-23 were inoculated into 200 ml of TY broth and incubated overnight with aeration. The cells were harvested from each overnight culture by centrifugation (8,000 rpm, 4°C, GSA rotor, 10 minutes, Beckman Sorvall centrifuge). The supernatants were discarded and the replicative form of pEWM13-21, pEWM13-22 and pEWM13-23 were isolated from the pelleted cells with a Qiagen Plasmid Midi Kit in substantial accordance with the manufacturer's recommendations (Qiagen Inc., 9259 Eton Avenue, Chatsworth, CA 91311). The HindIII/XbaI restriction fragment comprising the 5′ end of the mecA-27R gene from each of these plasmids was isolated as described in Example 2-A. The resultant purified DNA restriction fragments are illustrated in Figure 32. Each fragment (A,B,C and D as illustrated in Figure 32) was resuspended in approximately 20 μl of sterile water and comprised approximately 2 μg of the desired restriction fragments.

K. Preparation of *Hind*III/*Xba*I digested pUC19 plasmid DNA

Approximately 3.0 μg of pUC19 DNA (Bethesda Research Laboratories, Gaithersburg, MD 20877, Figure 1) were digested in 100 μl of HindIII buffer with 1 μl (~12 units) of restriction enzyme HindIII for 90 minutes at 37°C. The reaction was terminated by extraction with phenol:chloroform and the DNA, recovered in the aqueous phase was concentrated by ethanol precipitation. The DNA pellet was resuspended in 20 μl of sterile water. Another restriction enzyme digestion was performed on the concentrated DNA. This time XbaI buffer and the restriction enzyme XbaI (1 μl, 12 units) were used in place of HindIII. After 90 minutes digestion at 37°C the enzyme was inactivated by extraction with phenol:chloroform and concentrated by ethanol precipitation. The pellet was resuspended in 20 μl of water and constituted ~2 μg of the desired HindIII/XbaI-digested pUC19 vector. Vector DNA obtained in this manner was stored at -20°C.

L. Construction of plasmids pEWMSA24, pEWSA25, and pEWSA25 and *Escherichia coli* strains DH5α-/pEWSA24, DH5α/pEWSA25 and DH5α/pEWSA26.

A detailed description of the construction of plasmid pEWSA24 and Escherichia coli strain DH5α/pEWSA24 is provided here. Two μl of the ~1.74 kb HindIII/XbaI restriction fragment A prepared in Example 4-J were mixed with 1 μl of HindIII/XbaI-digested pUC19 plasmid vector DNA (prepared in example 4-K). This mixture was ligated in a 20 μl reaction containing the specified DNA fragments (3 μl), 2 μl of 10X DNA ligase buffer (0.5 M Tris-HCl, pH 7.5, and 100 mM $MgCl_2$), 2 μl of 5 mM ATP, 1 μl of 6 μg/ml BSA, 12 μl of glass-distilled water, and 1 μl (1 Weiss unit) of T4 DNA ligase (New England Biolabs). The reaction was incubated approximately 18 hours at 15°C. The ligated DNA constituted the desired plasmid pEWSA24 and other ligation products.

Transformation competent Escherichia coli K12 DH5α(MAX Efficiency) were purchased from Bethesda Research Laboratories (Gaithersburg, MD) and transformed with the ligation reaction mixture constituting plasmid pEWSA24 in substantial accordance with the manufacturer's directions except that the DNA was in a volume of 20 μl. Post-transformation, the cells were distributed to agar plates containing ampicillin (100 μg/ml), 40 μg/ml 5-bromo-4-chloro-3-indolyl-β-D-galactoside (X-gal) and 0.1 M isopropylthio-β-galactoside (IPTG), and the plates were incubated overnight at 37°C.

Escherichia coli transformants containing a plasmid with an insert were identified by inactivation of the β-galactosidase gene present on pUC19. Those transformants harboring an insert between the HindIII and XbaI restriction sites of pUC19 formed colorless colonies while those uninterrupted formed blue colonies under these cultural conditions. Miniprep plasmid DNA prepared from colorless colonies was screened by restriction endonuclease digestion in substantial accordance with the procedures described by Maniatis et al.(1982, "Molecular Cloning, A Laboratory Manual, Cold Spring Harbor Laboratory). One isolate was recovered which contained plasmid pEWSA24. The isolate containing this plasmid was designated DH5α/pEWSA24.

Plasmids pEWSA25 and pEWSA26 and Escherichia coli strains DH5α/pEWSA25, DH5α/pEWSA26 were constructed according to the method described immediately above for plasmid pEWSA24 and E. coli strain DH5α/pEWSA24. In the case of pEWSA25 fragment B from Example 4-J was incorporated into pUC19. For plasmid pEWSA26, fragment C from Example 4-J was incorporated into pUC19. Restriction/function maps for plasmids pEWSA24, pEWSA25 and pEWSA26 are provided in Figures 13, 14, 15 and 28 respectively.

M. Isolation of the approximately 2.1 kb *Xba*I restriction from plasmid pEWSA37 containing the 3′ end of the *mec*A-27R gene.

Plasmid DNA purified from Escherichia coli DH5α/pEWSA37 prepared in Example 2-C was digested to completion with the restriction endonuclease XbaI as follows. Ten μl (~10 μg) of plasmid DNA pEWSA37 were mixed with 33 μl sterile water, 5 μl 10X XbaI restriction enzyme buffer, and 2 μl of the restriction enzyme XbaI (24 units). The restriction enzyme reaction was incubated for approximately 3 hours at 37°C. The DNA fragments formed in the restriction endonuclease digestion were separated by electrophoresis on a 0.8% agarose gel until the desired approximately 2.1 kb XbaI fragment was clearly separated from the other digestion products. The electrophoresed DNA was visualized by staining with ethidium bromide (0.5 μg/ml) and exposing the stained gel to long-wave UV light. After the fragment was located, a small slit was made in the gel in front of the ~2.1 kb XbaI fragment, and a piece of Schleicher and Schuell (Keene, NH 03431) DEAE membrane cut to the appropriate size was placed in the slit. Upon further electrophoresis, the DNA non-covalently bound to the DEAE membrane. After the fragment was bound to the DEAE membrane, the membrane was removed and rinsed with low salt buffer (100 mM KCl; 0.1 mM EDTA; and 20 mM Tris-HCl, pH 8.0). Next, the membrane was placed in a small tube and immersed in high salt buffer (1 M NaCl; 0.1 mM EDTA; and 20 mM Tris-HCl, pH 8.0) and then incubated at 65°C for 30 minutes to remove the DNA from the DEAE paper. After the 65°C incubation, the incubation buffer, which contained the DNA, was collected, and the membrane was rinsed with high salt buffer. The rinse buffer was pooled with the incubation buffer

The volume of the high salt-DNA solution was adjusted so that the NaCl concentration was 0.25 M, and then three volumes of cold, absolute ethanol were added to the solution. The resulting solution was mixed and placed at -70°C for 30 minutes. The solution was then centrifuged at 10,000 rpm in an HB-4 rotor for 25 minutes at -15°C in a Beckman sorvall centrifuge. The supernatant was discarded and the pellet composed of DNA was air dried. The DNA pellet was resuspended in water and ethanol precipitated once more to remove residual salt as described in example 1-B. This time the air dried DNA pellet was resuspended in 20 μl of water and constituted the desired 2.1 kb XbaI restriction fragment containing the 3' end of the mecA-27R gene from plasmid pEWSA37.

N. Preparation of *Xba*I digested pEWSA24, pEWSA25 and pEWSA26 plasmid DNA.

A detailed description for preparation of plasmid pEWSA24 will be provided here. Plasmid DNA was isolated from Escherichia coli strain DH5α/pEWSA24 using a Qiagen Plasmid Midi Kit in substantial accordance with the manufacturer's recommendations (Qiagen Inc., 9259 Eton Avenue, Chatsworth, CA 91311). Approximately 3.0 μg of pEWSA24 DNA (Bethesda Research Laboratories, Gaithersburg, MD 20877) were digested in 100 μl of XbaI buffer with 1 μl (~12 units) of restriction enzyme XbaI for 90 minutes at 37°C. The reaction was terminated by extraction with phenol:chloroform and the DNA, recovered in the aqueous phase was concentrated by ethanol precipitation. The resulting pellet was resuspended in 20μ1 water and constituted ~2 μg of the desired XbaI-digested pEWSA24 vector. Vector DNA obtained in this manner was stored at - 20°C.

In precisely the same manner as described above for plasmid pEWSA24, XbaI digests were performed on plasmid DNA isolated from Escherichia coli DH5α/pEWSA25, DH5α/pEWSA26 and DH5α/pEWSA40 prepared in Example 4-L.

O. Construction of plasmids pEWSA27, pEWSA28, and pEWSA29 and *Escherichia coli* strains DH5a-/pEWSA27, DH5a/pEWSA28 and DH5a/pEWSA29.

A detailed description for the construction of plasmid pEWSA27 will be provided here. Two µl of the ~2.1 kb XbaI restriction fragment prepared in example 4-M were mixed with 1 µl of XbaI-digested pEWSA24 plasmid vector DNA (prepared in example 4-N). This mixture was ligated in a 20 µl reaction containing the specified DNA fragments (3 µl), 2 µl of 10X DNA ligase buffer (0.5 M Tris-HCl, pH 7.5, and 100 mM $MgCl_2$), 2 µl of 5 mM ATP, 1 µl of 6 µg/ml BSA, 12 µl of glass-distilled water, and 1 µl (1 Weiss unit) of T4 DNA ligase (New England Biolabs). The reaction was incubated approximately 18 hours at 15°C. The ligated DNA constituted the desired plasmid pEWSA27 and other ligation products.

Transformation competent Escherichia coli K12 DH5α (MAX Efficiency) were purchased from Bethesda Research Laboratories (Gaithersburg, MD) and transformed with the ligation reaction mixture constituting plasmid pEWSA24 in substantial accordance with the manufacturer's directions except that the DNA was in a volume of 20 µl. Post-transformation, the cells were distributed to agar plates containing ampicillin (100 µg/ml) and the plates were incubated overnight at 37°C.

Escherichia coli transformants containing a plasmid with an insert in the XbaI site of pEWSA24 were identified by restriction endonuclease digestion of miniprep plasmid DNA prepared using a Qiagen Plasmid Midi Kit in substantial accordance with the manufacturer's recommendations (Qiagen Inc., 9259 Eton Avenue, Chatsworth, CA 91311). Isolates recovered which contained plasmids with an insert in the XbaI site were then screened for orientation of the XbaI insert. The restriction endonuclease sites XbaI and BamHI were particularly useful for determination of the orientation of the inserted DNA. Plasmids containing the insert in the desired orientation would produce an approximately 0.52 kb restriction fragment plus other digestion products when digested with both of these enzymes. If the insert was in the wrong orientation, the 0.52 kb restriction fragment was present plus a 1.58 kb SphI restriction fragment which was not present in the correct orientation. An isolate containing the desired plasmid was found and designated pEWSA27.

Plasmids pEWSA28 and pEWSA29 and Escherichia coli strains DH5α/pEWSA28 and DH5α/pEWSA29 were constructed according to the method described immediately above for plasmid pEWSA27 and E. coli strain DH5α/pEWSA27. Plasmid pEWSA25 was used in the construction of pEWSA28. Plasmid pEWSA26 was used in the construction of plasmid pEWSA29. Restriction site and function maps for plasmids pEWSA27, pEWSA28 and pEWSA29 are provided in Figures 16, 17, 18 and 28 respectively. These plasmids contain the various mutated forms of the intact mecA-27R gene.

P. Isolation of *NcoI*/*Bam*HI restriction fragments containing mutant forms of *mec*A-27R for insertion into *Escherichia coli* expression vectors.

Escherichia coli cultures containing mutant forms of the mecA-27R gene (*i.e.* DH5α/pEWSA27, DH5α-/pEWSA28, and DH5α/pEWSA29) prepared in Example 4-O were grown overnight in 200 ml of TY broth. The cells were harvested by centrifugation from each of the overnight cultures (GSA rotor, 8,000 rpm, 10 minutes, 4°C, in a Beckman Sorvall centrifuge). Plasmid DNA was extracted from each pellet of harvested cells using a Qiagen Plasmid Midi Kit in substantial accordance with the manufacturer's recommendations (Qiagen Inc., 9259 Eton Avenue, Chatsworth, CA 91311). Plasmid DNA isolated in this manner was resuspended in 200 µl sterile water and constituted plasmids pEWSA27, pEWSA28, and pEWSA29.

Aliquots of approximately 10 µg of each of the plasmids isolated above were digested in 100 µl of NcoI buffer with 1 µl (~12 units) of restriction enzyme NcoI for 90 minutes at 37°C. The reaction was terminated by extraction with phenol:chloroform and the DNA, recovered in the aqueous phase was concentrated by ethanol precipitation. The DNA pellet was resuspended in 20 µl of sterile water. Another restriction enzyme digestion was performed on the concentrated DNA. This time BamHI buffer and the restriction enzyme BamHI (1 µl, 12 units) were used in place of NcoI. After 90 minutes digestion at 37°C the reactions were loaded onto a 0.8% agarose gel and electrophoresed until the desired NcoI/BamHI fragments (approximately 2.8 kb) were clearly separated from the other digestion products. The desired fragments were isolated from the agarose gel in substantial accordance with Example 2-A. The DNA products were resuspended in 50 µl of sterile water and constituted the desired NcoI/BamHI restriction fragments carrying the mutant forms of the mecA-27R gene from plasmids pEWSA27 (Fragment A, lacks the transmembrane region and contains an NcoI site), pEWSA28 (Fragment B, lacks the transmembrane region, contains an NcoI site, and a chelating peptide) and pEWSA29 (Fragment C, lacks the transmembrane region, contains an NcoI site, and a proline chelating peptide).

Q. Preparation of *NcoI/BamHI*-digested pOW241 plasmid DNA and isolation of the approximately 6.1 kb fragment.

Plasmid pOW241 is an expression vector useful for production of foreign proteins in Escherichia coli. Expression of foreign proteins in this plasmid is dependent upon induction of the thermal-inducible lambda pL promoter. This plasmid also contains a unique NcoI site at the translation initiation site and a BamHI site downstream from that position which is useful for the 3′ end of restriction fragments containing genes of interest. In pOW241 the pcbC gene occupies the region between the NcoI site and the BamHI site. The pcbC gene must be removed to make it useful for the expression of the various forms of the mecA-27R gene. A restriction site and function map of plasmid pOW241 is provided in Figure 19. Plasmid pOW241 is a derivative of plasmid pCZ336 (Samson et al., 1987, Bio/Technology, Vol. 5, No. 11, pages 1207-1214).

Approximately 3.0 µg of pOW241 plasmid DNA were digested in 100 µl of NcoI buffer with 1 µl (-12 units) of restriction enzyme NcoI for 90 minutes at 37°C. The reaction was terminated by extraction with phenol:chloroform and the DNA, recovered in the aqueous phase was concentrated by ethanol precipitation. The DNA pellet was resuspended in 20 µl of sterile water. Another restriction enzyme digestion was performed on the concentrated DNA. This time BamHI buffer and the restriction enzyme BamHI (1 µl, 12 units) were used in place of NcoI. After 90 minutes digestion at 37°C the reaction was loaded onto a 0.8% agarose gel and electrophoresed until the desired approximately 6.1 kb fragment was clearly separated from other digestion products. The approximately 6.1 kb fragment was isolated from the gel in substantial accordance with the method described in Example 2A. The DNA pellet recovered was resuspended in 20µ1 water and constituted ~2 µg of the desired XbaI/BamHI-digested pOW241 vector lacking the pcbC gene. Vector DNA obtained in this manner was stored at - 20°C.

R. Construction of plasmids pEWSA30, pEWSA31, and pEWSA32, and *Escherichia coli* strains DH5α-/pEWSA30, PH5α/pEWSA31 and DH5α/pEWSA32.

A detailed description for the construction of plasmid pEWSA30 and Escherichia coli strain DH5α-/pEWSA30 will be provided here. Two µl of the ~2.8 kb NcoI/BamHI restriction fragment from pEWSA27 (prepared in example 4-P) were mixed with 1 µl of NcoI/BamHI-digested pOW241 plasmid vector DNA (prepared in example 4-Q). This mixture was ligated in a 20 µl reaction containing the specified DNA fragments (3 µl), 2 µl of 10X DNA ligase buffer (0.5 M Tris-HCl, pH 7.5, and 100 mM $MgCl_2$), 2 µl of 5 mM ATP, 1 µl of 6 µg/ml BSA, 12 µl of glass-distilled water, and 1 µl (1 Weiss unit) of T4 DNA ligase (New England Biolabs). The reaction was incubated approximately 18 hours at 15°C. The ligated DNA constituted the desired plasmid pEWSA30 and other ligation products.

Transformation competent Escherichia coli K12 DH5α-(MAX Efficiency) were purchased from Bethesda Research Laboratories (Gaithersburg, MD) and transformed with the ligation reaction mixture constituting plasmid pEWSA30 in substantial accordance with the manufacturer's directions except that the DNA was in a volume of 20 µl. Post-transformation, the cells were distributed to agar plates containing L-agar and tetracycline (100 µg/ml), and the plates were incubated overnight at 30°C.

Escherichia coli transformants containing a plasmid with an insert were identified by restriction endonuclease digestion of miniprep plasmid DNA prepared using a Qiagen Plasmid Midi Kit in substantial accordance with the manufacturer's recommendations (Qiagen Inc., 9259 Eton Avenue, Chatsworth, CA 91311). Isolates recovered which contained an approximately 2.8 kb NcoI-BamHI restriction fragment were further analyzed by restriction analysis to verify that the proper fragment had been inserted. An isolate found possessing the proper plasmid was designated DH5α/pEWSA30. A restriction site/function map of pEWSA30 is presented in Figure 20.

Plasmids pEWSA31 and pEWSA32 and Escherichia coli strains DH5α/pEWSA31 and DH5α/pEWSA32 were constructed according to the method described immediately above for plasmid pEWSA30 and E. coli strain DH5α/pEWSA30. The NcoI/BamHI restriction fragment from plasmid pEWSA28 (prepared in Example 4-P) was used in the construction of plasmid pEWSA31. The XbaI/BamHI restriction fragment from plasmid pEWSA29 (prepared in Example 4-P) was used in the construction of plasmid pEWSA32. Restriction/function maps for plasmids pEWSA31 and pEWSA32 are provided in Figures 21 and 22.

S. Expression of mutant forms of *mec*A-27R in *Escherichia coli*

An Escherichia coli K12 DH5α/pEWSA30 transformant was grown at 30°C overnight in 500 ml of L broth (containing 10 µg/ml of tetracycline) in a gyrotory incubator (250 rpm). The cells were diluted 100-fold by adding 10 ml of the overnight culture to 990 ml of fresh L broth containing 10 µg/ml tetracycline in a 2.8 L flask and

incubated for one hour at 30°C under the same growth conditions. The temperature of the gyrotory incubator shaker was then raised to 42°C and incubation continued for an additional 6.5 hours. The c1857 temperature-sensitive repressor of the lambda pL promoter, positioned to drive expression of the mecA-27R coding sequence on plasmid pEWSA30, is inactivated at 42°C. Therefore, at 42°C expression of mecA-27R occurs in the host cells. After induction, the cells were harvested by centrifugation and used as a preferred source of E. coli produced PBP2A-27R activity.

In a manner analogous to that described above for expression of pEWSA30 in E. coli, cultures containing plasmids pEWSA31 and pEWSA32 were grown and induced to express their forms of the mecA-27R gene.

T. Assay of PBP2A-27R activity in *Escherichia coli* cells

Cells harvested from the cultures in Example 4-R were assayed for PBP2A-27R activity according to the protocol described in Example 3-C. In this case it was not necessary to prepare membranes of the host cells, however it is necessary to break open the E. coli cells. The transmembrane portion of the PBP2A-27R protein which causes the native protein to be associated with the membrane, has been removed in the mutant forms of the mecA-27R gene. As a result the PBP2A-27R protein is expressed in a cytosolic form.

U. Purification of PBP2A-27R-CP proteins from DH5α/pEWSA31 and DH5α/pEWSA32 cells

DH5α/pEWSA31 and DH5α/pEWSA32 cells carry plasmids which encode PBP2A-27R proteins which lack the native transmembrane region and have a chelating peptides incorporated into their structure (these plasmids are described in Example 4-R). The presence of the chelating peptide is useful in obtaining purified protein rapidly. Expression of the mutant forms of the mecA-27R gene is described in Example 4-S. Cell pellets containing pEWSA31 or pEWSA32 are processed in the following manner.

The pelleted cells are washed twice in 10 mM sodium phosphate buffer at pH 7.0 by resuspension and centrifugation. The washed cells are harvested by centrifugation and resuspended in 10 mM sodium phosphate buffer containing 50 μg/ml of DNaseI and 1 mM PMSF. The cells are broken by passing through a Fench press at 16,000 $lb/in^2$ (SLM Aminco, Urbana, Illinois). After centrifugation to remove unbroken bacterial cells, supernatants are harvested and centrifuged at 135,000 x g for one hour to remove cell membranes and vesicles. The supernatant from this centrifugation is decanted into a fresh container and constitutes a crude protein extract containing PBP 2A-27R and other proteins.

The crude protein extract is filtered through a 0.45 micron filter and applied to a Ni(II) or Co(II) IMAC column equilibrated in A buffer (50 mM $NaH_2PO_4$, 0.5 M NaC1, pH 7.5). The Ni(II) or Co(II) IMAC column was prepared as described (Smith, *et al.*, 1987). In this instance, when the Co(II) column is prepared, all of the buffers and water are degassed and purged with helium to exclude air, so as to prevent the premature oxidation of Co(II) to Co(III). Briefly, a 1.0 x 10.0 cm HR column is poured with Pharmacia Fast-Flow Chelating Gel, connected to an FPLC, and washed with four column volumes of distilled water before applying 4 ml of a 50 mM $NiCl_2$ or $CoCl_2$ solution to the column. The metal loaded column is washed again with four column volumes of distilled water before equilibrating with A buffer. A one ml sample of the crude protein extract solution is injected onto the column and washed with A buffer at 0.2 ml/min until the baseline returned to zero, generally about 1.5 column volumes. The bound material is then eluted by either lowering the pH or by introducing a displacing ligand, such as imidazole. The B buffer used to generate the descending pH gradient is 50 mM acetic acid, 0.5M NaCl, pH 3.7. A gradient from 0 to 100% B over 150 minutes, or about three column volumes, generated a linear pH gradient from pH 7.5 to 3.7 and was used to elute PBP2A-27R-CP. The B buffer used to generate an imidazole gradient consists of 0.5 M imidazole, 50 mM $NaH_2PO_4$, 0.5 M NaCl, pH 7.5. A gradient of 0 to 100% B over 300 ml, or about 4.5 column volumes, is used to elute PBP2A-27R-CP. The protein content of the peaks in the chromatogram is determined by SDS-PAGE using the Novex Tricine-PAGE gel system and by Western Blots. A blank IMAC run using the same imidazole gradient, is used to determine whether Ni(II) leached off the column at high imidazole concentrations. The Ni(II) concentration of each fraction is measured by titrating with excess EDTA, as described previously (Smith, et al. 1987).

Pools are made of the fractions containing PBP2A-27R-CP and then desalted over a Sephadex G25 column equilibrated in 50 mM ammonium bicarbonate pH 8.0 buffer. The desalted pools are then lyophilized to dryness. PBP2A-27R-CP protein purified in this manner may be used in structural studies of the protein and in assays to detect inhibitors of PBP2A-27R which may prove useful as therapeutic agents.

V. Production of Secretory forms of $PBP2A\text{-}27R^s$

Plasmids encoding secretory forms of the $PBP2A\text{-}27R^s$ variants are formed by the insertion of a DNA sequence encoding a signal peptide at the 5′ end of the PBP2a-27R$^s$ coding sequence. The following dsDNA sequence:

```
5'   CATGTTCAAAACGACGCTCTGCGCCTTATTAATTACCGCCTCTTGCTCCACATTTGCTGC  3'
         ||||||||||||||||||||||||||||||||||||||||||||||||||||||||||||
3'       AAGTTTTGCTGCGAGACGCGGAATAATTAATGGCGGAGAACGAGGTGTAAACGACGGTAC  5'
```

encodes the amino acid sequence:

MetPheLysThrThrLeuCysAlaLeuLeuIleThrAlaSerCysSerThrPhe

AlaAlaMetVal -- PBP2A)

corresponding to the ampC signal peptide. When inserted into plasmids pEWSA30, pEWSA31 and pEWSA32 will result in PBP2A-27R$^s$ proteins (and PBP2A-27R$^s$ derivatives with chelating peptides) with the ampC signal peptide at the amino terminus of the resultant protein.

The plasmid pEWSA30 is digested with the NcoI restriction endonuclease taking advantage of the unique NcoI site at the start codon of the PBP2A-27R$^s$ coding sequence. The linearized vector DNA is isolated by agarose gel electrophoresis and dephosphorylated with calf intestinal phosphatase. The above ssDNA sequences are synthesized on an Applied Biosystems Model 380A or 380B DNA synthesizer (Applied Biosystems, Foster City, CA). The single-stranded molecules are purified by polyacrylamide gel electrophoresis and isolated from the gel. The ends of the ssDNA molecules are phosphorylated using T4-polynucleotide kinase. The phosphorylated ssDNA molecules are annealed to form the above diagrammed dsDNA molecule with 5′ protruding ends that are compatible for ligation with the overhang left after cleavage with NcoI. The phosphorylated dsDNA encoding the signal peptide is mixed with the dephosphorylated vector DNA in the presence of T4 DNA ligase. The resultant mixture contains plasmid pEWSA39 containing the proper orientation of the synthetic dsDNA insert and other ligation products. The mixture is used to transform competent E. coli K12 DH5α cells. The cells are grown and colonies picked and resuspended in 2x TY broth.

Identification of those strains containing pEWSA39 is done by restriction analysis of the mini prepped DNA obtained from transformants. The DNA is digested with the NcoI and XbaI restriction endonucleases. Cells carrying the pEWSA39 vector should produce an ~675 bp fragment. Cells carrying the dsDNA fragment encoding the ampC signal peptide in reverse orientation will lack the band corresponding to the ~675 bp fragment and will produce a band corresponding to ~740 bp fragment.

An Escherichia coli culture containing the mutant form of the mecA-27R gene (*i.e* DH5α/pEWSA39) was grown overnight in 200 ml of TY broth. The cells were harvested and plasmid DNA was extracted using Qiagen Plasmid Midi kit. Plasmid DNA isolated in this manner was resuspended in 200 microliters of sterile water and constituted plasmid pEWSA39. Plasmid pEWSA39, formed with the double stranded insert as described above, lacks the transmembrane region, contains an Nco1 site, and the ampC signal peptide coding sequence.

Plasmids pEWSA40 and pEWSA41 and E. coli strains DH5α/pEWSA40, and DH5α/pEWSA41 were constructed according to the method described immediately above for plasmid pEWSA39, and E. coli strain DH5α-/pEWSA39 except that plasmids pEWSA31 and pEWSA32 were used in place of plasmid pEWSA30 to construct pEWSA40 and pEWSA41, respectively. A restriction site and function maps of plasmids pEWSA40 and pEWSA41 are provided in figures 29 and 30, respectively. Plasmid pEWSA40 contains the Nco1 site, a chelating peptide, and a signal peptide of ampC. Plasmid pEWSA41 comprises the Nco1 site, the ampC signal peptide, the chelating peptide and a proline residue between the chelating peptide and structural protein.

In a manner analogous to that described above for expression of pEWSA39 and E. coli, cultures containing plasmids pEWSA40 (K12 DH5α/pEWSA40) and pEWSA41 (K12 DH5α/pEWSA41) were grown and induced to express their forms of the mecA gene.

## EXAMPLE 5

### Screening for compounds which inhibit methicillin resistant *Staphylococcus* species using a crude cell extract.

The mecA-27R gene enhances our ability to screen for compounds effective against methicillin resistant staphylococcal species. The fact that the wild-type PBP2A-27R is membrane associated complicates the use of this protein in large-scale screening operations. This factor makes it necessary to isolate the membrane fraction from cells containing the native mecA-27R gene in order to accurately assess the ability to bind a particular

compound. We have identified the membrane spanning region of PBP2A-27R and subsequently removed it by modifying the mecA-27R gene through molecular biological techniques. The modified gene was inserted into an expression vector designated pEWSA30 (See example 4-R). The modified PBP2A-27R expressed off plasmid pEWSA30 is ammenable to use in large-scale screening processes. One such process is detailed herein.

A. Expression of the modified *mec*A-27R gene on plasmid pEWSA30 which encodes a PBP2A-27R protein lacking the transmembrane region

An Escherichia coli K12 DH5α/pEWSA30 transformant was grown at 30°C overnight in 500 ml of L broth (containing 10 μg/ml of tetracycline) in a gyrotory incubator (250 rpm). The cells were diluted 100-fold by adding 10 ml of the overnight culture to 990 ml of fresh L broth containing 10 μg/ml tetracycline in a 2.8 L flask and incubated for one hour at 30°C under the same growth conditions. The temperature of the gyrotory incubator shaker was then raised to 42°C and incubation continued for an additional 6.5 hours. The cl857 temperature-sensitive repressor of the lambda pL promoter, positioned to drive expression of the mecA-27R coding sequence on plasmid pEWSA30, is inactivated at 42°C. Therefore, at 42°C expression of mecA-27R occurs in the host cells. After induction, the cells were harvested by centrifugation and used as a preferred source of E. coli produced PBP2A-27R lacking the transmembrane region.

B. Preparation of crude protein extracts for analysis of the of PBP2A-27R penicillin binding activity

The pelleted cells obtained in Example 5-A are washed twice in 10 mM sodium phosphate buffer at pH 7.0 by resuspension and centrifugation. The washed cells are harvested by centrifugation and resuspended in 10 mM sodium phosphate buffer containing 50 μg/ml of DNaseI, 1 mM PMSF, and 0.14 mM 2-mercaptoethanol. The cells are broken by passing through a Fench press at 16,000 lb/in$^2$ (SLM Aminco, Urbana, Illinois). After centrifugation to remove unbroken bacterial cells, supernatants are harvested and centrifuged at 135,000 x g for one hour to remove cell membranes and vesicles. The supernatant from this centrifugation is decanted into a fresh container and constitutes a crude protein extract containing PBP2A-27R$^s$.

C. Assay of PBP2A-27R penicillin binding activity

The crude cell extract preparation made in Example 5-B is analyzed for the ability of PBP 2A-27R to bind penicillin by labeling with $^{125}$I-Penicillin V (IPV) in substantial accordance with the procedure described by Preston et al., (Antimicrobial Agents and Chemotherapy, 1990, pages 718-721). To adapt the procedure to a large-scale screen, a 96 well microtiter dish is used to contain the reactions. A 4 μl aliquot of the crude cell extract is incubated with 6 μl of IPV (96 μg/ml) for 15 minutes at 35°C. The proteins in the reaction are precipitated. The precipitated protein is collected on a glass filter and unbound IPV is washed off. The amount of IPV bound to PBP2A-27R can be estimated by scintillation counting. Alternatively, autoradiography can be used to estimate the degree of binding of IPV. Based on the above process, a competitive assay is used to assess the ability of test compounds to bind to the active site of PBP2A-27R. In this case the test compound is exposed to the crude cell-extract for 15 minutes prior to the addition of IPV. A positive result is indicated by a reduction in the amount of radioactivity present on the glass filter.

EXAMPLE 6

Screening for compounds which inhibit methicillin resistant *Staphylococcus* species using support-immobilized PBP2A-27R$^s$-CP.

As exemplified herein, the procedure of an immobilized PBP2A binding assay proceeds as follows. The PBP2A-27R$^s$-CP protein was produced by the addition of a DNA sequence encoding the Met-Gly-His-Trp-His-His-His chelating peptide at the 5′ end of the DNA sequence encoding the PBP2A-27R$^s$ protein as described above in Example 4.R. The pEWSA31 expression plasmid was transformed into competent E. coli K12 DH5α cells by techniques well known in the art. Expression of the PBP2A-27R-CP penicillin binding protein was confirmed by PBP binding assays.

A CP-IMAC column is prepared in substantial accordance with the teaching of Example 4.U. herein according to the teaching of Smith, et al., United States Patent No. 4,569,794. The immobilized Co ion must be in the labile Co(II) oxidation state. In order to prevent premature oxidation of the Co(II) all buffers and solutions were degassed with helium before use. As described above, an iminodiacetic acid (IDA) molecule possessing a bound Co(II) ion is covalently attached to fast flow agarose gel. As described in Example 4.U above, the immobilized Co(II) ion interacts with the chelating peptide portion of the PBP2A-27R$^s$-CP resulting in the formation of the [PBP2A-27R$^s$-CP/Co(II)-Resin] complex, i.e., the "unlocked" form. Non-specifically bound molecules are then liberated by the introduction of imidazole.

A kinetically inert complex is formed by changing the oxidation state of the bound metal ion causing the cobalt ion to change from the labile Co(II) oxidation state to the kinetically inert Co(III) oxidation state. This is achieved by introducing oxygen to the column by the following batch process. The contents of the column containing the immobilized IDA-Co(II)-PBP2A-27R-CP complex was transferred to a test tube. The space above the settled gel was replaced with the oxygen or an oxygen containing gas and then capped. The tube was continuously inverted for 24 hours to mix the oxygen containing gas with the bead containing the IDA-Co(II)-PBP2A-27R$^s$-CP complex to oxidize the Co(II) to Co(III). The PBP2A-27R$^s$-CP thereby assumes the "locked" conformation. Equal aliquots of the beads possessing the IDA-Co(III)-PBP2A-27R-CP complex are then be transferred to a reaction vessel for example an Eppendorf tube, or the well of a microtiter plate.

A sample of antibiotic test compound is introduced to the reaction vessel containing the immobilized PBP2A-27R$^s$-CP. Unbound test compound is then washed free. $^{125}$I-penV is then added to the reaction vessel. Unbound $^{125}$I-penV is then washed free. The level of binding of test compound is determined by the comparative level of binding of $^{125}$I-penV in the presence of the test compound versus the absence of the test commpound. The ratio of $^{125}$I-penV bound in the absence of test compound relative to the amount of $^{125}$I-penV bound in the presence of test compound is directly proportional to formation of the [PBP2A-27R$^s$-CP-test compound] complex. Test compounds which produce high ratios of $^{125}$I-penV bound in the absence of test compound relative to the amount of $^{125}$I-penV bound in the presence of test compound indicate a compound potentially useful as an antibiotic against MRS infections.

EXAMPLE 7 Screening for compounds which inhibit methicillin resistant *Staphylococcus* species using purified PBP2a-27R-CP

The mecA-27R gene enhances our ability to screen for compounds effective against methicillin resistant staphylococcal species. The fact that the wild-type PBP2A-27R is membrane associated complicates the use of this protein in large-scale screening operations. This factor makes it necessary to isolate the membrane fraction from cells containing the native mecA-27R gene in order to accurately assess the ability to bind a particular compound. We have identified the membrane spanning region of PBP2A-27R and subsequently removed it by modifying the mecA-27R gene through molecular biological techniques. In addition, a chelating peptide was added to the protein at the N-terminus to facilitate protein purification. The modified gene was inserted into an expression vector designated pEWSA31. Another form of the modified PBP2A-27R-CP was constructed which contained a proline residue between the chelating peptide and the remainder of the protein (pEWSA32). These modified PBP2A-27R-CPs expressed off plasmid pEWSA31 or pEWSA32 are ammenable to use in large-scale screening processes. One such process is detailed herein.

A. Expression of the modified *mec*A-27R-CP gene on plasmid pEWSA31 which encodes a PBP2A-27R-CP protein lacking the transmembrane region

An Echerichia coli K12 DH5α/pEWSA31 transformant was grown at 30°C overnight in 500 ml of L broth (containing 10 μg/ml of tetracycline) in a gyrotory incubator (250 rpm). The cells were diluted 100-fold by adding 10 ml of the overnight culture to 990 ml of fresh L broth containing 10 μg/ml tetracycline in a 2.8 L flask and incubated for one hour at 30°C under the same growth conditions. The temperature of the gyrotory incubator shaker was then raised to 42°C and incubation continued for an additional 6.5 hours. The cI857 temperature-sensitive repressor of the lambda pL promoter, positioned to drive expression of the mecA-27R-CP coding sequence on plasmid pEWSA31, is inactivated at 42°C. Therefore, at 42°C expression of mecA-27R-CP occurs in the host cells. After induction, the cells were harvested by centrifugation and used as a preferred source of E. coli produced PBP2A-27R-CP lacking the transmembrane region and containing a chelating peptide. PBP2A-27R-CP protein expressed off plasmid pEWSA32 is expressed in the same manner.

B. Purification of PBP2A-27R-CP protein from DH5a/pEWSA31

DH5α/pEWSA31 cells carry plasmids which encodes a PBP2A-27R-CP protein which lacks the native transmembrane region and has a chelating peptide incorporated into it's structure (this plasmid is described in Example 4-R). The presence of the chelating peptide is useful in obtaining purified protein rapidly. Expression of the mutant forms of the mecA-27R gene is described in Example 4-S. Cell pellets containing pEWSA31 are processed in the following manner.

The pelleted cells are washed twice in 10 mM sodium phosphate buffer at pH 7.0 by resuspension and centrifugation. The washed cells are harvested by centrifugation and resuspended in 10 mM sodium phosphate buffer containing 50 μg/ml of DNaseI and 1 mM PMSF. The cells are broken by passing through a Fench press at 16,000 lb/in$^2$ (SLM Aminco, Urbana, Illinios). After centrifugation to remove unbroken bacterial cells, supernatants are harvested and centrifuged at 135,000 x g for one hour to remove cell membranes and vesicles. The supernatant from this centrifugation is decanted into a fresh container and constitutes a crude protein extract containing PBP 2A-27R-CP and other proteins.

The crude protein extract is filtered through a 0.45 micron filter and applied to a Ni(II) or Co(II) IMAC column equilibrated in A buffer (50 mM $NaH_2PO_4$, 0.5 M NaC1, pH 7.5). The Ni(II) or Co(II) IMAC column was prepared as described (Smith, et al., 1987). In this instance, when the Co(II) column is prepared, all of the buffers and water are degassed and purged with helium to exclude air, so as to prevent the premature oxidation of Co(II) to Co(III). Briefly, a 1.0 x 10.0 cm HR column is poured with Pharmacia Fast-Flow Chelating Gel, connected to an FPLC, and washed with four column volumes of distilled water before applying 4 ml of a 50 mM $NiCl_2$ or $CoCl_2$ solution to the column. The metal loaded column is washed again with four column volumes of distilled water before equilibrating with A buffer. A one ml sample of the crude protein extract solution is injected onto the column and washed with A buffer at 0.2 ml/min until the baseline returned to zero, generally about 1.5 column volumes. The bound material is then eluted by either lowering the pH or by introducing a displacing ligand, such as imidazole. The B buffer used to generate the descending pH gradient is 50 mM acetic acid, 0.5M NaCl, pH 3.7. A gradient from 0 to 100% B over 150 minutes, or about three column volumes, generated a linear pH gradient from pH 7.5 to 3.7 and was used to elute PBP2A-27R-CP. The B buffer used to generate an imidazole gradient consists of 0.5 M imidazole, 50 mM $NaH_2PO_4$, 0.5 M NaCl, pH 7.5. A gradient of 0 to 100% B over 300 ml, or about 4.5 column volumes, is used to elute PBP2A-27R-CP. The protein content of the peaks in the chromatogram is determined by SDS-PAGE using the Novex Tricine-PAGE gel system and by Western Blots. A blank IMAC run using the same imidazole gradient, is used to determine whether Ni(II) leached off the column at high imidazole concentrations. The Ni(II) concentration of each fraction is measured by titrating with excess EDTA, as described previously (Smith, et al. 1987).

Pools are made of the fractions containing PBP2A-27R-CP and then desalted over a Sephadex G25 column equilibrated in 50 mM ammonium bicarbonate pH 8.0 buffer. The desalted pools are then lyophilized to dryness. PBP2A-27R-CP protein purified in this manner may be used in structural studies of the protein and in assays to detect inhibitors of PBP2A-27R which may prove useful as therapeutic agents.

Plasmid pEWSA32 which encodes a another useful PBP2a-27R-CP contains a proline residue at the carboxyl end of the chelating peptide. Protein purification proceeds as described above, upon completion the chelating peptide is removed through the action of diaminopeptidase. This protein is also useful in the assay described below.

C. Assay of PBP2A-27R-CP penicillin binding activity

Purified PBP2A-27R-CP is analyzed for it's ability to bind penicllin by labeling with $^{125}$I-Penicillin V (IPV) in substantial accordance with the procedure described by Preston et al., (Antimicrobial Agents and Chemotherapy, 1990, pages 718-721). To adapt the procedure to a large-scale screen, a 96 well microtiter dish is used to contain the reactions. A 4 μl aliquot of the purified protein (produced from plasmid pEWSA31 or pEWSA32) is incubated with two μl of test compound for 15 minutes. Then 6 μl of IPV (96 μg/ml) is added and incubated for an additional 15 minutes at 35°C. The proteins in the reaction are precipitated. The precipitated protein is collected on a glass filter and unbound IPV is washed off. The amount of IPV bound to PBP2A-27R-CP can be estimated by scintillation counting. Alternatively, autoradiography can be used to estimate the degree of binding of IPV. A positive result is indicated by a reduction in the amount of radioactivity present on the glass filter.

## Claims

1. A polynucleotide compound encoding the PBP2A-27R protein or a functional derivative thereof, and polynucleotide compounds which are functional equivalents thereof.

2. The compound of Claim 1 wherein said polynucleotide compound comprises the DNA sequence:

1 ATGAAAAAGATAAAAATTGTTCCACTTATTTTAATAGTTGTAGTTGTCGGGTTTGGTATA 60

61 TATTTTTATGCTTCAAAAGATAAAGAAATTAATAATACTATTGATGCAATTGAAGATAAA 120

121 AATTTCAAACAAGTTTATAAAGATAGCAGTTATATTTCTAAAAGCGATAATGGTGAAGTA 180

181 GAAATGACTGAACGTCCGATAAAAATATATAATAGTTTAGGCGTTAAAGATATAAACATT 240

241 CAGGATCGTAAAATAAAAAAAGTATCTAAAAATAAAAAACGAGTAGATGCTCAATATAAA 300

301 ATTAAAACAAACTACGGTAACATTGATCGCAACGTTCAATTTAATTTTGTTAAAGAAGAT 360

361 GGTATGTGGAAGTTAGATTGGGATCATAGCGTCATTATTCCAGGAATGCAGAAAGACCAA 420

421 AGCATACATATTGAAAATTTAAAATCAGAACGTGGTAAAATTTTAGACCGAAACAATGTG 480

481 GAATTGGCCAATACAGGAACAGCATATGAGATAGGCATCGTTCCAAAGAATGTATCTAAA 540

541 AAAGATTATAAAGCAATCGCTAAAGAACTAAGTATTTCTGAAGACTATATCAAACAACAA 600

601 ATGGATCAAAATTGGGTACAAGATGATACCTTCGTTCCACTTAAAACCGTTAAAAAAATG 660

661 GATGAATATTTAAGTGATTTCGCAAAAAAATTTCATCTTACAACTAATGAAACAGAAAGT 720

721 CGTAACTATCCTCTAGAAAAAGCGACTTCACATCTATTAGGTTATGTTGGTCCCATTAAC 780

781 TCTGAAGAATTAAAACAAAAAGAATATAAAGGCTATAAAGATGATGCAGTTATTGGTAAA 840

841 AAGGGACTCGAAAAACTTTACGATAAAAAGCTCCAACATGAAGATGGCTATCGTGTCACA 900

901 ATCGTTGACGATAATAGCAATACAATCGCACATACATTAATAGAGAAAAAGAAAAAAGAT 960

961 GGCAAAGATATTCAACTAACTATTGATGCTAAAGTTCAAAAGAGTATTTATAACAACATG 1020

1021 AAAAATGATTATGGCTCAGGTACTGCTATCCACCCTCAAACAGGTGAaTTATTAGCACTT 1080

1081 GTAAGCACACCTTCATATGACGTCTATCCATTTATGTATGGCATGAGTAACGAAGAATAT 1140

1141 AATAAATTAACCGAAGATAAAAAAGAACCTCTGCTCAACAAGTTCCAGATTACAACTTCA 1200

1201 CCAGGTTCAACTCAAAAAATATTAACAGCAATGATTGGGTTAAATAACAAAACATTAGAC 1260

1261 GATAAAACAAGTTATAAAATCGATGGTAAAGGTTGGCAAAAAGATAAATCTTGGGGTGGT 1320

1321 TACAACGTTACAAGATATGAAGTGGTAAATGGTAATATCGACTTAAAACAAGCAATAGAA 1380

1381 TCATCAGATAACATTTTCTTTGCTAGAGTAGCACTCGAATTAGGCAGTAAGAAATTTGAA 1440

1441 AAAGGCATGAAAAAACTAGGTGTTGGTGAAGATATACCAAGTGATTATCCATTTTATAAT 1500

1501 GCTCAAATTTCAAACAAAAATTTAGATAATGAAATATTATTAGCTGATTCAGGTTACGGA 1560

1561 CAAGGTGAAATACTGATTAACCCAGTACAGATCCTTTCAATCTATAGCGCATTAGAAAAT 1620

1621 AATGGCAATATTAACGCACCTCACTTATTAAAAGACACGAAAAACAAAGTTTGGAAGAAA 1680

1681 AATATTATTTCCAAAGAAAATATCAATCTATTAACTGATGGTATGCAACAAGTCGTAAAT 1740

1741 AAAACACATAAAGAAGATATTTATAGATCTTATGCAAACTTAATTGGCAAATCCGGTACT 1800

1801 GCAGAACTCAAAATGAAACAAGGAGAAACTGGCAGACAAATTGGGTGGTTTATATCATAT 1860

1861 GATAAAGATAATCCAAACATGATGATGGCTATTAATGTTAAAGATGTACAAGATAAAGGA 1920

1921 ATGGCTAGCTACAATGCCAAAATCTCAGGTAAAGTGTATGATGAGCTATATGAGAACGGT 1980

1981 AATAAAAAATACGATATAGATGAATAA 2007,

or a functional equivalent thereof.

**3.** The compound of Claim 2 operably linked with a promoter-operator sequence functional in an E. coli, host cell.

**4.** A vector comprising the compound of Claim 4.

**5.** A polypeptide compound comprising the PBP2A-27R protein or a functional derivative thereof.

**6.** The compound of Claim 5 comprising the amino acid sequence:

1 MKKIKIVPLILIVVVVGFGIYFYASKDKEINNTIDAIEDKNFKQVYKDSS 50

51 YISKSDNGEVEMTERPIKIYNSLGVKDINIQDRKIKKVSKNKKRVDAQYK 100

101 IKTNYGNIDRNVQFNFVKEDGMWKLDWDHSVIIPGMQKDQSIHIENLKSE 150

151 RGKILDRNNVELANTGTAYEIGIVPKNVSKKDYKAIAKELSISEDYIKQQ 200

201 MDQNWVQDDTFVPLKTVKKMDEYLSDFAKKFHLTTNETESRNYPLEKATS 250

251 HLLGYVGPINSEELKQKEYKGYKDDAVIGKKGLEKLYDKKLQHEDGYRVT 300

301 IVDDNSNTIAHTLIEKKKKDGKDIQLTIDAKVQKSIYNNMKNDYGSGTAI 350

351 HPQTGELLALVSTPSYDVYPFMYGMSNEEYNKLTEDKKEPLLNKFQITTS 400

401 PGSTQKILTAMIGLNNKTLDDKTSYKIDGKGWQKDKSWGGYNVTRYEVVN 450

```
451  GNIDLKQAIESSDNIFFARVALELGSKKFEKGMKKLGVGEDIPSDYPFYN 500

501  AQISNKNLDNEILLADSGYGQGEILINPVQILSIYSALENNGNINAPHLL 550

551  KDTKNKVWKKNIISKENINLLTDGMQQVVNKTHKEDIYRSYANLIGKSGT 600

601  AELKMKQGETGRQIGWFISYDKDNPNMMMAINVKDVQDKGMASYNAKISG 650

651  KVYDELYENGNKKYDIDE  668
```

7. A polypeptide compound of the formula:

$$SP\text{-}L\text{-}PBP2A^{s}$$

wherein:

SP = 0 or signal peptide,
L = Met-Val, or a compound of the formula:
Met-Gly-CP-(Pro)n-PBP2A$^{s}$
wherein:
CP = 0 or a chelating peptide of the formula:

$$(His)_{x}\text{-}(A)_{y}\text{-}(His)_{z}$$

wherein:
A is an amino acid,
X=1-10,
y=0-4,
Z=1-10,
and monomers, dimers and trimers thereof wherein each monomer unit is the same or different,
Pro = the amino acid proline,
n = 0 or 1, and,
PBP2A$^{s}$ is a soluble form of the PBP2A protein.

8. The compound of Claim 7 wherein x=1-3, y=0-4, z=1-3.

9. The compound of Claim 8 wherein said chelating peptide is selected from chelating peptides of the formula His-Trp-His-Met-Tyr, His-His-His-Met-Tyr, His-Trp-His-Trp-His, His-Trp-His-His-His, His-His-His-His-Tyr-Met-His-His-His-His-Tyr, His-His-His-His-His, and His-Trp-His-His-His.

10. The compound of Claim 9 wherein SP is a signal peptide selected from the group comprising the signal peptides of the <u>amp</u>C gene product, the <u>omp</u>A gene product and the β-lactamase gene product.

11. The compound of Claim 10 wherein said PBP2A$^{s}$ is selected from the group consisting of PBP2A$^{s}$ having the amino acid sequences:

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTAYEIG IVPKNVSKKD YKAIAKELSI SEDYIKQQMD

181 230
QNWVQDDTFV PLKTVKKMDE YLSDFAKKFH LTTNETESRN YPLEKATSHL

231 280
LGYVGPINSE ELKQKEYKGY KDDAVIGKKG LEKLYDKKLQ HEDGYRVTIV

281 330
DDNSNTIAHT LIEKKKKDGK DIQLTIDAKV QKSIYNNMKN DYGSGTAIHP

331 380
QTGELLALVS TPSYDVYPFM YGMSNEEYNK LTEDKKEPLL NKFQITTSPG

381 430
STQKILTAMI GLNNKTLDDK TSYKIDGKGW QKDKSWGGYN VTRYEVVNGN

431 480
IDLKQAIESS DNIFFARVAL ELGSKKFEKG MKKLGVGEDI PSDYPFYNAQ

481 530
ISNKNLDNEI LLADSGYGQG EILINPVQIL SIYSALENNG NINAPHLLKD

531 580
TKNKVWKKNI ISKENINLLT DGMQQVVNKT HKEDIYRSYA NLIGKSGTAE

581 630
LKMKQGETGR QIGWFISYDK DNPNMMMAIN VKDVQDKGMA SYNAKISGKV

631 646
YDELYENGNK KYDIDE,

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTAYEIG IVPKNVSKKD YKAIAKELSI SEDYIKQQMD

181 230
QNWVQDDTFV PLKTVKKMDE YLSDFAKKFH LTTNETESRN YPLGKATSHL

231 280
LGYVGPINSE ELKQKEYKGY KDDAVIGKKG LEKLYDKKLQ HEDGYRVTIV

281 330
DDNSNTIAHT LIEKKKKDGK DIQLTIDAKV QKSIYNNMKN DYGSGTAIHP

331 380
QTGELLALVS TPSYDVYPFM YGMSNEEYNK LTEDKKEPLL NKFQITTSPG

381 430
STQKILTAMI GLNNKTLDDK TSYKIDGKGW QKDKSWGGYN VTRYEVVNGN

431 280
IDLKQAIESS DNIFFARVAL ELGSKKFEKG MKKLGVGEDI PSDYPFYNAQ

481 530
ISNKNLDNEI LLADSGYGQG EILINPVQIL SIYSALENNG NINAPHLLKD

531 580
TKNKVWKKNI ISKENINLLT DGMQQVVNKT HKEDIYRSYA NLIGKSGTAE

581 630
LKMKQGETGR QIGWFISYDK DNPNMMMAIN VKDVQDKGMA SYNAKISGKV

631 646
YDELYENGNK KYDIDE,

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTHMRLG IVPKNVSKKD YKAIAKELSI SEDYINNKWI

181 230
KIGYKMIPSF HFKTVKKMDE YLSDFAKKFH LTTNETESRN YPLGKATSHL

231 280
LGYVGPINSE ELKQKEYKGY KDDAVIGKKG LEKLYDKKLQ HEDGYRVTIV

281 330
RVDDNSNTIA HTLIEKKKKD GKDIQLTIDA KVQKSIYNNM KNDYGSGTAI

331 380
HPQTGELLAL VSTPSYDVYP FMYGMSNEEY NKLTEDKKEP LLNKFQITTS

381 430
PGSTQKILTA MIGLNNKTLD DKTSYKIDGK GWQKDKSWGG YNVTRYEVVN

431 280
GNIDLKQAIE SSDNIFFARV ALELGSKKFE KGMKKLGVGE DIPSDYPFYN

481 530
AQISNKNLDN EILLADSGYG QGEILINPVQ ILSIYSALEN NGNINAPHLL

531 580
KDTKNKVWKK NIISKENINL LNDGMQQVVN KTHKEDIYRS YANLIGKSGT

581 630
AELKMKQGET GRQIGWFISY DKDNPNMMMA INVKDVQDKG MASYNAKISG

631 648
KVYDELYENG NKKYDIDE, and

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTHMRLG IVPKNVSKKD YKAIAKELSI SEDYINNKWI

181 230
KIGYKMIPSF HFKTVKKMDE YLSDFAKKFH LTTNETESRN YPLEKATSHL

231 280
LGYVGPINSE ELKQKEYKGY KDDAVIGKKG LEKLYDKKLQ HEDGYRVTIV

281 330
DDNSNTIAHT LIEKKKKDGK DIQLTIDAKV QKSIYNNMKN DYGSGTAIHP

331 380
QTGELLALVS TPSYDVYPFM YGMSNEEYNK LTEDKKEPLL NKFQITTSPG

381 430
STQKILTAMI GLNNKTLDDK TSYKIDGKGW QKDKSWGGYN VTRYEVVNGN

431 480
IDLKQAIESS DNIFFARVAL ELGSKKFEKG MKKLGVGEDI PSDYPFYNAQ

481 530

ISNKNLDNEI LLADSGYGQG EILINPVQIL SIYSALENNG NINAPHLLKD

531 580
TKNKVWKKNI ISKENINLLN DGMQQVVNKT HKEDIYRSYA NLIGKSGTAE

581 630
LKMKQGESGR QIGWFISYDK DNPNMMMAIN VKDVQDKGMA SYNAKISGKV

631 646
YDELYENGNK KYDIDE

12. The compound of Claim 11 wherein said chelating peptide is a chelating peptide of the formula His-Trp-His-His-His.

13. The compound of Claim 12 wherein SP is the signal peptide of the ampC gene product.

14. The compound of Claim 13 wherein n=0.

15. A polynucleotide compound encoding the compound of Claim 14.

16. The compound of Claim 7 wherein SP = 0, L = Met-Val, and said is selected from the group consisting of PBP2A[s] comprising the amino acid sequences:

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTAYEIG IVPKNVSKKD YKAIAKELSI SEDYIKQQMD

181 230
QNWVQDDTFV PLKTVKKMDE YLSDFAKKFH LTTNETESRN YPLEKATSHL

231 280
LGYVGPINSE ELKQKEYKGY KDDAVIGKKG LEKLYDKKLQ HEDGYRVTIV

281 330
DDNSNTIAHT LIEKKKKDGK DIQLTIDAKV QKSIYNNMKN DYGSGTAIHP

331 380
QTGELLALVS TPSYDVYPFM YGMSNEEYNK LTEDKKEPLL NKFQITTSPG

381 430
STQKILTAMI GLNNKTLDDK TSYKIDGKGW QKDKSWGGYN VTRYEVVNGN

431 480
IDLKQAIESS DNIFFARVAL ELGSKKFEKG MKKLGVGEDI PSDYPFYNAQ

481 530
ISNKNLDNEI LLADSGYGQG EILINPVQIL SIYSALENNG NINAPHLLKD

531 580
TKNKVWKKNI ISKENINLLT DGMQQVVNKT HKEDIYRSYA NLIGKSGTAE

581 630
LKMKQGETGR QIGWFISYDK DNPNMMMAIN VKDVQDKGMA SYNAKISGKV

631 646
YDELYENGNK KYDIDE,

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTAYEIG IVPKNVSKKD YKAIAKELSI SEDYIKQQMD

181 230
QNWVQDDTFV PLKTVKKMDE YLSDFAKKFH LTTNETESRN YPLGKATSHL

231 280
LGYVGPINSE ELKQKEYKGY KDDAVIGKKG LEKLYDKKLQ HEDGYRVTIV

281 330
DDNSNTIAHT LIEKKKKDGK DIQLTIDAKV QKSIYNNMKN DYGSGTAIHP

331 380
QTGELLALVS TPSYDVYPFM YGMSNEEYNK LTEDKKEPLL NKFQITTSPG

381 430
STQKILTAMI GLNNKTLDDK TSYKIDGKGW QKDKSWGGYN VTRYEVVNGN

431 280
IDLKQAIESS DNIFFARVAL ELGSKKFEKG MKKLGVGEDI PSDYPFYNAQ

481 530
ISNKNLDNEI LLADSGYGQG EILINPVQIL SIYSALENNG NINAPHLLKD

531 580
TKNKVWKKNI ISKENINLLT DGMQQVVNKT HKEDIYRSYA NLIGKSGTAE

581 630
LKMKQGETGR QIGWFISYDK DNPNMMMAIN VKDVQDKGMA SYNAKISGKV

631 646
YDELYENGNK KYDIDE,

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTHMRLG IVPKNVSKKD YKAIAKELSI SEDYINNKWI

181 230
KIGYKMIPSF HFKTVKKMDE YLSDFAKKFH LTTNETESRN YPLGKATSHL

231 280
LGYVGPINSE ELKQKEYKGY KDDAVIGKKG LEKLYDKKLQ HEDGYRVTIV

281 330
RVDDNSNTIA HTLIEKKKKD GKDIQLTIDA KVQKSIYNNM KNDYGSGTAI

331 380
HPQTGELLAL VSTPSYDVYP FMYGMSNEEY NKLTEDKKEP LLNKFQITTS

381 430
PGSTQKILTA MIGLNNKTLD DKTSYKIDGK GWQKDKSWGG YNVTRYEVVN

431 280
GNIDLKQAIE SSDNIFFARV ALELGSKKFE KGMKKLGVGE DIPSDYPFYN

481 530
AQISNKNLDN EILLADSGYG QGEILINPVQ ILSIYSALEN NGNINAPHLL

531 580
KDTKNKVWKK NIISKENINL LNDGMQQVVN KTHKEDIYRS YANLIGKSGT

581 630
AELKMKQGET GRQIGWFISY DKDNPNMMMA INVKDVQDKG MASYNAKISG

631 648
KVYDELYENG NKKYDIDE, and

1 30
YASKDKEINN TIDAIEDKNF KQVYKDSSYI

31 80
SKSDNGEVEM TERPIKIYNS LGVKDINIQD RKIKKVSKNK KRVDAQYKIK

81 130
TNYGNIDRNV QFNFVKEDGM WKLDWDHSVI IPGMQKDQSI HIENLKSERG

131 180
KILDRNNVEL ANTGTHMRLG IVPKNVSKKD YKAIAKELSI SEDYINNKWI

181 230

KIGYKMIPSF HFKTVKKMDE YLSDFAKKFH LTTNETESRN YPLEKATSHL

231 280

LGYVGPINSE ELKQKEYKGY KDDAVIGKKG LEKLYDKKLQ HEDGYRVTIV

281 330

DDNSNTIAHT LIEKKKKDGK DIQLTIDAKV QKSIYNNMKN DYGSGTAIHP

331 380

QTGELLALVS TPSYDVYPFM YGMSNEEYNK LTEDKKEPLL NKFQITTSPG

381 430

STQKILTAMI GLNNKTLDDK TSYKIDGKGW QKDKSWGGYN VTRYEVVNGN

431 480

IDLKQAIESS DNIFFARVAL ELGSKKFEKG MKKLGVGEDI PSDYPFYNAQ

481 530

ISNKNLDNEI LLADSGYGQG EILINPVQIL SIYSALENNG NINAPHLLKD

531 580

TKNKVWKKNI ISKENINLLN DGMQQVVNKT HKEDIYRSYA NLIGKSGTAE

581 630

LKMKQGESGR QIGWFISYDK DNPNMMMAIN VKDVQDKGMA SYNAKISGKV

631 646

YDELYENGNK KYDIDE

**17.** A polynucleotide compound encoding the compound of Claim 16.

**18.** A method of purifying and assaying for agents which possess the ability to bind to PBP2As of MRS strains, said method comprising the steps of:

A. producing a PBP2A protein covalently bonded to a chelating agent capable of forming a coordinate covalent complex with a transition metal ion,

B. introducing said PBP2A covalently bonded to a chelating agent to a solid support, said solid support possessing an immobilized transition metal ion, said transition metal ion being in a kinetically labile oxidation state, so as to form a coordinate covalent complex between the PBP2A protein covalently bonded to a chelating agent and said immobilized transition metal ion,

C. changing the oxidation state of said transition metal ion to a kinetically inert oxidation state so as to form a kinetically inert coordinate covalent complex between the PBP2A protein possessing the chelating agent and the immobilized transition metal ion,

D. introducing compounds to said kinetically inert complex, and

E. assaying for binding of said compounds to the PBP2A protein portion of said kinetically inert coordinate covalent complex.

19. The method of Claim 18 wherein said transition metal ion is selected from the group comprising V, Cr, Mn, Fe, Ru, Os, Co, Rh, Pd, or Pt.

20. The method of Claim 19 wherein at least one of said chelators is an organic chelating moiety selected from the group comprising bidentate, tridentate, quadridentate, tripod, and macrocyclic ligands.

21. The method of Claim 20 wherein at least one of said chelators is an organic chelating moiety selected from the group comprising iminodiacetic acid, nitrilotriacetic acid, terpyridine, bipyridiene, triethylenetetraamine, biethylene triamine and derivatives thereof.

22. The method of Claim 20 wherein at least one of said chelators is a chelating peptide of the formula:

$$(His)_x\text{-}(A)_y\text{-}(His)_z\text{-}(Pro)_n$$

wherein:

A is one or more amino acids,
x = 1-10,
y = 0-4,
z = 1-10,
n = 0 or 1,
and monomers, dimers, and trimers thereof wherein each monomer unit of said dimers or trimers is the same or different.

23. The method of Claim 22 wherein x=1-3, y=0-4, z=1-3.

24. The method of Claim 23 wherein said chelating peptide is selected from chelating peptides of the formula His-Trp-His-Met-Tyr, His-His-His-Met-Tyr, His-Trp-His-Trp-His, His-Trp-His-His-His, His-His-His-His-Tyr-Met-His-His-His-His-Tyr, His-His-His-His-His, His-Trp-His-His-His His-Trp-His-Met-Tyr, His-His-His-Met-Tyr, His-Trp-His-Trp-His-Pro, His-Trp-His-His-His-Pro, His-His-His-His-Tyr-Met-His-His-His-His-Tyr-Pro, His-His-His-His-His-Pro, and His-Trp-His-His-His-Pro.

25. The method of Claim 24 wherein said penicillin binding protein comprises the compound of Claim 16.

26. A method of assaying for agents which bind to PBP2A proteins, said method comprising the steps of:
A. culturing cells which recombinantly express a $PBP2A^s$ protein,
B. lysing said cells and removing the intact cells, membranes, and vesicles,
C. isolating the crude cell exract,
D. exposing said crude cell extracts to potential inhibitors of PBP2A,
D. introducing to said crude cell extract to labelled penicillin V,
E. removing unbound labelled penicillin V, and
F. assaying for the presence of labelled penicillin V remaining in the crude extract.

27. The method of Claim 26 wherein said $PBP2A^s$ protein comprises the compound of Claim 16.

28. A method of assaying for agents which bind to PBP2A proteins, said method comprising the steps of:
A. culturing cells which recombinantly express a $PBP2A^s$-CP protein,
B. lysing said cells and purifying said $PBP2A^s$-CP protein,
C. exposing said $PBP2A^s$-CP to potential inhibitors of PBP2A,
D. introducing labelled penicillin V to said purified $PBP2A^s$-CP,
E. removing unbound labelled penicillin V, and
F. assaying for the presence of labelled penicillin V remaining bound to the purified $PBP2A^s$-CP.

29. The method of Claim 28 wherein said $PBP2A^s$ protein comprises the compound of Claim 16.

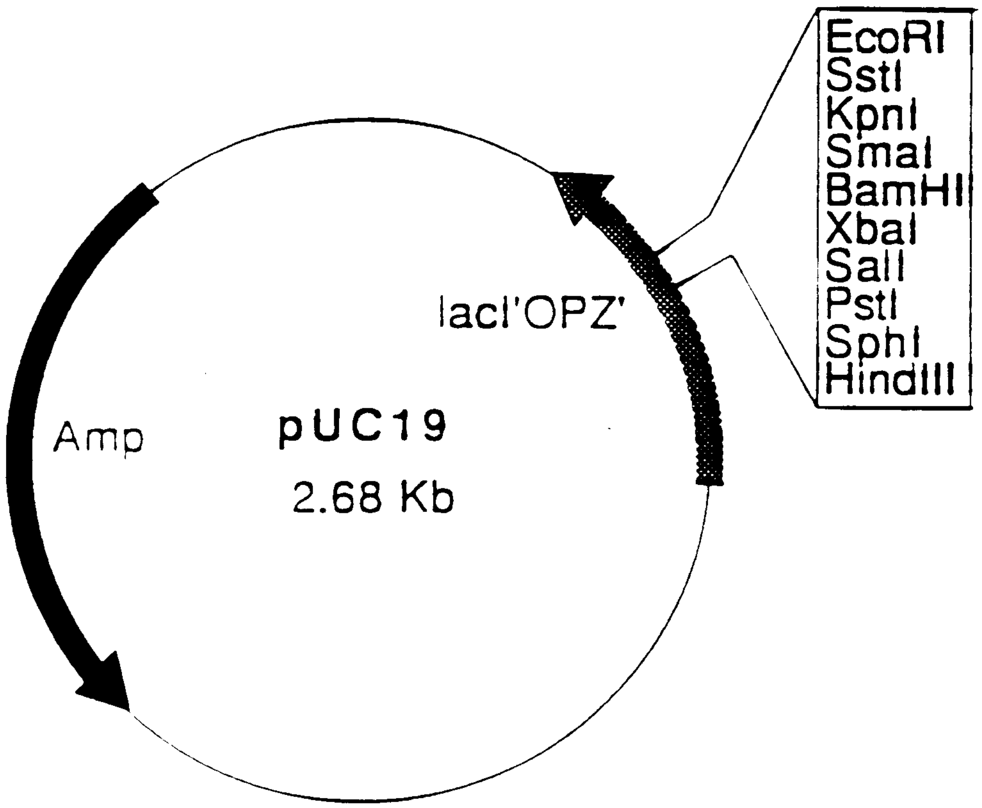
EcoRI
SstI
KpnI
SmaI
BamHI
XbaI
SalI
PstI
SphI
HindIII
lacI'OPZ'
Amp
pUC19
2.68 Kb

FIG. 1

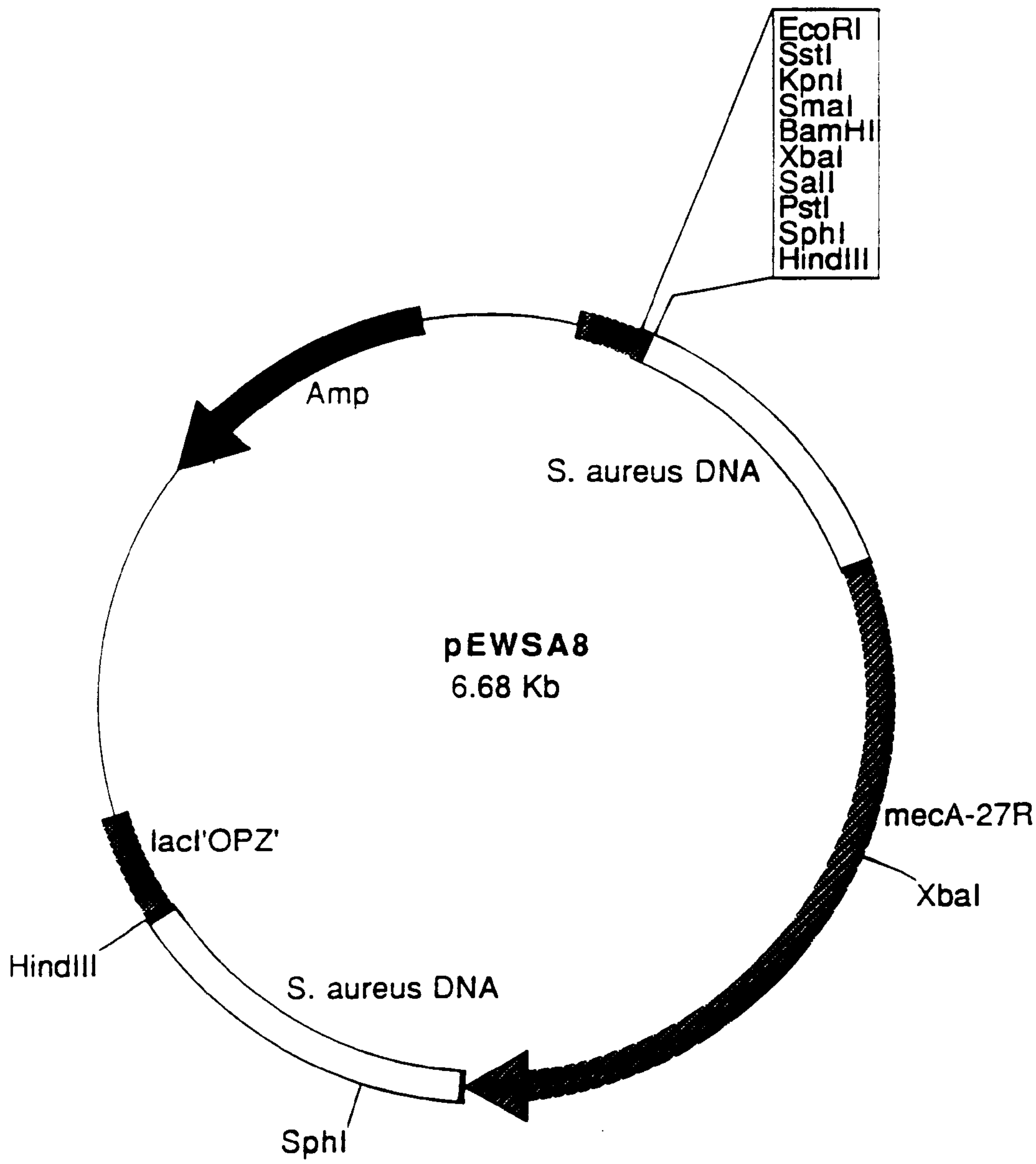
EcoRI
SstI
KpnI
SmaI
BamHI
XbaI
SalI
PstI
SphI
HindIII
Amp
S. aureus DNA
pEWSA8
6.68 Kb
mecA-27R
XbaI
lacI'OPZ'
HindIII
S. aureus DNA
SphI

FIG. 2

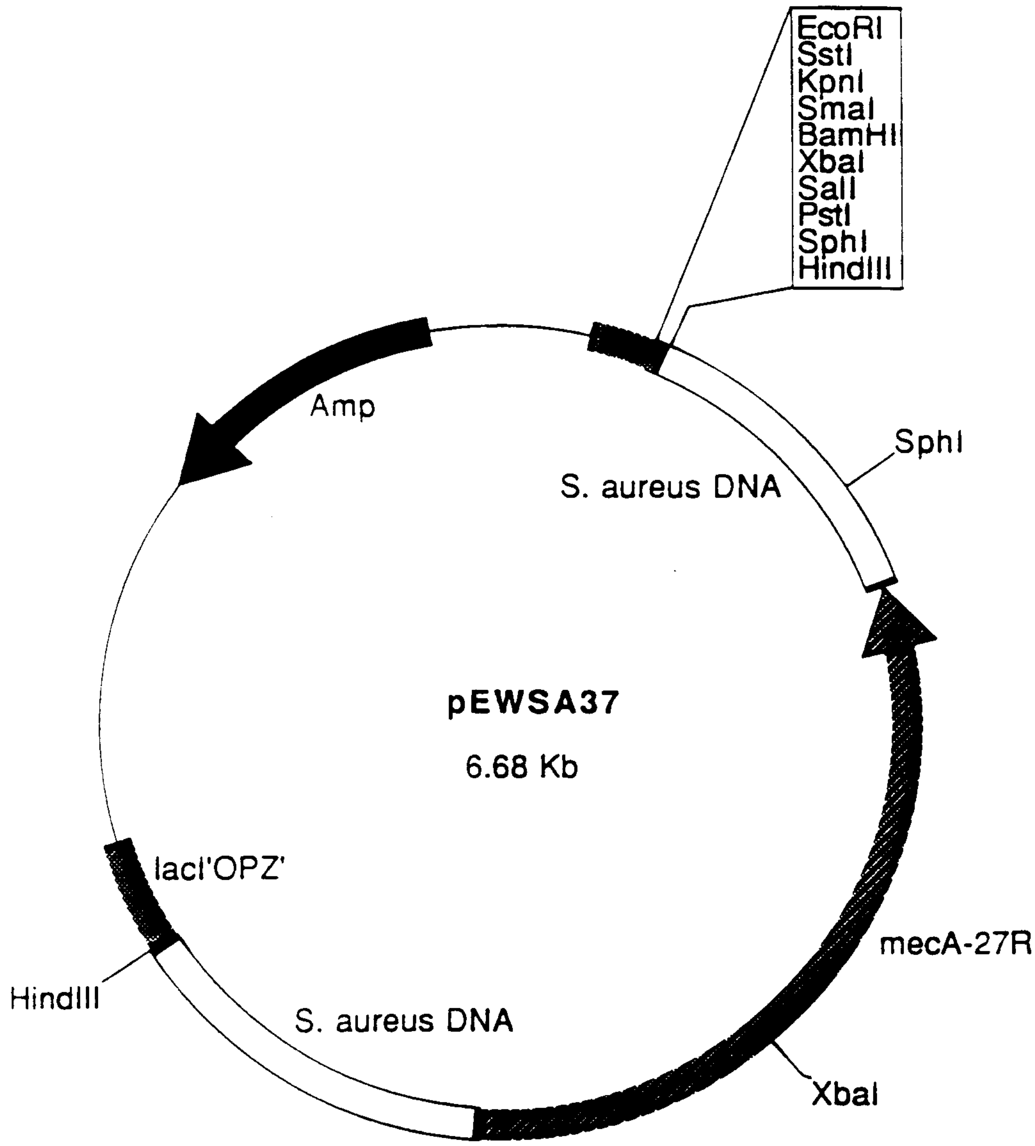
EcoRI
SstI
KpnI
SmaI
BamHI
XbaI
SalI
PstI
SphI
HindIII
Amp
SphI
S. aureus DNA
pEWSA37
6.68 Kb
lacI'OPZ'
mecA-27R
HindIII
S. aureus DNA
XbaI

FIG. 3

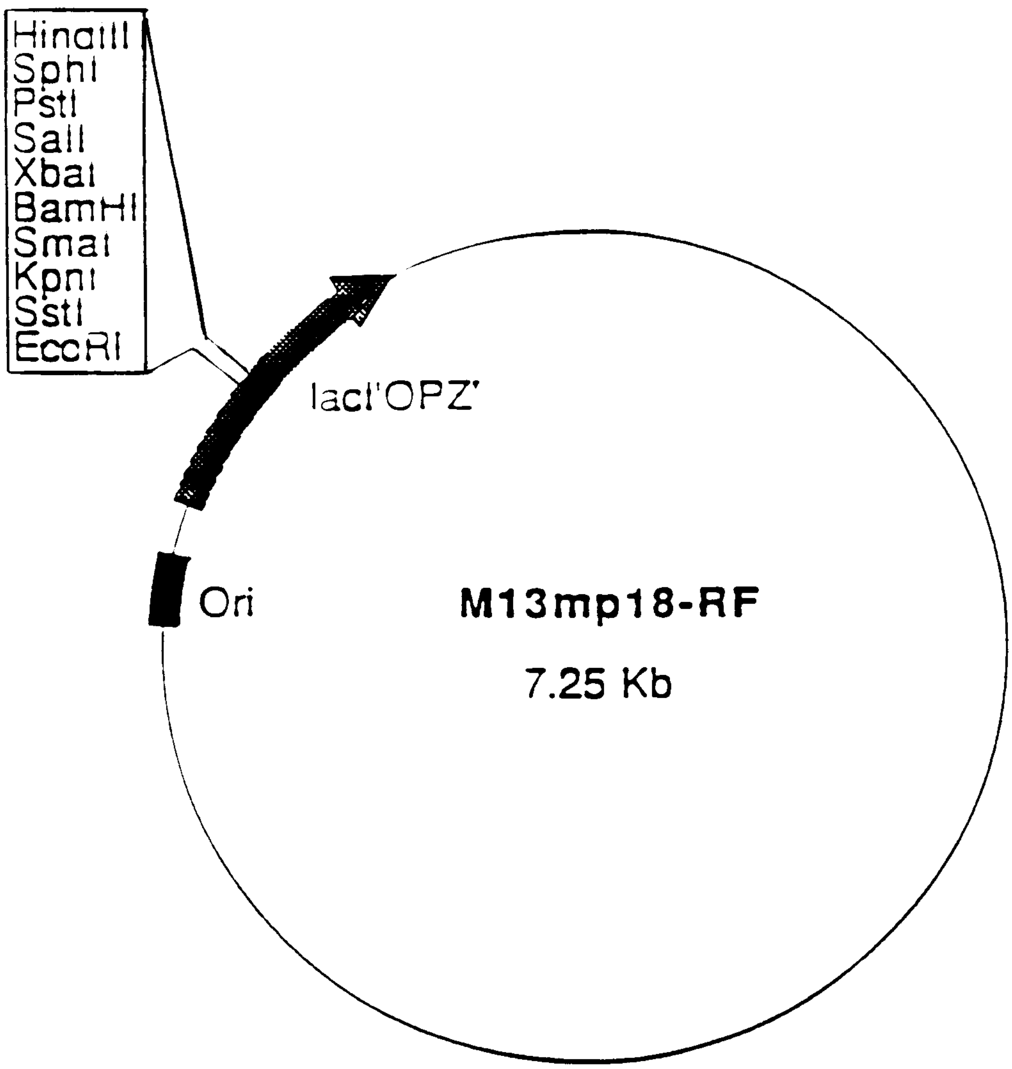
HincII
SphI
PstI
SalI
XbaI
BamHI
SmaI
KpnI
SstI
EcoRI
lacI'OPZ'
Ori
M13mp18-RF
7.25 Kb

FIG. 4

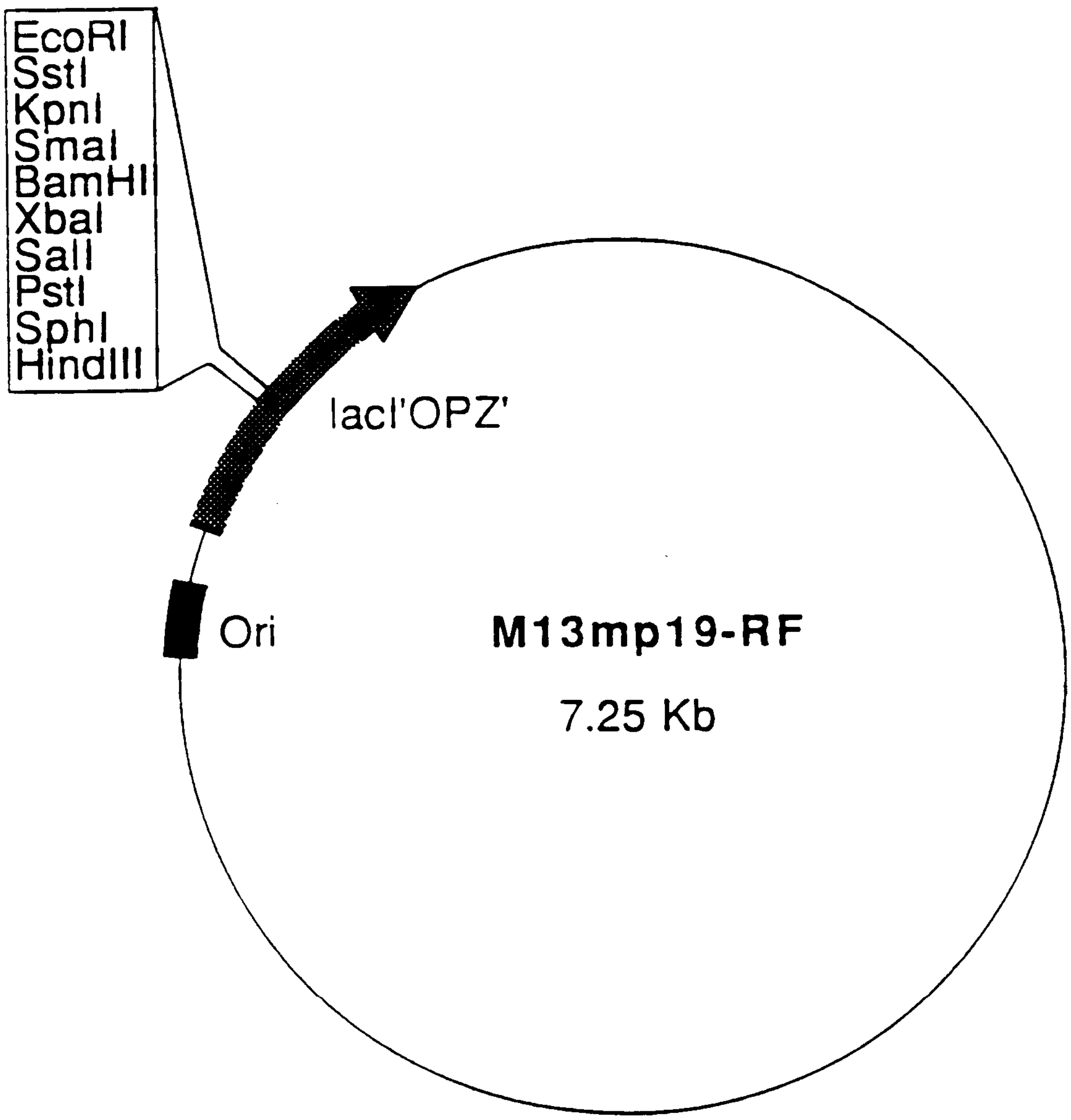
EcoRI
SstI
KpnI
SmaI
BamHI
XbaI
SalI
PstI
SphI
HindIII
lacI'OPZ'
Ori
M13mp19-RF
7.25 Kb

FIG. 5

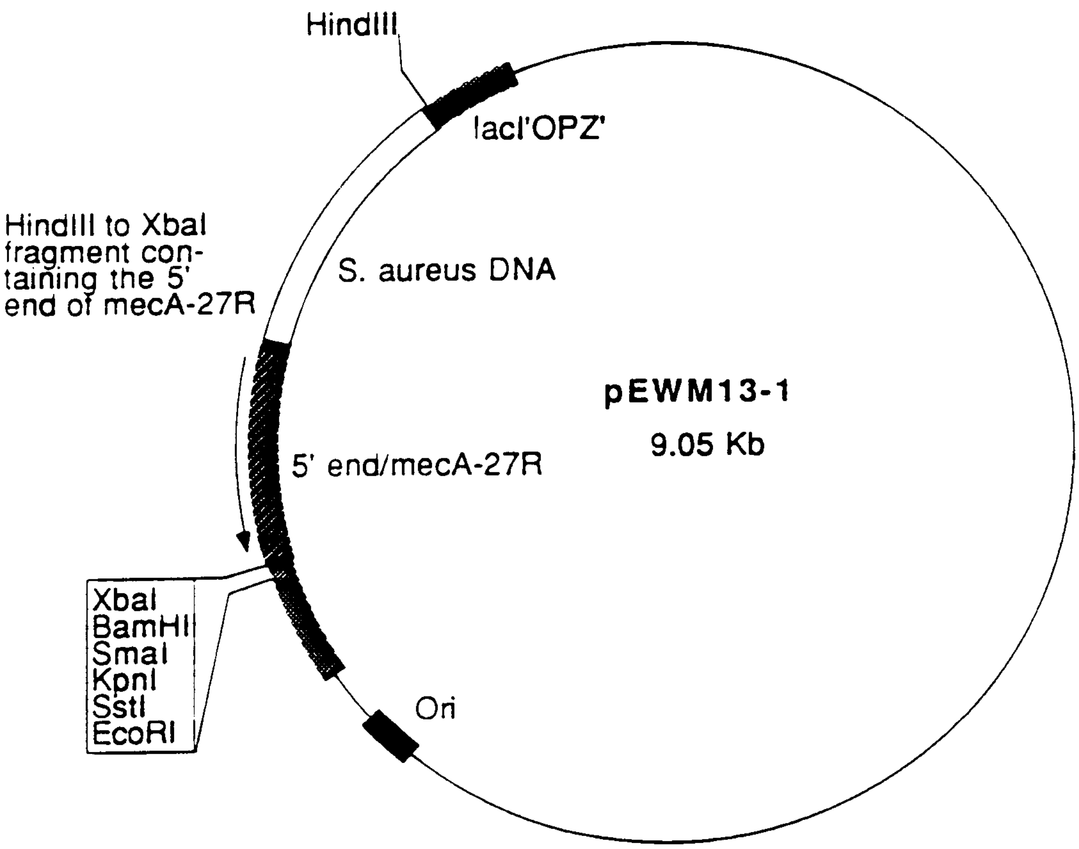
HindIII
lacI'OPZ'
HindIII to XbaI fragment containing the 5' end of mecA-27R
S. aureus DNA
pEWM13-1
9.05 Kb
5' end/mecA-27R
XbaI
BamHI
SmaI
KpnI
SstI
EcoRI
Ori

FIG. 6

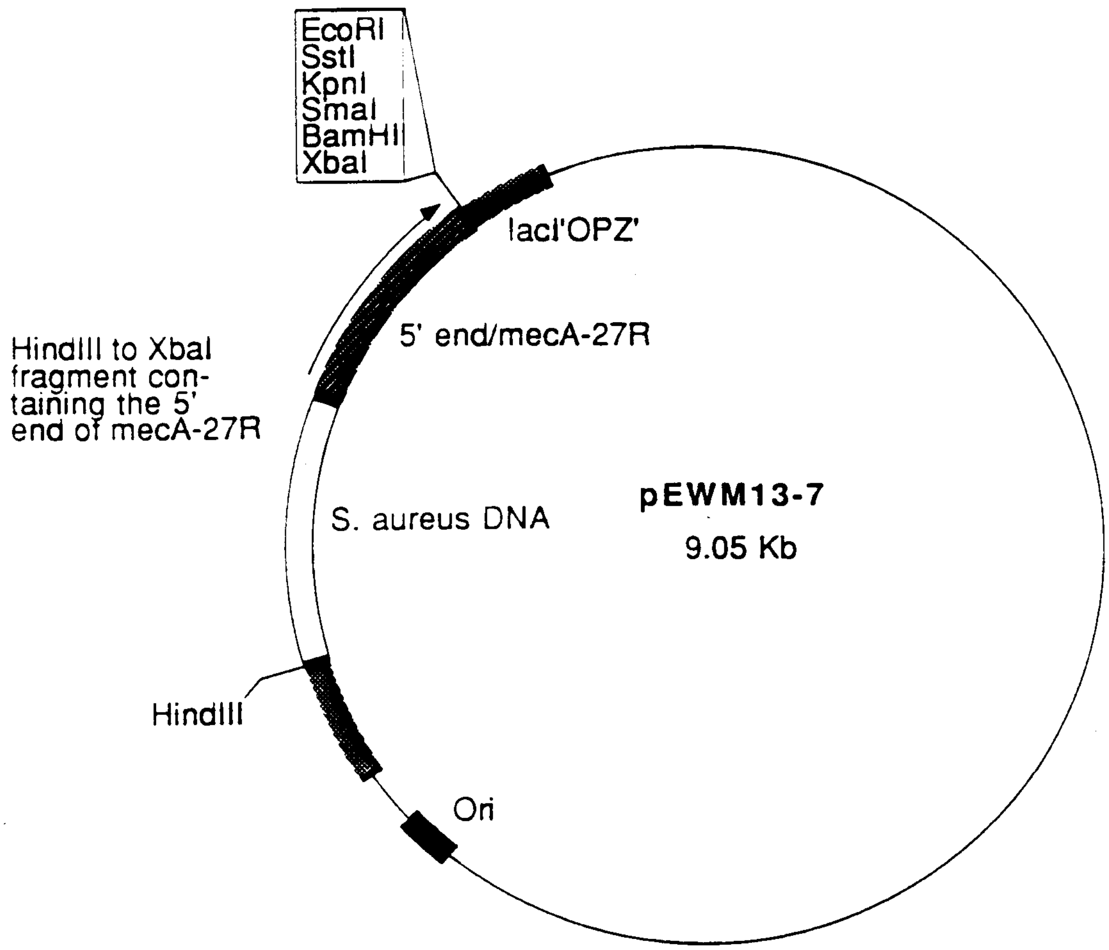
EcoRI
SstI
KpnI
SmaI
BamHI
XbaI
lacI'OPZ'
5' end/mecA-27R
HindIII to XbaI fragment containing the 5' end of mecA-27R
S. aureus DNA
pEWM13-7
9.05 Kb
HindIII
Ori

FIG. 7

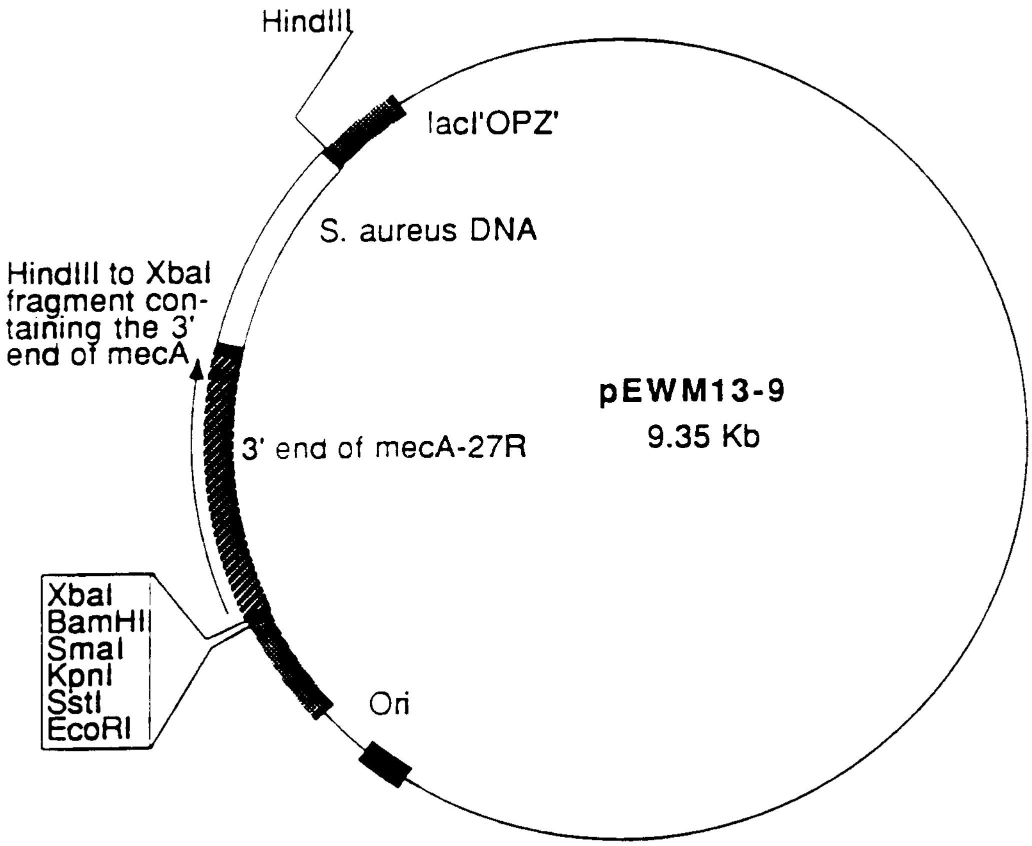
HindIII
lacI'OPZ'
S. aureus DNA
HindIII to XbaI fragment containing the 3' end of mecA
pEWM13-9
9.35 Kb
3' end of mecA-27R
XbaI
BamHI
SmaI
KpnI
SstI
EcoRI
Ori

FIG. 8

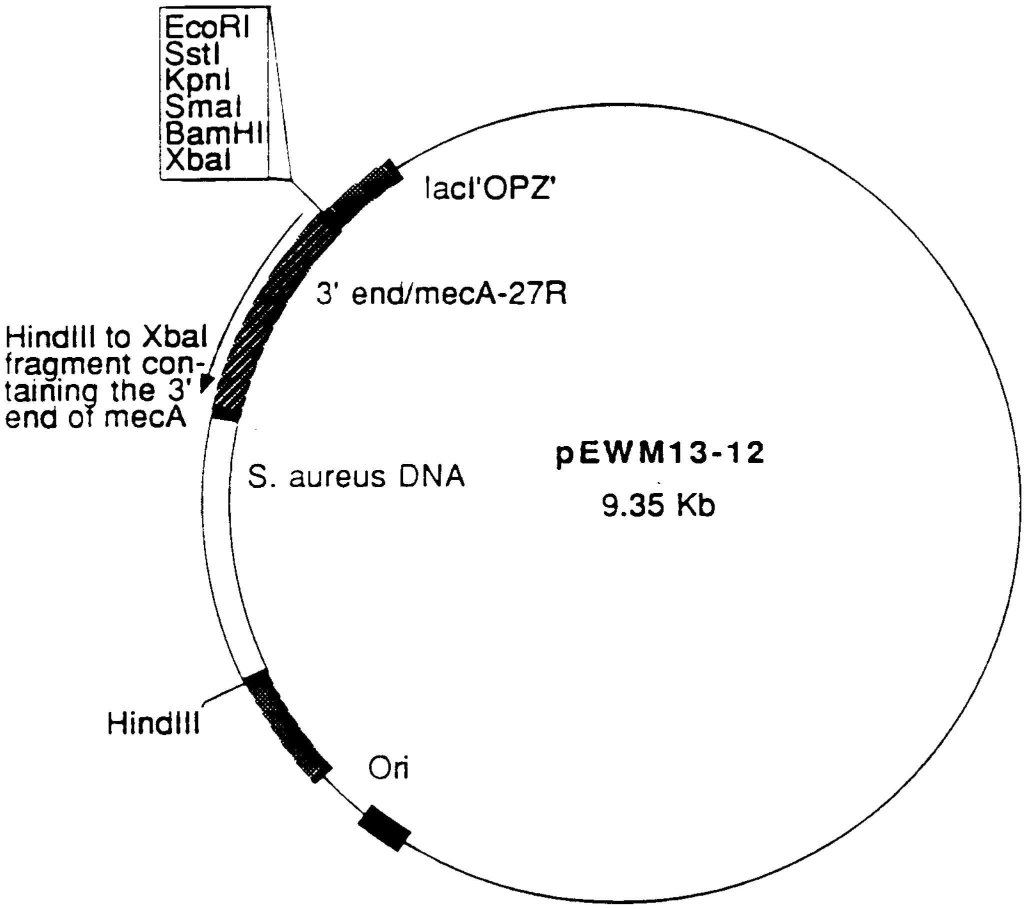
EcoRI
SstI
KpnI
SmaI
BamHI
XbaI
lacI'OPZ'
3' end/mecA-27R
HindIII to XbaI fragment containing the 3' end of mecA
S. aureus DNA
pEWM13-12
9.35 Kb
HindIII
Ori

FIG. 9

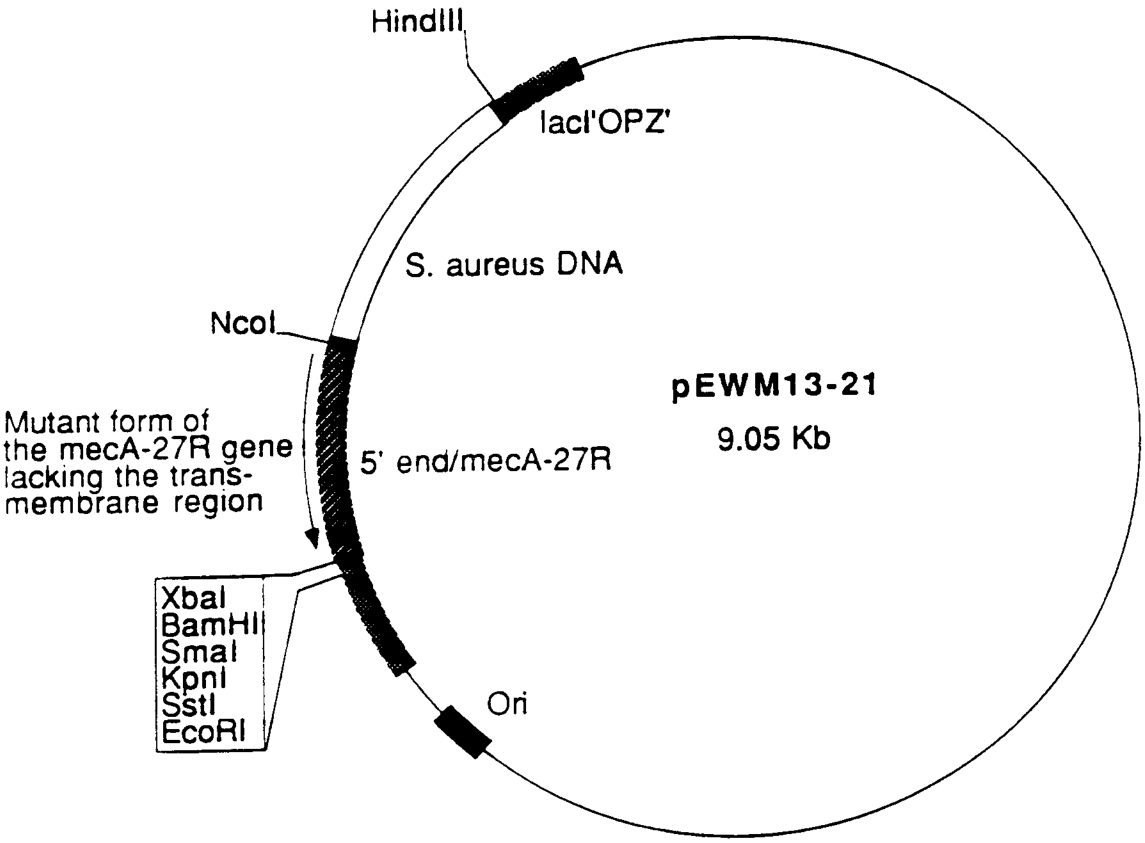
HindIII
lacI'OPZ'
S. aureus DNA
NcoI
pEWM13-21
9.05 Kb
Mutant form of the mecA-27R gene lacking the trans-membrane region
5' end/mecA-27R
XbaI
BamHI
SmaI
KpnI
SstI
EcoRI
Ori

FIG. 10

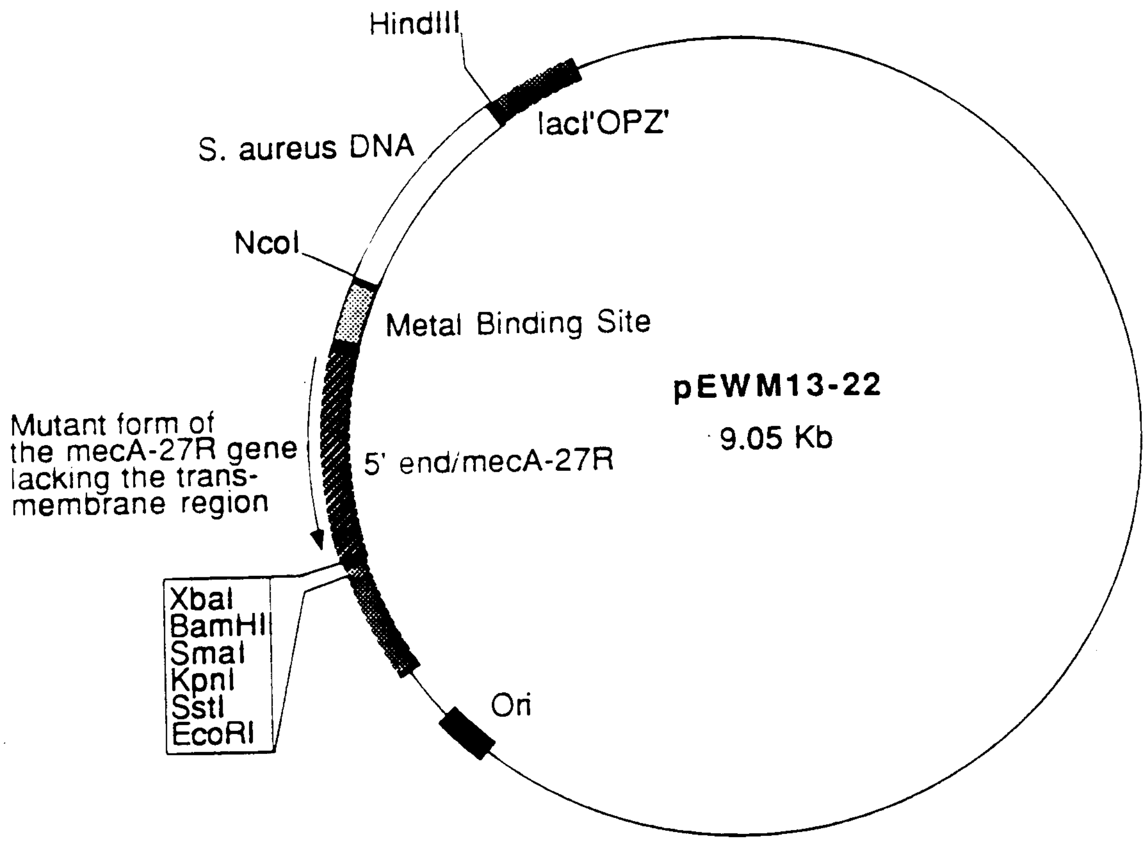
HindIII
lacI'OPZ'
S. aureus DNA
NcoI
Metal Binding Site
pEWM13-22
9.05 Kb
Mutant form of the mecA-27R gene lacking the trans-membrane region
5' end/mecA-27R
XbaI
BamHI
SmaI
KpnI
SstI
EcoRI
Ori

FIG. 11

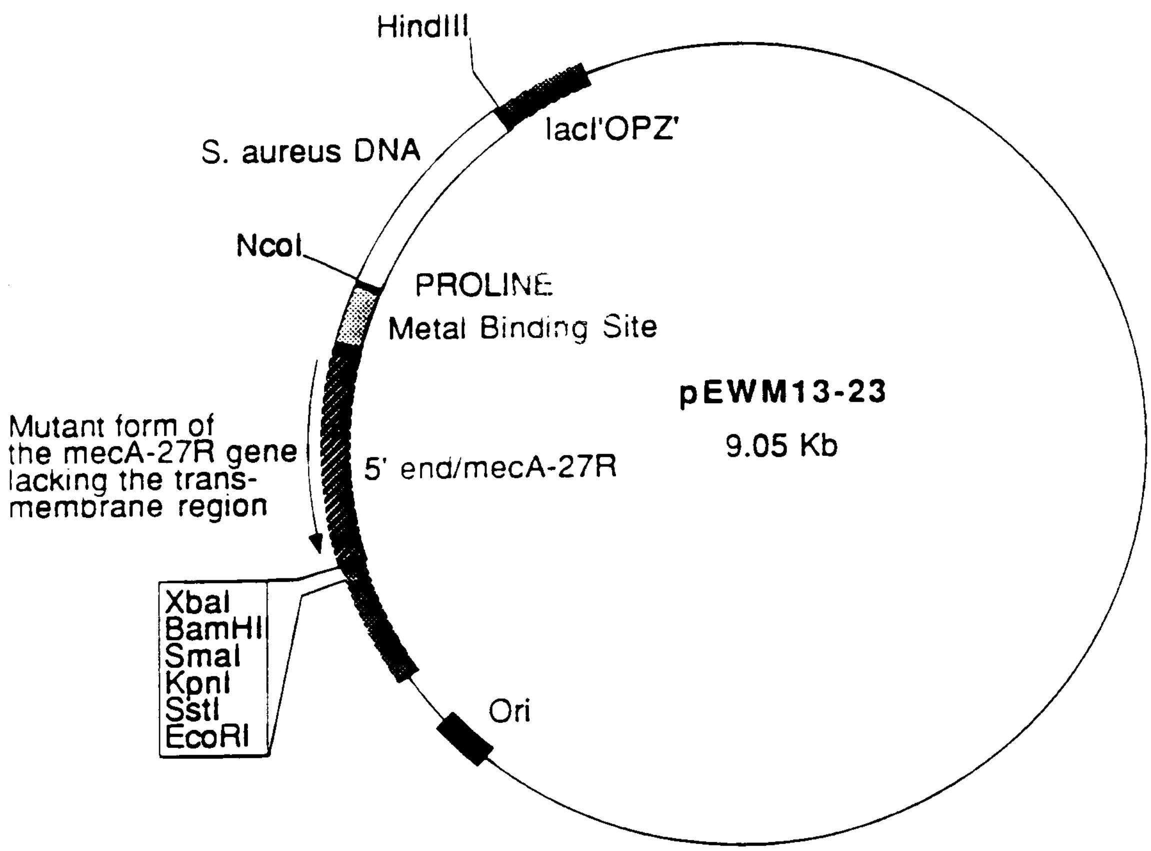
HindIII
lacI'OPZ'
S. aureus DNA
NcoI
PROLINE
Metal Binding Site
pEWM13-23
9.05 Kb
Mutant form of
the mecA-27R gene
lacking the trans-
membrane region
5' end/mecA-27R
XbaI
BamHI
SmaI
KpnI
SstI
EcoRI
Ori

FIG. 12

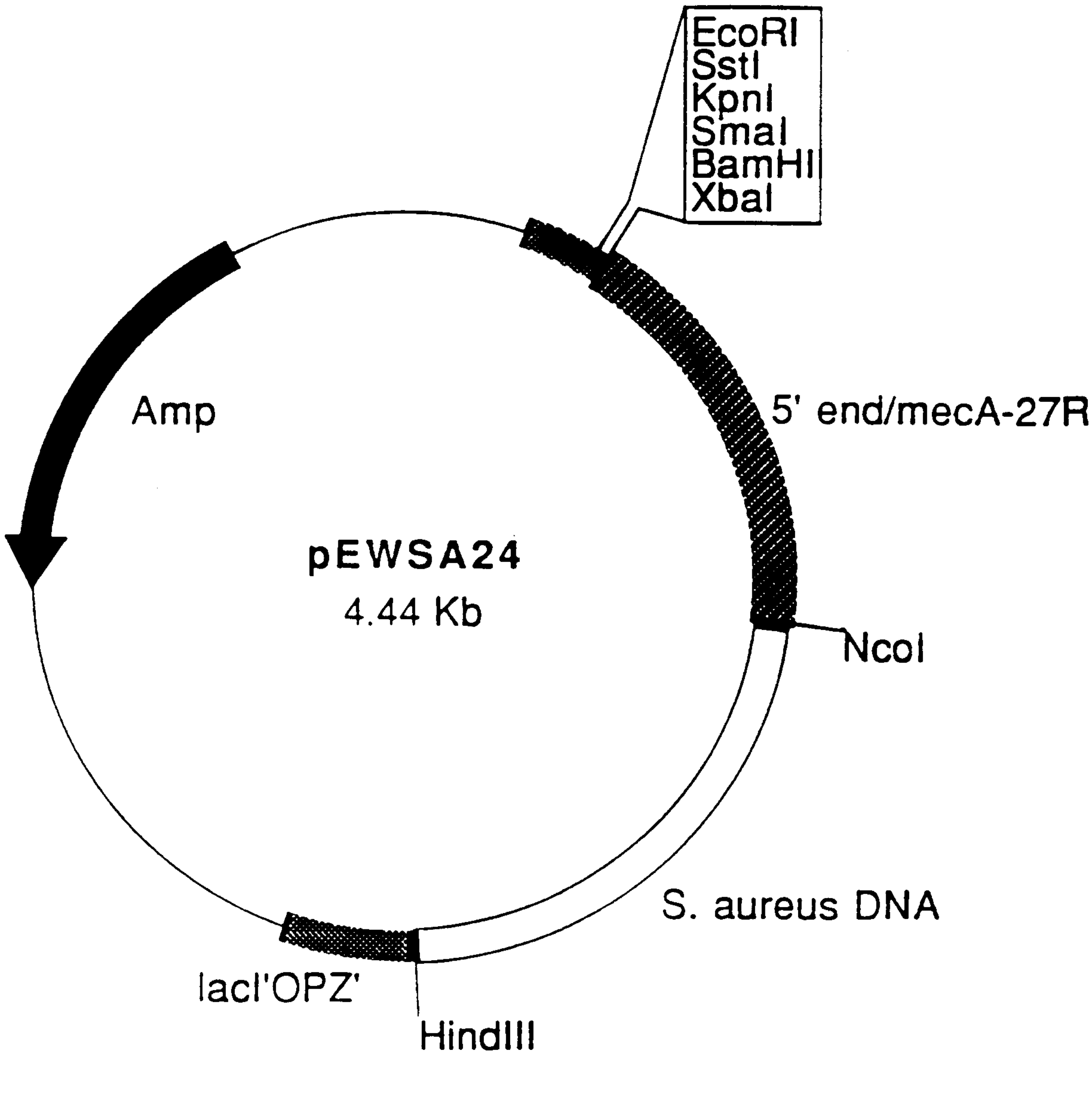
EcoRI
SstI
KpnI
SmaI
BamHI
XbaI
Amp
5' end/mecA-27R
pEWSA24
4.44 Kb
NcoI
S. aureus DNA
lacI'OPZ'
HindIII

FIG. 13

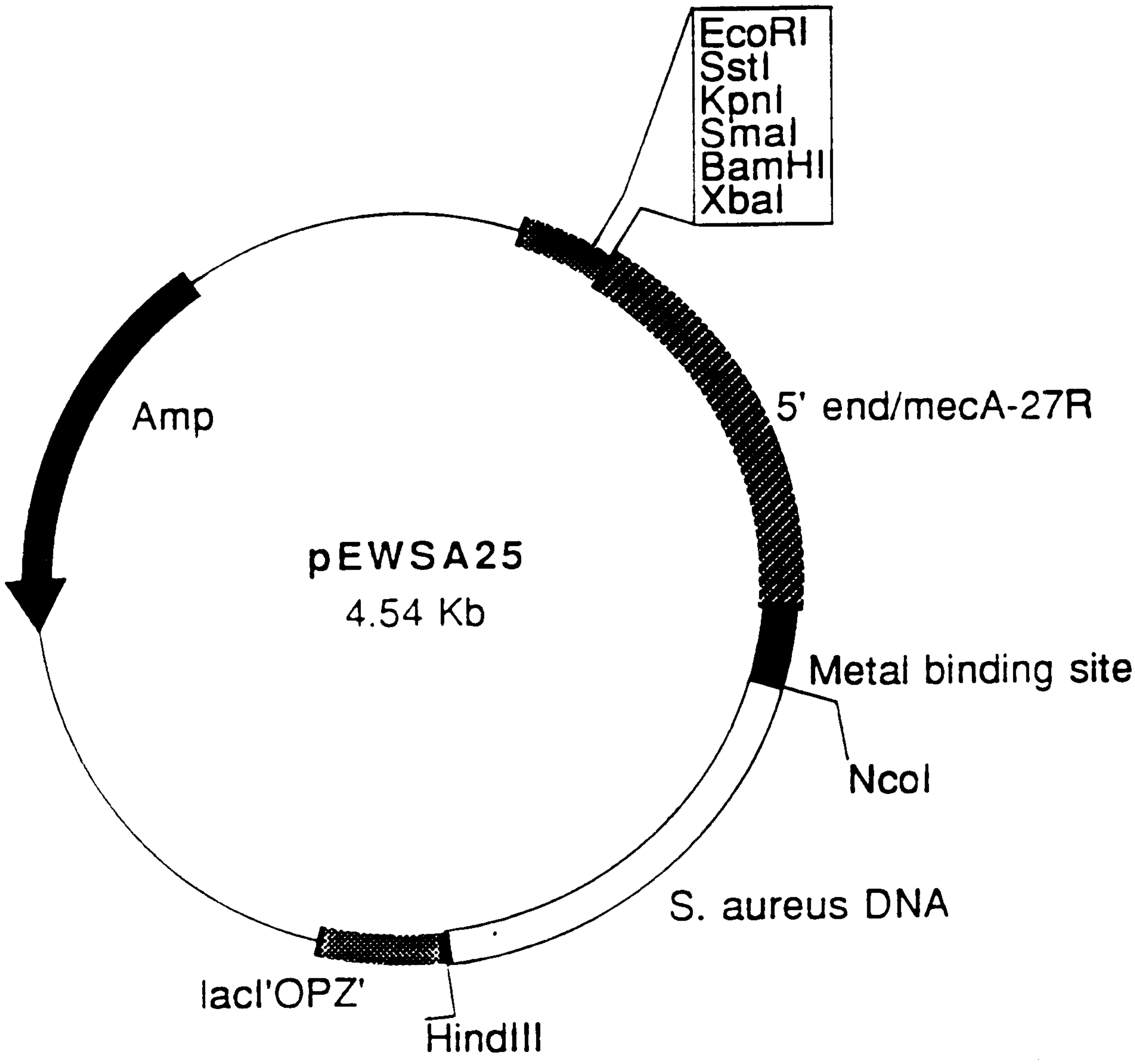
EcoRI
SstI
KpnI
SmaI
BamHI
XbaI
5' end/mecA-27R
Amp
pEWSA25
4.54 Kb
Metal binding site
NcoI
S. aureus DNA
lacI'OPZ'
HindIII

FIG. 14

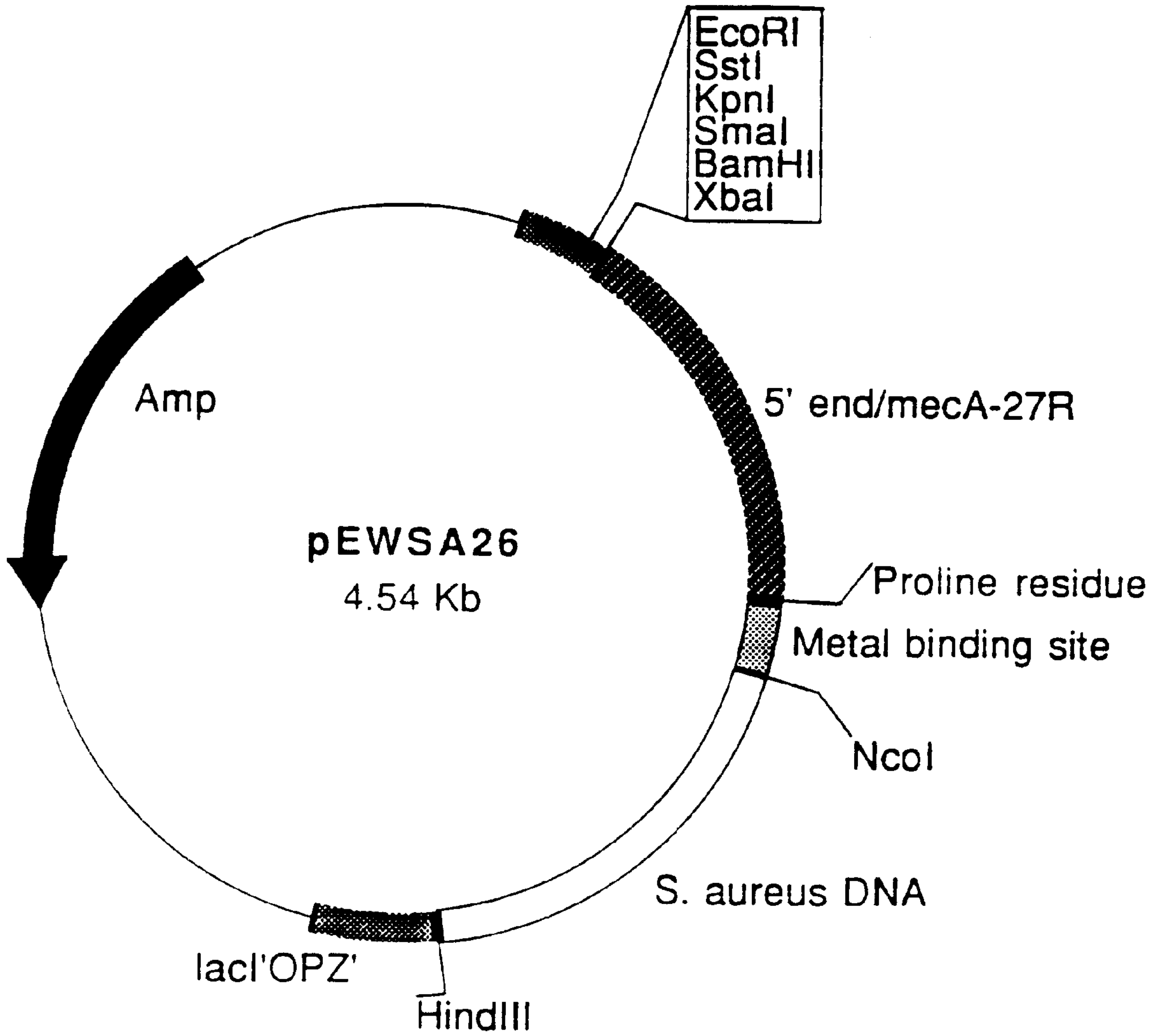
EcoRI
SstI
KpnI
SmaI
BamHI
XbaI
Amp
5' end/mecA-27R
pEWSA26
4.54 Kb
Proline residue
Metal binding site
NcoI
S. aureus DNA
lacI'OPZ'
HindIII

FIG. 15

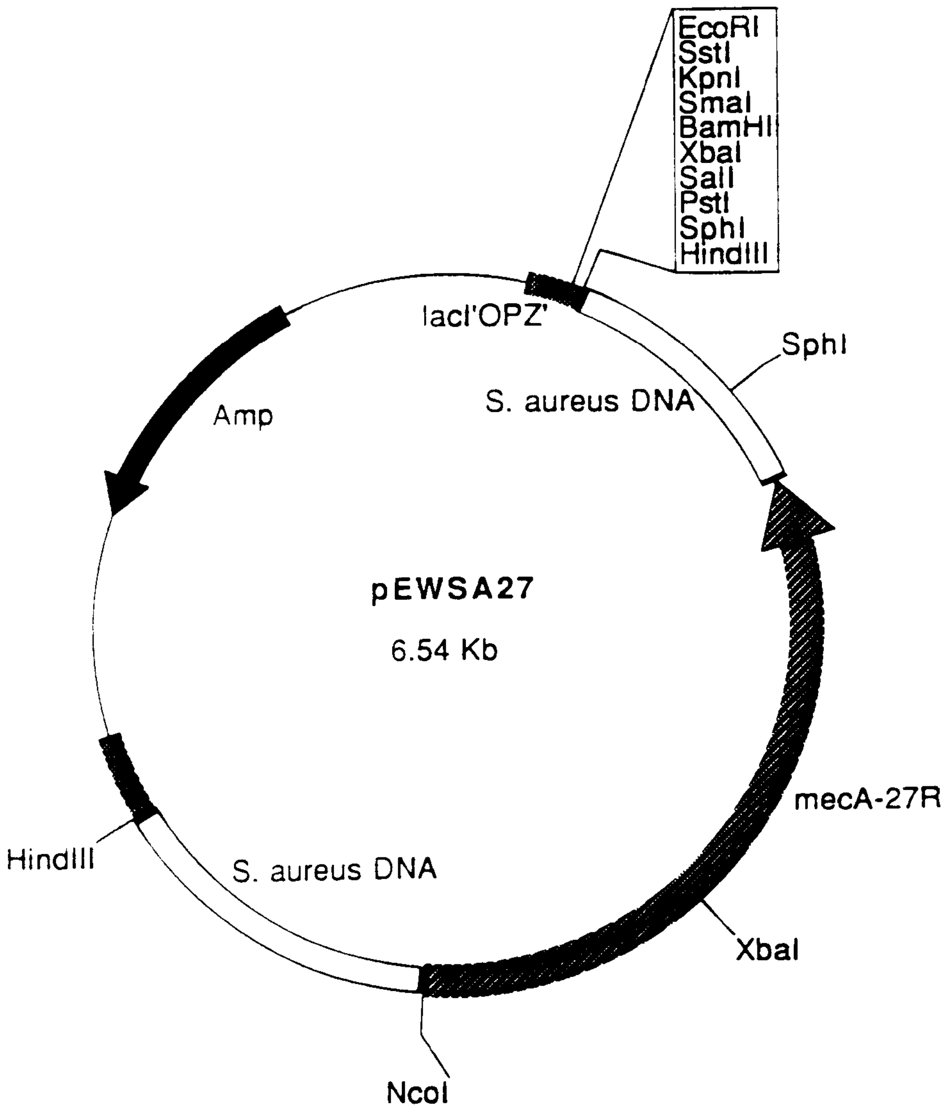
EcoRI
SstI
KpnI
SmaI
BamHI
XbaI
SalI
PstI
SphI
HindIII
lacI'OPZ'
SphI
S. aureus DNA
Amp
pEWSA27
6.54 Kb
mecA-27R
HindIII
S. aureus DNA
XbaI
NcoI

FIG. 16

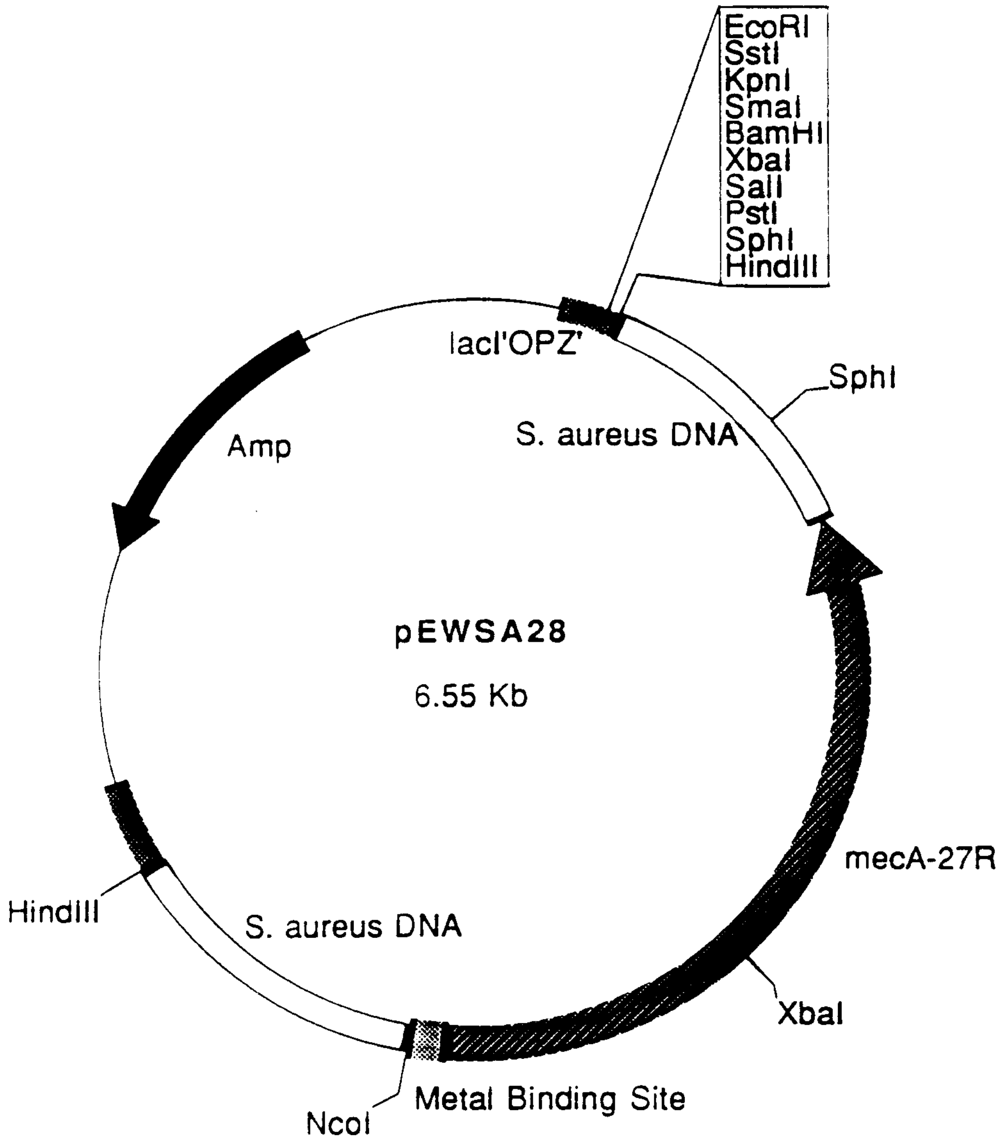
EcoRI
SstI
KpnI
SmaI
BamHI
XbaI
SalI
PstI
SphI
HindIII
lacI'OPZ'
SphI
S. aureus DNA
Amp
pEWSA28
6.55 Kb
mecA-27R
HindIII
S. aureus DNA
XbaI
Metal Binding Site
NcoI

FIG. 17

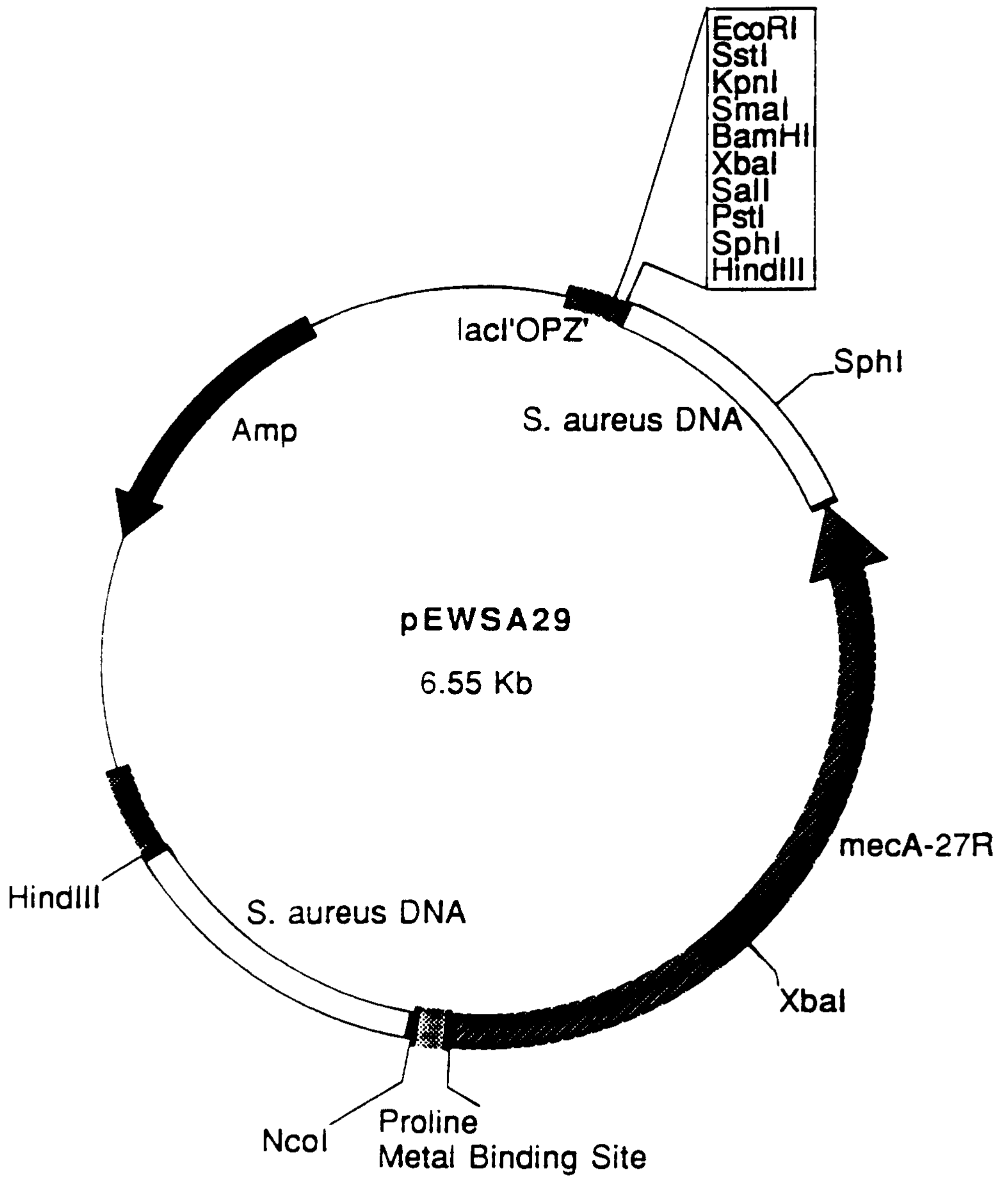
EcoRI
SstI
KpnI
SmaI
BamHI
XbaI
SalI
PstI
SphI
HindIII
lacI'OPZ'
SphI
S. aureus DNA
Amp
pEWSA29
6.55 Kb
mecA-27R
HindIII
S. aureus DNA
XbaI
NcoI
Proline
Metal Binding Site

FIG. 18

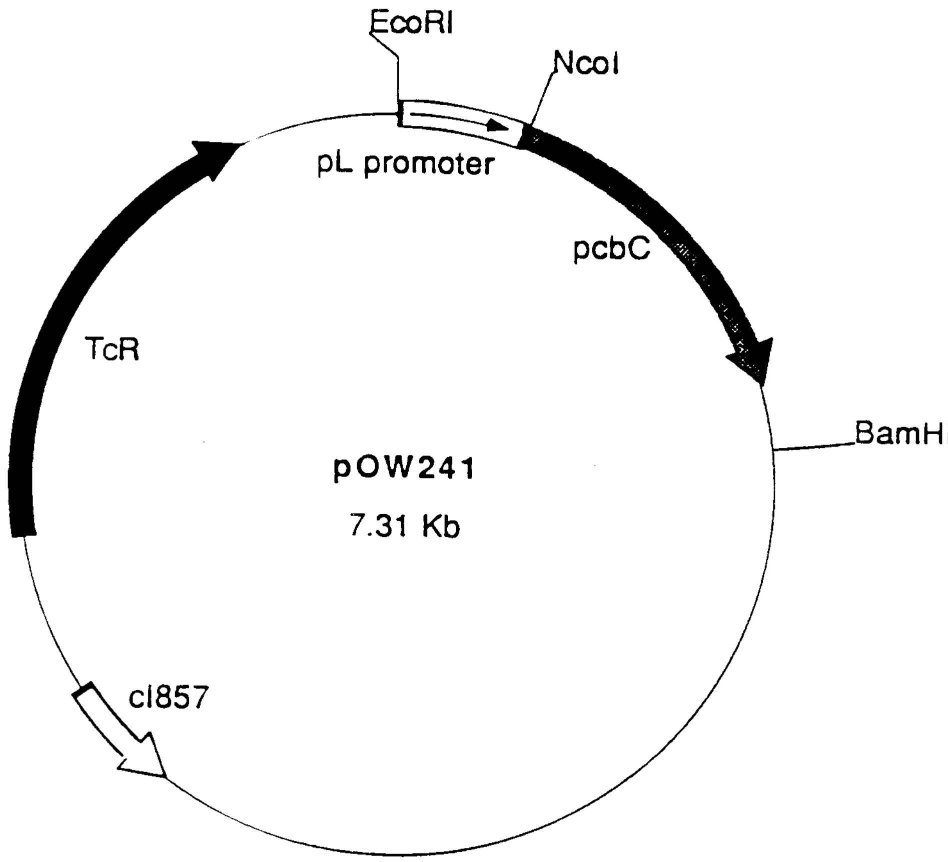


FIG. 19

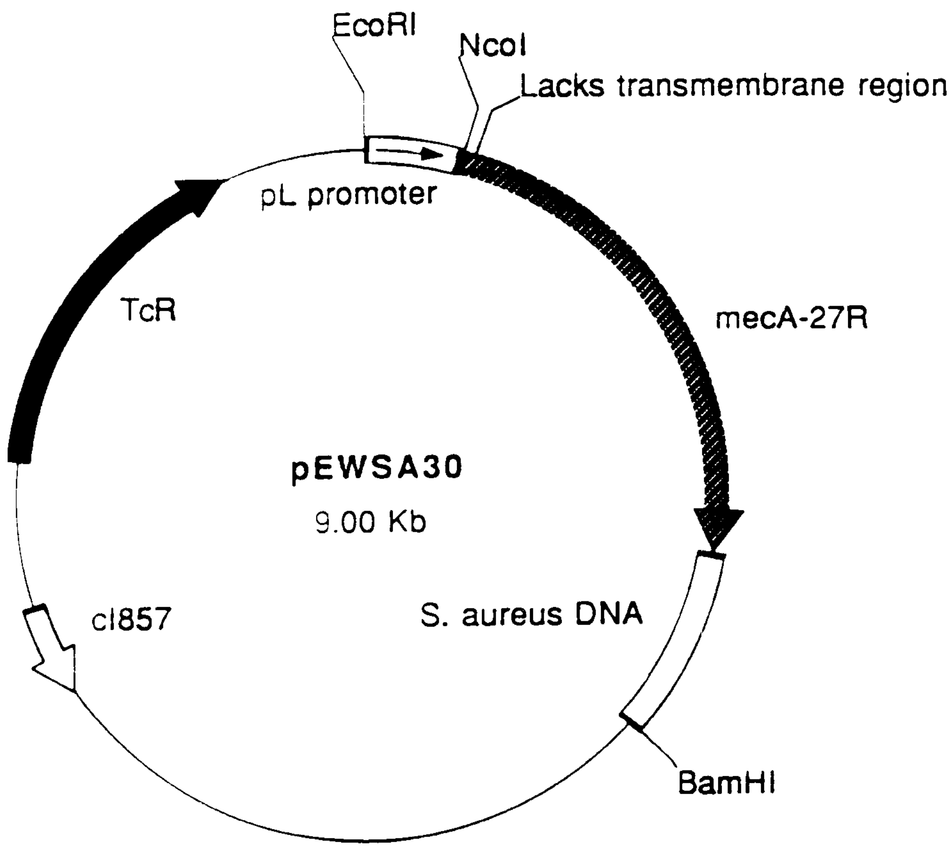
EcoRI
NcoI
Lacks transmembrane region
pL promoter
TcR
mecA-27R
pEWSA30
9.00 Kb
cI857
S. aureus DNA
BamHI

FIG. 20

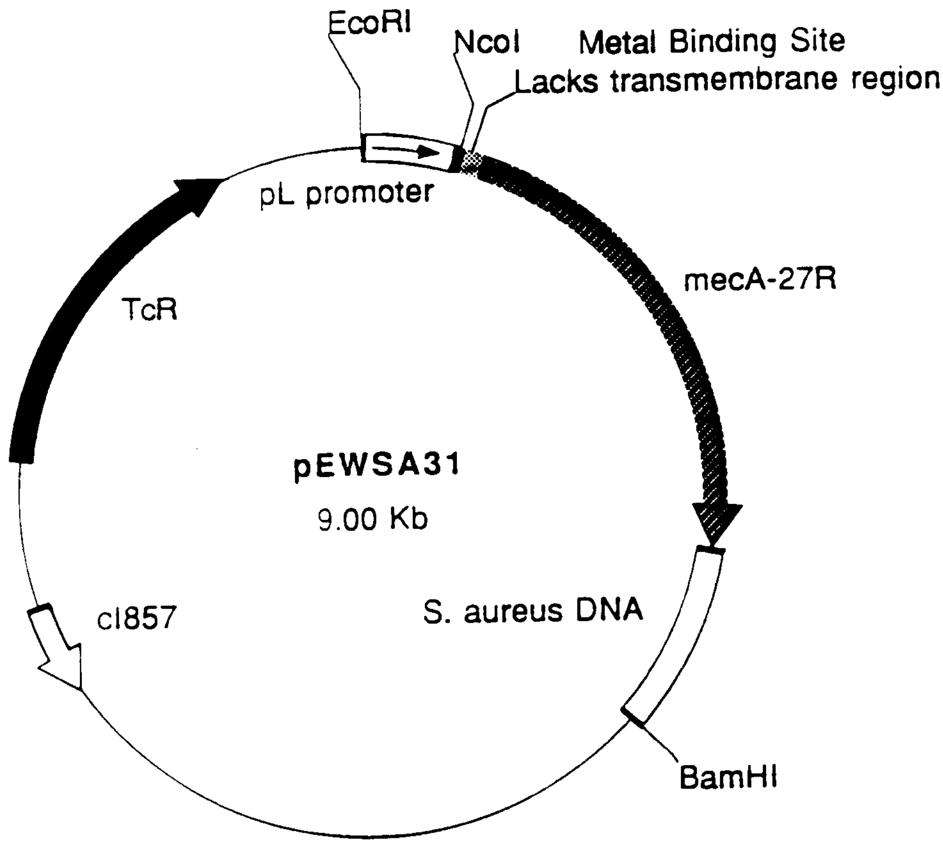
EcoRI
NcoI
Metal Binding Site
Lacks transmembrane region
pL promoter
mecA-27R
TcR
pEWSA31
9.00 Kb
cI857
S. aureus DNA
BamHI

FIG. 21

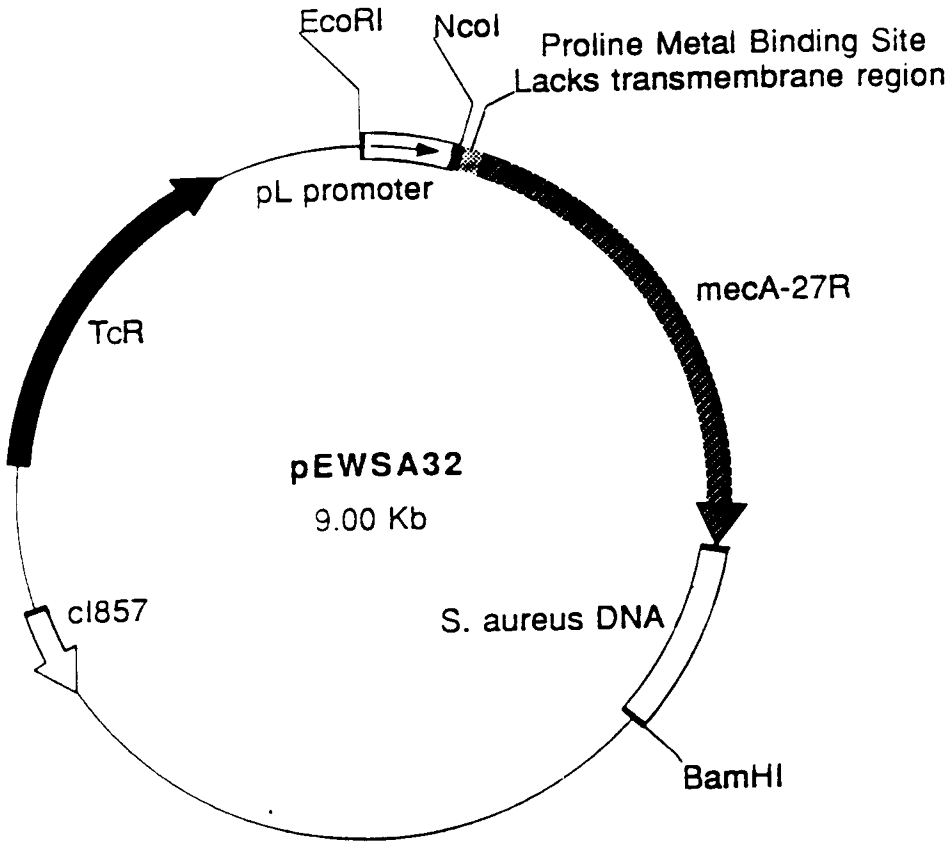
EcoRI
NcoI
Proline Metal Binding Site
Lacks transmembrane region
pL promoter
mecA-27R
TcR
pEWSA32
9.00 Kb
cI857
S. aureus DNA
BamHI

FIG. 22

1 ATGAAAAAGATAAAAATTGTTCCACTTATTTTAATAGTTGTAGTTGTCGGGTTTGGTATA 60

61 TATTTTTATGCTTCAAAAGATAAAGAAATTAATAATACTATTGATGCAATTGAAGATAAA 120

121 AATTTCAAACAAGTTTATAAAGATAGCAGTTATATTTCTAAAAGCGATAATGGTGAAGTA 180

181 GAAATGACTGAACGTCCGATAAAAATATATAATAGTTTAGGCGTTAAAGATATAAACATT 240

241 CAGGATCGTAAAATAAAAAAAGTATCTAAAAATAAAAAACGAGTAGATGCTCAATATAAA 300

301 ATTAAAACAAACTACGGTAACATTGATCGCAACGTTCAATTTAATTTTGTTAAAGAAGAT 360

361 GGTATGTGGAAGTTAGATTGGGATCATAGCGTCATTATTCCAGGAATGCAGAAAGACCAA 420

421 AGCATACATATTGAAAATTTAAAATCAGAACGTGGTAAAATTTTAGACCGAAACAATGTG 480

481 GAATTGGCCAATACAGGAACAGCATATGAGATAGGCATCGTTCCAAAGAATGTATCTAAA 540

541 AAAGATTATAAAGCAATCGCTAAAGAACTAAGTATTTCTGAAGACTATATCAAACAACAA 600

601 ATGGATCAAAATTGGGTACAAGATGATACCTTCGTTCCACTTAAAACCGTTAAAAAAATG 660

661 GATGAATATTTAAGTGATTTCGCAAAAAAATTTCATCTTACAACTAATGAAACAGAAAGT 720

721 CGTAACTATCCTCTAGAAAAAGCGACTTCACATCTATTAGGTTATGTTGGTCCCATTAAC 780

781 TCTGAAGAATTAAAACAAAAAGAATATAAAGGCTATAAAGATGATGCAGTTATTGGTAAA 840

841 AAGGGACTCGAAAAACTTTACGATAAAAAGCTCCAACATGAAGATGGCTATCGTGTCACA 900

901 ATCGTTGACGATAATAGCAATACAATCGCACATACATTAATAGAGAAAAAGAAAAAAGAT 960

961 GGCAAAGATATTCAACTAACTATTGATGCTAAAGTTCAAAAGAGTATTTATAACAACATG 1020

1021 AAAAATGATTATGGCTCAGGTACTGCTATCCACCCTCAAACAGGTGAaTTATTAGCACTT 1080

1081 GTAAGCACACCTTCATATGACGTCTATCCATTTATGTATGGCATGAGTAACGAAGAATAT 1140

1141 AATAAATTAACCGAAGATAAAAAAGAACCTCTGCTCAACAAGTTCCAGATTACAACTTCA 1200

1201 CCAGGTTCAACTCAAAAAATATTAACAGCAATGATTGGGTTAAATAACAAAACATTAGAC 1260

1261 GATAAAACAAGTTATAAAATCGATGGTAAAGGTTGGCAAAAAGATAAATCTTGGGGTGGT 1320

1321 TACAACGTTACAAGATATGAAGTGGTAAATGGTAATATCGACTTAAAACAAGCAATAGAA 1380

1381 TCATCAGATAACATTTTCTTTGCTAGAGTAGCACTCGAATTAGGCAGTAAGAAATTTGAA 1440

FIG. 23

```
1441  AAAGGCATGAAAAAACTAGGTGTTGGTGAAGATATACCAAGTGATTATCCATTTTATAAT  1500
1501  GCTCAAATTTCAAACAAAAATTTAGATAATGAAATATTATTAGCTGATTCAGGTTACGGA  1560
1561  CAAGGTGAAATACTGATTAACCCAGTACAGATCCTTTCAATCTATAGCGCATTAGAAAAT  1620
1621  AATGGCAATATTAACGCACCTCACTTATTAAAAGACACGAAAAACAAAGTTTGGAAGAAA  1680
1681  AATATTATTTCCAAAGAAAATATCAATCTATTAACTGATGGTATGCAACAAGTCGTAAAT  1740
1741  AAAACACATAAAGAAGATATTTATAGATCTTATGCAAACTTAATTGGCAAATCCGGTACT  1800
1801  GCAGAACTCAAAATGAAACAAGGAGAAACTGGCAGACAAATTGGGTGGTTTATATCATAT  1860
1861  GATAAAGATAATCCAAACATGATGATGGCTATTAATGTTAAAGATGTACAAGATAAAGGA  1920
1921  ATGGCTAGCTACAATGCCAAAATCTCAGGTAAAGTGTATGATGAGCTATATGAGAACGGT  1980
1981  AATAAAAAATACGATATAGATGAATAA  2007
```

FIG. 23 **(continued)**

1 MetLysLysIleLysIleValProLeuIleLeuIleValValValVal 16

17 GlyPheGlyIleTyrPheTyrAlaSerLysAspLysGluIleAsnAsn 32

33 ThrIleAspAlaIleGluAspLysAsnPheLysGlnValTyrLysAsp 48

49 SerSerTyrIleSerLysSerAspAsnGlyGluValGluMetThrGlu 64

65 ArgProIleLysIleTyrAsnSerLeuGlyValLysAspIleAsnIle 80

81 GlnAspArgLysIleLysLysValSerLysAsnLysLysArgValAsp 96

97 AlaGlnTyrLysIleLysThrAsnTyrGlyAsnIleAspArgAsnVal 112

113 GlnPheAsnPheValLysGluAspGlyMetTrpLysLeuAspTrpAsp 128

129 HisSerValIleIleProGlyMetGlnLysAspGlnSerIleHisIle 144

145 GluAsnLeuLysSerGlyArgGlyLysIleLeuAspArgAsnAsnVal 160

161 GluLeuAlaAsnThrGlyThrAlaTyrGluIleGlyIleValProLys 176

177 AsnValSerLysLysAspTyrLysAlaIleAlaLysGluLeuSerIle 192

193 SerGluAspTyrIleLysGlnGlnMetAspGlnAsnTrpValGlnAsp 208

209 AspThrPheValProLeuLysThrValLysLysMetAspGluTyrLeu 224

225 SerAspPheAlaLysLysPheHisLeuThrThrAsnGluThrGluSer 240

241 ArgAsnTyrProLeuGluLysAlaThrSerHisLeuLeuGlyTyrVal 256

257 GlyProIleAsnSerGluGluLeuLysGlnLysGluTyrLysGlyTyr 272

273 LysAspAspAlaValIleGlyLysLysGlyLeuGluLysLeuTyrAsp 288

289 LysLysLeuGlnHisGluAspGlyTyrArgValThrIleValAspAsp 304

305 AsnSerAsnThrIleAlaHisThrLeuIleGluLysLysLysLysAsp 320

321 GlyLysAspIleGlnLeuThrIleAspAlaLysValGlnLysSerIle 336

337 TyrAsnAsnMetLysAsnAspTyrGlySerGlyThrAlaIleHisPro 352

353 GlnThrGlyGluLeuLeuAlaLeuValSerThrProSerTyrAspVal 368

FIG. 24

369 TyrProPheMetTyrGlyMetSerAsnGluGluTyrAsnLysLeuThr 384

385 GluAspLysLysGluProLeuLeuAsnLysPheGlnIleThrThrSer 400

401 ProGlySerThrGlnLysIleLeuThrAlaMetIleGlyLeuAsnAsn 416

417 LysThrLeuAspAspLysThrSerTyrLysIleAspGlyLysGlyTrp 432

433 GlnLysAspLysSerTrpGlyGlyTyrAsnValThrArgTyrGluVal 448

449 ValAsnGlyAsnIleAspLeuLysGlnAlaIleGluSerSerAspAsn 464

465 IlePhePheAlaArgValAlaLeuGluLeuGlySerLysLysPheGlu 480

481 LysGlyMetLysLysLeuGlyValGlyGluAspIleProSerAspTyr 496

497 ProPheTyrAsnAlaGlnIleSerAsnLysAsnLeuAspAsnGluIle 512

513 LeuLeuAlaAspSerGlyTyrGlyGlnGlyGluIleLeuIleAsnPro 528

529 ValGlnIleLeuSerIleTyrSerAlaLeuGluAsnAsnGlyAsnIle 544

545 AsnAlaProHisLeuLeuLysAspThrLysAsnLysValTrpLysLys 560

561 AsnIleIleSerLysGluAsnIleAsnLeuLeuThrAspGlyMetGln 576

577 GlnValValAsnLysThrHisLysGluAspIleTyrArgSerTyrAla 592

593 AsnLeuIleGlyLysSerGlyThrAlaGluLeuLysMetLysGlnGly 608

609 GluThrGlyArgGlnIleGlyTrpPheIleSerTyrAspLysAspAsn 624

625 ProAsnMetMetMetAlaIleAsnValLysAspValGlnAspLysGly 640

641 MetAlaSerTyrAsnAlaLysIleSerGlyLysValTyrAspGluLeu 656

657 TyrGluAsnGlyAsnLysLysTyrAspIleAspGluEnd 668

FIG. 24 **(continued)**

```
1   ATGAAAAAGATAAAAATTGTTCCACTTATTTTAATAGTTGTAGTTGTC   48
    MetLysLysIleLysIleValProLeuIleLeuIleValValValVal

49  GGGTTTGGTATATATTTTTATGCTTCAAAAGATAAAGAAATTAATAAT   96
    GlyPheGlyIleTyrPheTyrAlaSerLysAspLysGluIleAsnAsn

97  ACTATTGATGCAATTGAAGATAAAAATTTCAAACAAGTTTATAAAGAT   144
    ThrIleAspAlaIleGluAspLysAsnPheLysGlnValTyrLysAsp

145 AGCAGTTATATTTCTAAAAGCGATAATGGTGAAGTAGAAATGACTGAA   192
    SerSerTyrIleSerLysSerAspAsnGlyGluValGluMetThrGlu

193 CGTCCGATAAAAATATATAATAGTTTAGGCGTTAAAGATATAAACATT   240
    ArgProIleLysIleTyrAsnSerLeuGlyValLysAspIleAsnIle

241 CAGGATCGTAAAATAAAAAAAGTATCTAAAAATAAAAAACGAGTAGAT   288
    GlnAspArgLysIleLysLysValSerLysAsnLysLysArgValAsp

289 GCTCAATATAAAATTAAAACAAACTACGGTAACATTGATCGCAACGTT   336
    AlaGlnTyrLysIleLysThrAsnTyrGlyAsnIleAspArgAsnVal

337 CAATTTAATTTTGTTAAAGAAGATGGTATGTGGAAGTTAGATTGGGAT   384
    GlnPheAsnPheValLysGluAspGlyMetTrpLysLeuAspTrpAsp

385 CATAGCGTCATTATTCCAGGAATGCAGAAAGACCAAAGCATACATATT   432
    HisSerValIleIleProGlyMetGlnLysAspGlnSerIleHisIle

433 GAAAATTTAAAATCAGAACGTGGTAAAATTTTAGACCGAAACAATGTG   480
    GluAsnLeuLysSerGluArgGlyLysIleLeuAspArgAsnAsnVal

481 GAATTGGCCAATACAGGAACAGCATATGAGATAGGCATCGTTCCAAAG   528
    GluLeuAlaAsnThrGlyThrAlaTyrGluIleGlyIleValProLys
```

FIG. 25

```
529   AATGTATCTAAAAAAGATTATAAAGCAATCGCTAAAGAACTAAGTATT   576
      AsnValSerLysLysAspTyrLysAlaIleAlaLysGluLeuSerIle

577   TCTGAAGACTATATCAAACAACAAATGGATCAAAATTGGGTACAAGAT   624
      SerGluAspTyrIleLysGlnGlnMetAspGlnAsnTrpValGlnAsp

625   GATACCTTCGTTCCACTTAAAACCGTTAAAAAAATGGATGAATATTTA   672
      AspThrPheValProLeuLysThrValLysLysMetAspGluTyrLeu

673   AGTGATTTCGCAAAAAAATTTCATCTTACAACTAATGAAACAGAAAGT   720
      SerAspPheAlaLysLysPheHisLeuThrThrAsnGluThrGluSer

721   CGTAACTATCCTCTAGAAAAAGCGACTTCACATCTATTAGGTTATGTT   768
      ArgAsnTyrProLeuGluLysAlaThrSerHisLeuLeuGlyTyrVal

769   GGTCCCATTAACTCTGAAGAATTAAAACAAAAAGAATATAAAGGCTAT   816
      GlyProIleAsnSerGluGluLeuLysGlnLysGluTyrLysGlyTyr

817   AAAGATGATGCAGTTATTGGTAAAAAGGGACTCGAAAAACTTTACGAT   864
      LysAspAspAlaValIleGlyLysLysGlyLeuGluLysLeuTyrAsp

865   AAAAAGCTCCAACATGAAGATGGCTATCGTGTCACAATCGTTGACGAT   912
      LysLysLeuGlnHisGluAspGlyTyrArgValThrIleValAspAsp

913   AATAGCAATACAATCGCACATACATTAATAGAGAAAAAGAAAAAAGAT   960
      AsnSerAsnThrIleAlaHisThrLeuIleGluLysLysLysLysAsp

961   GGCAAAGATATTCAACTAACTATTGATGCTAAAGTTCAAAAGAGTATT   1008
      GlyLysAspIleGlnLeuThrIleAspAlaLysValGlnLysSerIle

1009  TATAACAACATGAAAAATGATTATGGCTCAGGTACTGCTATCCACCCT   1056
      TyrAsnAsnMetLysAsnAspTyrGlySerGlyThrAlaIleHisPro

1057  CAAACAGGTGAaTTATTAGCACTTGTAAGCACACCTTCATATGACGTC   1104
      GlnThrGlyGluLeuLeuAlaLeuValSerThrProSerTyrAspVal

1105  TATCCATTTATGTATGGCATGAGTAACGAAGAATATAATAAATTAACC   1152
      TyrProPheMetTyrGlyMetSerAsnGluGluTyrAsnLysLeuThr
```

FIG. 25 **(continued)**

```
1153  GAAGATAAAAAAGAACCTCTGCTCAACAAGTTCCAGATTACAACTTCA  1200
      GluAspLysLysGluProLeuLeuAsnLysPheGlnIleThrThrSer

1201  CCAGGTTCAACTCAAAAAATATTAACAGCAATGATTGGGTTAAATAAC  1248
      ProGlySerThrGlnLysIleLeuThrAlaMetIleGlyLeuAsnAsn

1249  AAAACATTAGACGATAAAACAAGTTATAAAATCGATGGTAAAGGTTGG  1296
      LysThrLeuAspAspLysThrSerTyrLysIleAspGlyLysGlyTrp

1297  CAAAAAGATAAATCTTGGGGTGGTTACAACGTTACAAGATATGAAGTG  1344
      GlnLysAspLysSerTrpGlyGlyTyrAsnValThrArgTyrGluVal

1345  GTAAATGGTAATATCGACTTAAAACAAGCAATAGAATCATCAGATAAC  1392
      ValAsnGlyAsnIleAspLeuLysGlnAlaIleGluSerSerAspAsn

1393  ATTTTCTTTGCTAGAGTAGCACTCGAATTAGGCAGTAAGAAATTTGAA  1440
      IlePhePheAlaArgValAlaLeuGluLeuGlySerLysLysPheGlu

1441  AAAGGCATGAAAAAACTAGGTGTTGGTGAAGATATACCAAGTGATTAT  1488
      LysGlyMetLysLysLeuGlyValGlyGluAspIleProSerAspTyr

1489  CCATTTTATAATGCTCAAATTTCAAACAAAAATTTAGATAATGAAATA  1536
      ProPheTyrAsnAlaGlnIleSerAsnLysAsnLeuAspAsnGluIle

1537  TTATTAGCTGATTCAGGTTACGGACaAGGTGAAATACTGATTAACCCA  1584
      LeuLeuAlaAspSerGlyTyrGlyGlnGlyGluIleLeuIleAsnPro

1585  GTACAGATCCTTTCAATCTATAGCGCATTAGAAAATAATGGCAATATT  1632
      ValGlnIleLeuSerIleTyrSerAlaLeuGluAsnAsnGlyAsnIle

1633  AACGCACCTCACTTATTAAAAGACACGAAAAACAAAGTTTGGAAGAAA  1680
      AsnAlaProHisLeuLeuLysAspThrLysAsnLysValTrpLysLys

1681  AATATTATTTCCAAAGAAAATATCAATCTATTAACTGATGGTATGCAA  1728
      AsnIleIleSerLysGluAsnIleAsnLeuLeuThrAspGlyMetGln

1729  CAAGTCGTAAATAAAACACATAAAGAAGATATTTATAGATCTTATGCA  1776
      GlnValValAsnLysThrHisLysGluAspIleTyrArgSerTyrAla
```

FIG. 25 **(continued)**

```
1777  AACTTAATTGGCAAATCCGGTACTGCAGAACTCAAAATGAAACAAGGA  1824
      AsnLeuIleGlyLysSerGlyThrAlaGluLeuLysMetLysGlnGly

1825  GAAACTGGCAGACAAATTGGGTGGTTTATATCATATGATAAAGATAAT  1872
      GluThrGlyArgGlnIleGlyTrpPheIleSerTyrAspLysAspAsn

1873  CCAAACATGATGATGGCTATTAATGTTAAAGATGTACAAGATAAAGGA  1920
      ProAsnMetMetMetAlaIleAsnValLysAspValGlnAspLysGly

1921  ATGGCTAGCTACAATGCCAAAATCTCAGGTAAAGTGTATGATGAGCTA  1968
      MetAlaSerTyrAsnAlaLysIleSerGlyLysValTyrAspGluLeu

1969  TATGAGAACGGTAATAAAAAATACGATATAGATGAATAA  2007
      TyrGluAsnGlyAsnLysLysTyrAspIleAspGluEnd
```

FIG. 25 **(continued)**

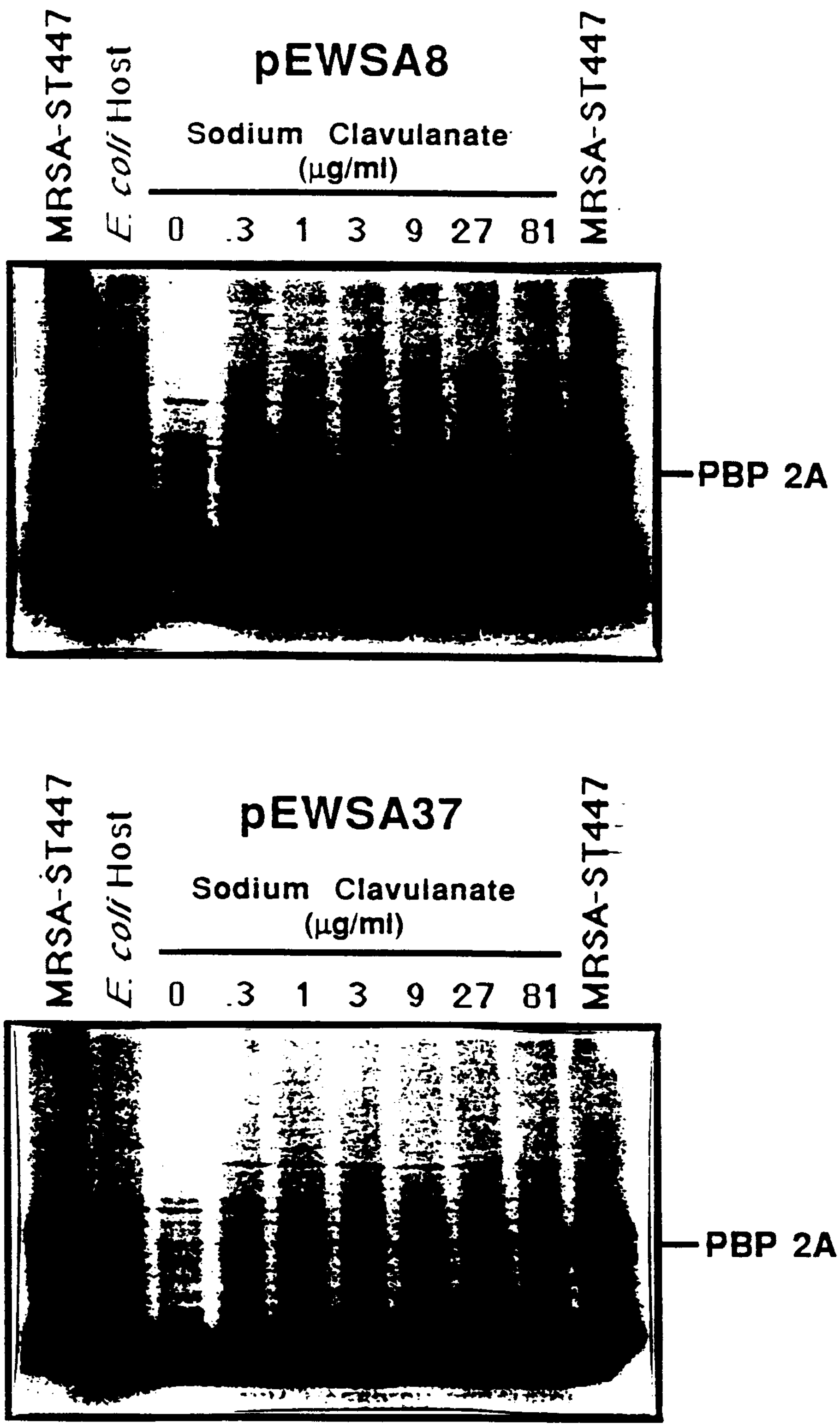
MRSA-ST447
E. coli Host
pEWSA8
Sodium Clavulanate
(μg/ml)
0 .3 1 3 9 27 81
MRSA-ST447
PBP 2A
MRSA-ST447
E. coli Host
pEWSA37
Sodium Clavulanate
(μg/ml)
0 .3 1 3 9 27 81
MRSA-ST447
PBP 2A

FIG. 26

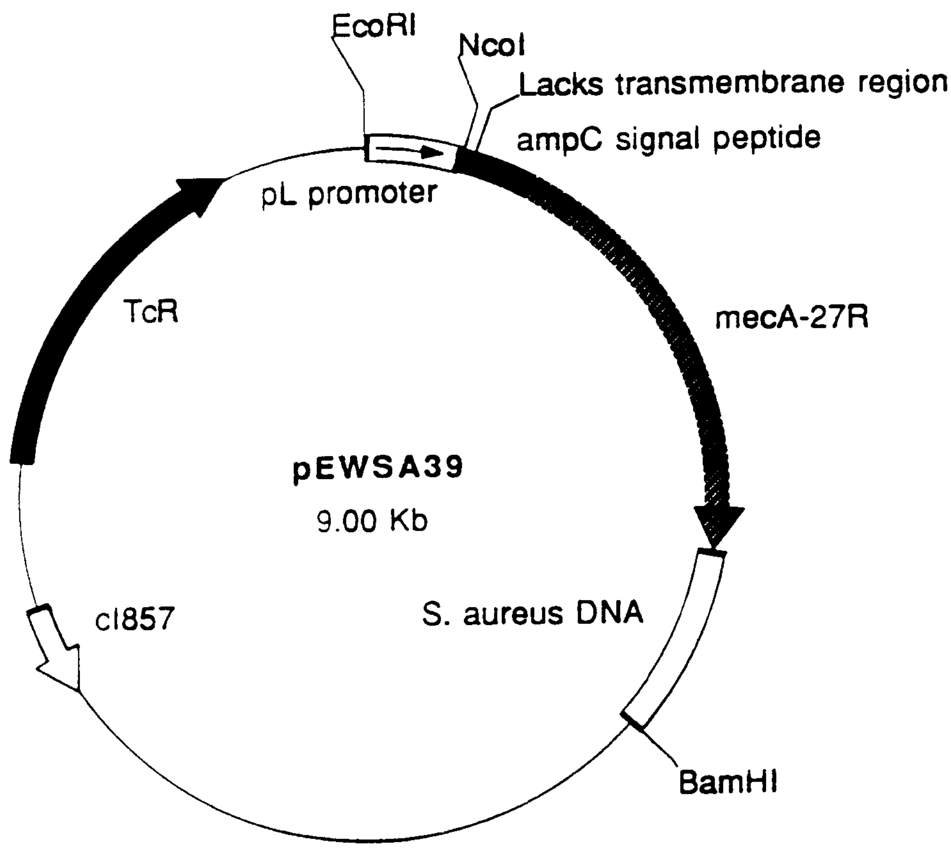
EcoRI
NcoI
Lacks transmembrane region
ampC signal peptide
pL promoter
TcR
mecA-27R
pEWSA39
9.00 Kb
cI857
S. aureus DNA
BamHI

FIG. 27

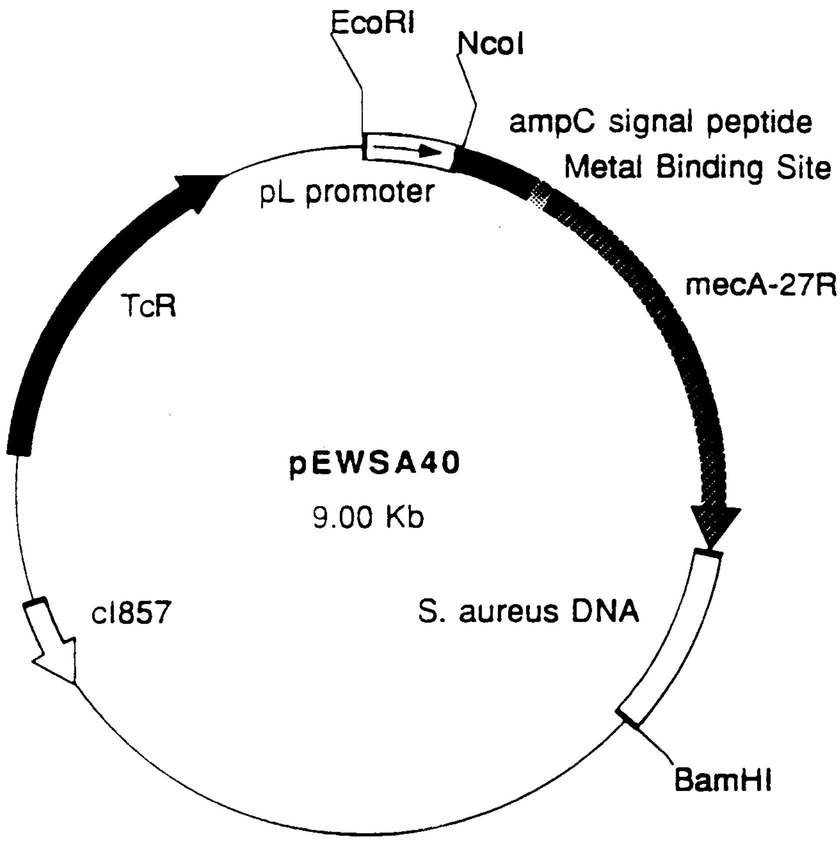
EcoRI
NcoI
ampC signal peptide
Metal Binding Site
pL promoter
mecA-27R
TcR
pEWSA40
9.00 Kb
cI857
S. aureus DNA
BamHI

FIG. 28

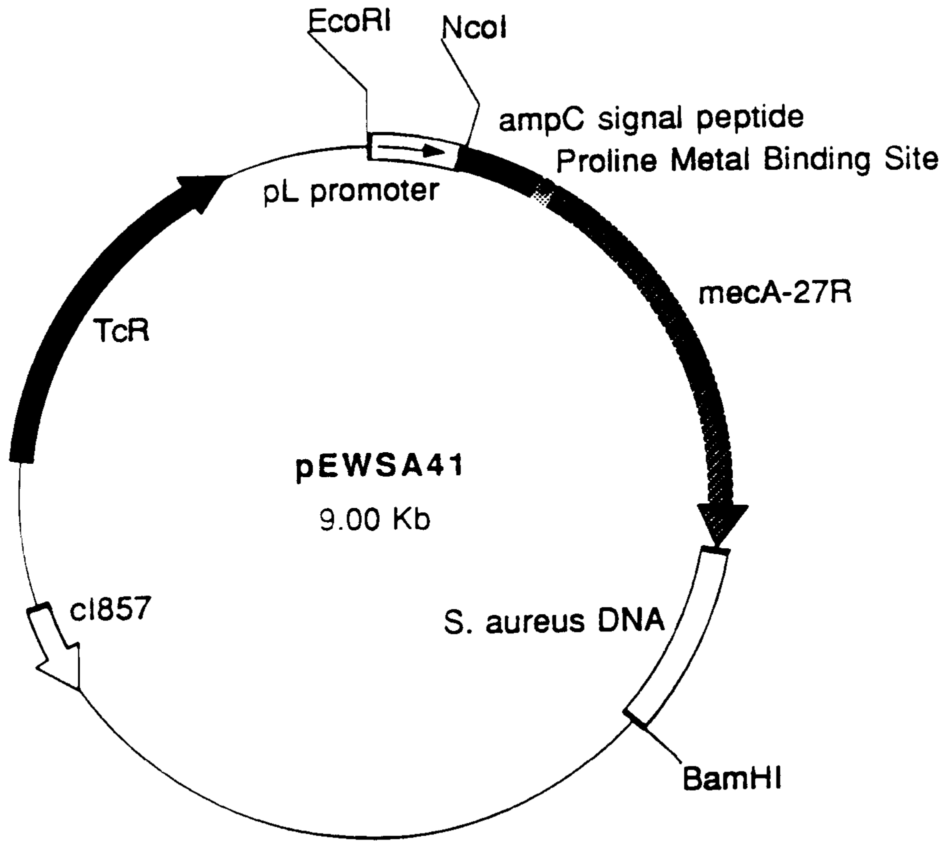
EcoRI
NcoI
ampC signal peptide
Proline Metal Binding Site
pL promoter
mecA-27R
TcR
pEWSA41
9.00 Kb
cI857
S. aureus DNA
BamHI

FIG. 29

```
          1                                                     49
Mecaepi   ATGAAAAAGA TAAAAATTGT TCCACTTATT TTAATAGTTG TAGTTGTCGG
Mecaaur   ATGAAAAAGA TAAAAATTGT TCCACTTATT TTAATAGTTG TAGTTGTCGG
  Matso   ATGAAAAAGA TAAAAATTGT TCCACTTATT TTAATAGTTG TAGTTGTCGG
  Ewpbp   ATGAAAAAGA TAAAAATTGT TCCACTTATT TTAATAGTTG TAGTTGTCGG

          50                                                    99
Mecaepi   GTTTGGTATA TATTTTTATG CTTCAAAAGA TAAAGAAATT AATAATACTA
Mecaaur   GTTTGGTATA TATTTTTATG CTTCAAAAGA TAAAGAAATT AATAATACTA
  Matso   GTTTGGTATA TATTTTTATG CTTCAAAAGA TAAAGAAATT AATAATACTA
  Ewpbp   GTTTGGTATA TATTTTTATG CTTCAAAAGA TAAAGAAATT AATAATACTA

          100                                                  149
Mecaepi   TTGATGCAAT TGAAGATAAA AATTTCAAAC AAGTTTATAA AGATAGCAGT
Mecaaur   TTGATGCAAT TGAAGATAAA AATTTCAAAC AAGTTTATAA AGATAGCAGT
  Matso   TTGATGCAAT TGAAGATAAA AATTTCAAAC AAGTTTATAA AGATAGCAGT
  Ewpbp   TTGATGCAAT TGAAGATAAA AATTTCAAAC AAGTTTATAA AGATAGCAGT

          150                                                  199
Mecaepi   TATATTTCTA AAAGCGATAA TGGTGAAGTA GAAATGACTG AACGTCCGAT
Mecaaur   TATATTTCTA AAAGCGATAA TGGTGAAGTA GAAATGACTG AACGTCCGAT
  Matso   TATATTTCTA AAAGCGATAA TGGTGAAGTA GAAATGACTG AACGTCCGAT
  Ewpbp   TATATTTCTA AAAGCGATAA TGGTGAAGTA GAAATGACTG AACGTCCGAT

          200                                                  249
Mecaepi   AAAAATATAT AATAGTTTAG GCGTTAAAGA TATAAACATT CAGGATCGTA
Mecaaur   AAAAATATAT AATAGTTTAG GCGTTAAAGA TATAAACATT CAGGATCGTA
  Matso   AAAAATATAT AATAGTTTAG GCGTTAAAGA TATAAACATT CAGGATCGTA
  Ewpbp   AAAAATATAT AATAGTTTAG GCGTTAAAGA TATAAACATT CAGGATCGTA

          250                                                  299
Mecaepi   AAATAAAAAA AGTATCTAAA AATAAAAAAC GAGTAGATGC TCAATATAAA
Mecaaur   AAATAAAAAA AGTATCTAAA AATAAAAAAC GAGTAGATGC TCAATATAAA
  Matso   AAATAAAAAA AGTATCTAAA AATAAAAAAC GAGTAGATGC TCAATATAAA
  Ewpbp   AAATAAAAAA AGTATCTAAA AATAAAAAAC GAGTAGATGC TCAATATAAA

          300                                                  349
Mecaepi   ATTAAAACAA ACTACGGTAA CATTGATCGC AACGTTCAAT TTAATTTTGT
Mecaaur   ATTAAAACAA ACTACGGTAA CATTGATCGC AACGTTCAAT TTAATTTTGT
  Matso   ATTAAAACAA ACTACGGTAA CATTGATCGC AACGTTCAAT TTAATTTTGT
  Ewpbp   ATTAAAaCAA ACTACGGTAA CATTGATCGC AACGTTCAAT TTAATTTTGT

          350                                                  399
Mecaepi   TAAAGAAGAT GGTATGTGGA AGTTAGATTG GGATCATAGC GTCATTATTC
Mecaaur   TAAAGAAGAT GGTATGTGGA AGTTAGATTG GGATCATAGC GTCATTATTC
  Matso   TAAAGAAGAT GGTATGTGGA AGTTAGATTG GGATCATAGC GTCATTATTC
  Ewpbp   TAAAGAAgaT GGTATGTGgA AGTTAGATTG GGATCATAGC GTCATTATTC
```

FIG. 30

```
         400                                                449
Mecaepi  CAGGAATGCA GAAAGACCAA AGCATACATA TTGAAAATTT AAAATCAGAA
Mecaaur  CAGGAATGCA GAAAGACCAA AGCATACATA TTGAAAATTT AAAATCAGAA
  Matso  CAGGAATGCA GAAAGACCAA AGCATACATA TTGAAAATTT AAAATCAGAA
  Ewpbp  CAGGAATGCA GAAAGACCAA AGCATACATA TTGAAAATTT AAAATCAGAa

         450                                                499
Mecaepi  CGTGGTAAAA TTTTAGACCG AAACAATGTG GAATTGGCCA ATACAGGAAC
Mecaaur  CGTGGTAAAA TTTTAGACCG AAACAATGTG GAATTGGCCA ATACAGGAAC
  Matso  CGTGGTAAAA TTTTAGACCG AAACAATGTG GAATTGGCCA ATACAGGAAC
  Ewpbp  CGTGGTAAAA TTTTAGACCG AAACAATGTG GAATTGGCCA ATACAGGAAc

         500                                                549
Mecaepi  AGCATATGAG A.TAGGCATC GTTCCAAAGA ATGTATCTAA AAAAGATTAT
Mecaaur  A.CATATGAG ATTAGGCATC GTTCCAAAGA ATGTATCTAA AAAAGATTAT
  Matso  A.CATATGAG ATTAGGCATC GTTCCAAAGA ATGTATCTAA AAAAGATTAT
  Ewpbp  AGCATATGAG A.TAGGCATC GTTCCAAAGA ATGTATCTAA AAAAGATTAT

         550                                                599
Mecaepi  AAAGCAATCG CTAAAGAACT AAGTATTTCT GAAGACTATA TCAAACAACA
Mecaaur  AAAGCAATCG CTAAAGAACT AAGTATTTCT GAAGACTATA TCAA.CAACA
  Matso  AAAGCAATCG CTAAAGAACT AAGTATTTCT GAAGACTATA TCAA.CAACA
  Ewpbp  AAAGCAATCG CTAAAGAACT AAGTATTTCT GAAGACTATA TCAAACAACA

         600                                                649
Mecaepi  AATGGATCAA AATTGGGTAC AAGATGATAC CTTCGTTCCA CTT.AAAACC
Mecaaur  AATGGATCAA AATTGGGTAC AAGATGATAC CTTCGTTCCA CTTTAAAACC
  Matso  AATGGATCAA AATTGGGTAC AAGATGATAC CTTCGTTCCA CTTTAAAACC
  Ewpbp  AATGGATCAA AATTGGGTAC AAGATGATAC CTTCGTTCCA CTT.AAAACC

         650                                                699
Mecaepi  GTTAAAAAAA TGGATGAATA TTTAAGTGAT TTCGCAAAAA AATTTCATCT
Mecaaur  GTTAAAAAAA TGGATGAATA TTTAAGTGAT TTCGCAAAAA AATTTCATCT
  Matso  GTTAAAAAAA TGGATGAATA TTTAAGTGAT TTCGCAAAAA AATTTCATCT
  Ewpbp  GTTAAAAAAA TGGATGAATA TTTAAGTGAT TTCGCAAAAA AATTTCATCT

         700                                                749
Mecaepi  TACAACTAAT GAAACAGAAA GTCGTAACTA TCCTCTAGGA AAAGCGACTT
Mecaaur  TACAACTAAT GAAACAGAAA GTCGTAACTA TCCTCTAGAA AAAGCGACTT
  Matso  TACAACTAAT GAAACAGAAA GTCGTAACTA TCCTCTAGGA AAAGCGACTT
  Ewpbp  TACAACTAAT GAAACAGAAA GTCGTAACTA TCCTCTAGAA AAAGCGACTT

         750                                                799
Mecaepi  CACATCTATT AGGTTATGTT GGTCCCATTA ACTCTGAAGA ATTAAAACAA
Mecaaur  CACATCTATT AGGTTATGTT GGTCCCATTA ACTCTGAAGA ATTAAAACAA
  Matso  CACATCTATT AGGTTATGTT GGTCCCATTA ACTCTGAAGA ATTAAAACAA
  Ewpbp  CACATCTATT AGGTTATGTT GGTCCCATTA ACTCTGAAGA ATTAAAACAA

         800                                                849
Mecaepi  AAAGAATATA AAGGCTATAA AGATGATGCA GTTATTGGTA AAAAGGGACT
Mecaaur  AAAGAATATA AAGGCTATAA AGATGATGCA GTTATTGGTA AAAAGGGACT
  Matso  AAAGAATATA AAGGCTATAA AGATGATGCA GTTATTGGTA AAAAGGGACT
  Ewpbp  AAAGAATATA AAGGCTATAA AGATGATGCA GTTATTGGTA AAAAGGGACT
```

FIG. 30(continued)

```
         850                                                899
Mecaepi  CGAAAAACTT TACGATAAAA AGCTCCAACA TGAAGATGGC TATCGTGTCA
Mecaaur  CGAAAAACTT TACGATAAAA AGCTCCAACA TGAAGATGGC TATCGTGTCA
  Matso  CGAAAAACTT TACGATAAAA AGCTCCAACA TGAAGATGGC TATCGTGTCA
  Ewpbp  CGAAAAACTT TACGATAAAA AGCTCCAACA TGAAGATGGC TATCGTGTCA

         900                                                949
Mecaepi  CAATCGTT.. ....GACGAT AATAGCAATA CAATCGCACA TACATTAATA
Mecaaur  CAATCGTT.. ....GACGAT AATAGCAATA CAATCGCACA TACATTAATA
  Matso  CAATCGTTAG AGTCGACGAT AATAGCAATA CAATCGCACA TACATTAATA
  Ewpbp  CAATCGTT.. ....GACGAT AATAGCAATA CAATCGCACA TACATTAATA

         950                                                999
Mecaepi  GAGAAAAAGA AAAAAGATGG CAAAGATATT CAACTAACTA TTGATGCTAA
Mecaaur  GAGAAAAAGA AAAAAGATGG CAAAGATATT CAACTAACTA TTGATGCTAA
  Matso  GAGAAAAAGA AAAAAGATGG CAAAGATATT CAACTAACTA TTGATGCTAA
  Ewpbp  GAGAAAAAGA AAAAAGATGG CAAAGATATT CAACTAACTA TTGATGCTAA

         1000                                              1049
Mecaepi  AGTTCAAAAG AGTATTTATA ACAACATGAA AAATGATTAT GGCTCAGGTA
Mecaaur  AGTTCAAAAG AGTATTTATA ACAACATGAA AAATGATTAT GGCTCAGGTA
  Matso  AGTTCAAAAG AGTATTTATA ACAACATGAA AAATGATTAT GGCTCAGGTA
  Ewpbp  AGTTCAAAAG AGTATTTATA ACAACATGAA AAATGATTAT GGCTCAGGTA

         1050                                              1099
Mecaepi  CTGCTATCCA CCCTCAAACA GGTGAATTAT TAGCACTTGT AAGCACACCT
Mecaaur  CTGCTATCCA CCCTCAAACA GGTGAATTAT TAGCACTTGT AAGCACACCT
  Matso  CTGCTATCCA CCCTCAAACA GGTGAATTAT TAGCACTTGT AAGCACACCT
  Ewpbp  CTGCTATCCA CCCTCAAACA GGTGAaTTAT TAGCACTTGT AAGCACACCT

         1100                                              1149
Mecaepi  TCATATGACG TCTATCCATT TATGTATGGC ATGAGTAACG AAGAATATAA
Mecaaur  TCATATGACG TCTATCCATT TATGTATGGC ATGAGTAACG AAGAATATAA
  Matso  TCATATGACG TCTATCCATT TATGTATGGC ATGAGTAACG AAGAATATAA
  Ewpbp  TCATATGACG TCTATCCATT TATGTATGGC ATGAGTAACG AAGAATATAA

         1150                                              1199
Mecaepi  TAAATTAACC GAAGATAAAA AAGAACCTCT GCTCAACAAG TTCCAGATTA
Mecaaur  TAAATTAACC GAAGATAAAA AAGAACCTCT GCTCAACAAG TTCCAGATTA
  Matso  TAAATTAACC GAAGATAAAA AAGAACCTCT GCTCAACAAG TTCCAGATTA
  Ewpbp  TAAATTAACC GAAGATAAAA AAGAACCTCT GCTCAACAAG TTCCAGATTA

         1200                                              1249
Mecaepi  CAACTTCACC AGGTTCAACT CAAAAAATAT TAACAGCAAT GATTGGGTTA
Mecaaur  CAACTTCACC AGGTTCAACT CAAAAAATAT TAACAGCAAT GATTGGGTTA
  Matso  CAACTTCACC AGGTTCAACT CAAAAAATAT TAACAGCAAT GATTGGGTTA
  Ewpbp  CAACTTCACC AGGTTCAACT CAAAAAATAT TAACAGCAAT GATTGGGTTA

         1250                                              1299
Mecaepi  AATAACAAAA CATTAGACGA TAAAACAAGT TATAAAATCG ATGGTAAAGG
Mecaaur  AATAACAAAA CATTAGACGA TAAAACAAGT TATAAAATCG ATGGTAAAGG
  Matso  AATAACAAAA CATTAGACGA TAAAACAAGT TATAAAATCG ATGGTAAAGG
  Ewpbp  AATAACAAAA CATTAGACGA TAAAACAAGT TATAAAATCG ATGGTAAAGG
```

FIG. 30(continued)

```
         1300                                               1349
Mecaepi  TTGGCAAAAA GATAAATCTT GGGGTGGTTA CAACGTTACA AGATATGAAG
Mecaaur  TTGGCAAAAA GATAAATCTT GGGGTGGTTA CAACGTTACA AGATATGAAG
  Matso  TTGGCAAAAA GATAAATCTT GGGGTGGTTA CAACGTTACA AGATATGAAG
  Ewpbp  TTGGCAAAAA GATAAATCTT GGGGTGGTTA CAACGTTACA AGATATGAAG

         1350                                               1399
Mecaepi  TGGTAAATGG TAATATCGAC TTAAAACAAG CAATAGAATC ATCAGATAAC
Mecaaur  TGGTAAATGG TAATATCGAC TTAAAACAAG CAATAGAATC ATCAGATAAC
  Matso  TGGTAAATGG TAATATCGAC TTAAAACAAG CAATAGAATC ATCAGATAAC
  Ewpbp  TGGTAAATGG TAATATCGAC TTAAAACAAG CAATAGAATC ATCAGATAAC

         1400                                               1449
Mecaepi  ATTTTCTTTG CTAGAGTAGC ACTCGAATTA GGCAGTAAGA AATTTGAAAA
Mecaaur  ATTTTCTTTG CTAGAGTAGC ACTCGAATTA GGCAGTAAGA AATTTGAAAA
  Matso  ATTTTCTTTG CTAGAGTAGC ACTCGAATTA GGCAGTAAGA AATTTGAAAA
  Ewpbp  ATTTTCTTTG CTAGAGTAGC ACTCGAATTA GGCAGTAAGA AATTTGAAAA

         1450                                               1499
Mecaepi  AGGCATGAAA AAACTAGGTG TTGGTGAAGA TATACCAAGT GATTATCCAT
Mecaaur  AGGCATGAAA AAACTAGGTG TTGGTGAAGA TATACCAAGT GATTATCCAT
  Matso  AGGCATGAAA AAACTAGGTG TTGGTGAAGA TATACCAAGT GATTATCCAT
  Ewpbp  AGGCATGAAA AAACTAGGTG TTGGTGAAGA TATACCAAGT GATTATCCAT

         1500                                               1549
Mecaepi  TTTATAATGC TCAAATTTCA AACAAAAATT TAGATAATGA AATATTATTA
Mecaaur  TTTATAATGC TCAAATTTCA AACAAAAATT TAGATAATGA AATATTATTA
  Matso  TTTATAATGC TCAAATTTCA AACAAAAATT TAGATAATGA AATATTATTA
  Ewpbp  TTTATAATGC TCAAATTTCA AACAAAAATT TAGATAATGA AATATTATTA

         1550                                               1599
Mecaepi  GCTGATTCAG GTTACGGACA AGGTGAAATA CTGATTAACC CAGTACAGAT
Mecaaur  GCTGATTCAG GTTACGGACA AGGTGAAATA CTGATTAACC CAGTACAGAT
  Matso  GCTGATTCAG GTTACGGACA AGGTGAAATA CTGATTAACC CAGTACAGAT
  Ewpbp  GCTGATTCAG GTTACGGACa AGGTGAAATA CTGATTAACC CAGTACAGAT

         1600                                               1649
Mecaepi  CCTTTCAATC TATAGCGCAT TAGAAAATAA TGGCAATATT AACGCACCTC
Mecaaur  CCTTTCAATC TATAGCGCAT TAGAAAATAA TGGCAATATT AACGCACCTC
  Matso  CCTTTCAATC TATAGCGCAT TAGAAAATAA TGGCAATATT AACGCACCTC
  Ewpbp  CCTTTCAATC TATAGCGCAT TAGAAAATAA TGGCAATATT AACGCACCTC

         1650                                               1699
Mecaepi  ACTTATTAAA AGACACGAAA AACAAAGTTT GGAAGAAAAA TATTATTTCC
Mecaaur  ACTTATTAAA AGACACGAAA AACAAAGTTT GGAAGAAAAA TATTATTTCC
  Matso  ACTTATTAAA AGACACGAAA AACAAAGTTT GGAAGAAAAA TATTATTTCC
  Ewpbp  ACTTATTAAA AGACACGAAA AACAAAGTTT GGAAGAAAAA TATTATTTCC

         1700                                               1749
Mecaepi  AAAGAAAATA TCAATCTATT AACTGATGGT ATGCAACAAG TCGTAAATAA
Mecaaur  AAAGAAAATA TCAATCTATT AAATGATGGT ATGCAACAAG TCGTAAATAA
  Matso  AAAGAAAATA TCAATCTATT AAATGATGGT ATGCAACAAG TCGTAAATAA
  Ewpbp  AAAGAAAATA TCAATCTATT AACTGATGGT ATGCAACAAG TCGTAAATAA
```

FIG. 30**(continued)**

```
          1750                                                     1799
Mecaepi   AACACATAAA GAAGATATTT ATAGATCTTA TGCAAACTTA ATTGGCAAAT
Mecaaur   AACACATAAA GAAGATATTT ATAGATCTTA TGCAAACTTA ATTGGCAAAT
  Matso   AACACATAAA GAAGATATTT ATAGATCTTA TGCAAACTTA ATTGGCAAAT
  Ewpbp   AACACATAAA GAAGATATTT ATAGATCTTA TGCAAACTTA ATTGGCAAAT

          1800                                                     1849
Mecaepi   CCGGTACTGC AGAACTCAAA ATGAAACAAG GAGAAACTGG CAGACAAATT
Mecaaur   CCGGTACTGC AGAACTCAAA ATGAAACAAG GAGAAAGTGG CAGACAAATT
  Matso   CCGGTACTGC AGAACTCAAA ATGAAACAAG GAGAAACTGG CAGACAAATT
  Ewpbp   CCGGTACTGC AGAACTCAAA ATGAAACAAG GAGAAACTGG CAGACAAATT

          1850                                                     1899
Mecaepi   GGGTGGTTTA TATCATATGA TAAAGATAAT CCAAACATGA TGATGGCTAT
Mecaaur   GGGTGGTTTA TATCATATGA TAAAGATAAT CCAAACATGA TGATGGCTAT
  Matso   GGGTGGTTTA TATCATATGA TAAAGATAAT CCAAACATGA TGATGGCTAT
  Ewpbp   GGGTGGTTTA TATCATATGA TAAAGATAAT CCAAACATGA TGATGGCTAT

          1900                                                     1949
Mecaepi   TAATGTTAAA GATGTACAAG ATAAAGGAAT GGCTAGCTAC AATGCCAAAA
Mecaaur   TAATGTTAAA GATGTACAAG ATAAAGGAAT GGCTAGCTAC AATGCCAAAA
  Matso   TAATGTTAAA GATGTACAAG ATAAAGGAAT GGCTAGCTAC AATGCCAAAA
  Ewpbp   TAATGTTAAA GATGTACAAG ATAAAGGAAT GGCTAGCTAC AATGCCAAAA

          1950                                                     1999
Mecaepi   TCTCAGGTAA AGTGTATGAT GAGCTATATG AGAACGGTAA TAAAAAATAC
Mecaaur   TCTCAGGTAA AGTGTATGAT GAGCTATATG AGAACGGTAA TAAAAAATAC
  Matso   TCTCAGGTAA AGTGTATGAT GAGCTATATG AGAACGGTAA TAAAAAATAC
  Ewpbp   TCTCAGGtAA AGTGTATGAT GAGCTATATG AGAACGGTAA TAAAAAATAC

          2000
Mecaepi   GATATAGATG AATAA
Mecaaur   GATATAGATG AATAA
  Matso   GATATAGATG AATAA
  Ewpbp   GATATAGATG AATAA
```

FIG. 30**(continued)**